(19) Europäisches Patentamt
European Patent Office
Office européen des brevets

(11) Veröffentlichungsnummer : **0 119 161**
**B1**

# (12) EUROPÄISCHE PATENTSCHRIFT

(45) Veröffentlichungstag der Patentschrift : **28.06.89**

(21) Anmeldenummer : **84810068.1**

(22) Anmeldetag : **06.02.84**

(51) Int. Cl.[4] : **C 07 D225/00**, A 61 K 37/02, A 61 K 37/64

(54) **Gewisse Benzazocinon- und Benzazoninon-Derivate, Verfahren zu ihrer Herstellung, pharmazeutische Präparate enthaltend diese Verbindungen, sowie ihre therapeutische Verwendung.**

(30) Priorität : **10.02.83 US 465696**

(43) Veröffentlichungstag der Anmeldung : **19.09.84 Patentblatt 84/38**

(45) Bekanntmachung des Hinweises auf die Patenterteilung : **28.06.89 Patentblatt 89/26**

(84) Benannte Vertragsstaaten :
**AT BE CH DE FR GB IT LI LU NL SE**

(56) Entgegenhaltungen :
**EP-A- 0 107 095**
**CHEMICAL ABSTRACTS, Band 100, 1984, Seite 19, Ref. Nr. 167718k, Columbus, Ohio, US. W. H. PARSONS et al.: "BLenzolactams. A new class of converting enyzme inhibitors" & Biochem. Biophys. Res. Commun. 1983, 117(1), 103-13**

(73) Patentinhaber : **CIBA-GEIGY AG**
**Klybeckstrasse 141**
**CH-4002 Basel (CH)**

(72) Erfinder : **Watthey, Jeffrey W.H., Dr.**
**61 Deepwood Drive**
**Chappaqua New York 10514 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 119 161 B1

Jouve, 18, rue St-Denis, 75001 Paris, France

## Beschreibung

Die Erfindung betrifft neue Benzazocinon- und Benzazoninon-Derivate der allgemeinen Formel

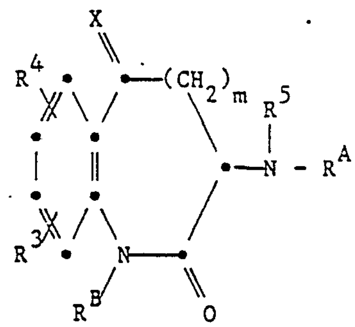

(I)

worin $R^A$ und $R^B$ Reste der Formel

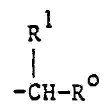

bzw.

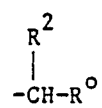

darstellen, worin $R^0$ Carboxy oder ein funktionell modifiziertes Carboxy darstellt; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Aryl-(thio-, sulfinyl- oder sulfonyl-)-niederalkyl, Aryl-N-niederalkylamino-niederalkyl oder Arylamino-niederalkyl bedeutet; $R^2$ für Wasserstoff oder Niederalkyl steht; $R^3$ und $R^4$ unabhängig voneinander Wasserstoff, Niederalkyl, Niederalkoxy, Niederalkanoyloxy, Hydroxy, Halogen oder Trifluormethyl bedeuten, oder $R^3$ und $R^4$ gemeinsam für Niederalkylendioxy stehen; $R^5$ Wasserstoff oder Niederalkyl bedeutet; m für 2 oder 3 steht; und X Oxo, zwei Wasserstoffatome oder Hydroxy oder acyliertes Hydroxy zusammen mit einem Wasserstoffatom bedeutet; und worin der carbocyclische Ring auch perhydriert sein kann; Salze, insbesondere ihre pharmazeutisch verträglichen Salze, Stereoisomere von allen diesen Verbindungen; Verfahren zu ihrer Herstellung, pharmazeutische Präparate enthaltend diese Verbindungen, sowie ihre therapeutische Verwendung.

Eine funktionell modifizierte Carboxylgruppe $R^0$ ist beispielsweise eine veresterte Carboxylgruppe oder eine Carbamoylgruppe, die gegebenenfalls N-substituiert ist. Insbesondere bedeuten einer oder beide Reste $R^0$ unabhängig voneinander Carboxy, verestertes Carboxy, Carbamoyl oder substituiertes Carbamoyl.

Eine Gruppe $R^0$ wird durch $COR^6$ im Radikal $R^A$ und durch $COR^7$ im Radikal $R^B$ dargestellt.

Eine Carboxylgruppe $R^0$ wird durch $COR^6$, worin $R^6$ Hydroxy ist, oder durch $COR^7$, worin $R^7$ Hydroxy ist, dargestellt.

Eine veresterte Carboxylgruppe $R^0$ ist insbesondere eine solche, worin der veresternde Rest gegebenenfalls substituiertes Niederalkyl oder gegebenenfalls substituiertes Phthalidyl darstellt, und wird dargestellt durch $COR^6$ oder $COR^7$, worin einer der Reste $R^6$ und $R^7$ oder beide Reste Niederalkoxy bedeuten, welches gegebenenfalls durch Amino, Mono- oder Di-niederalkylamino, Carboxy, z. B. in der $\alpha$-Stellung, Niederalkoxycarbonyl, z. B. in der $\alpha$-Stellung, Aryl, Hydroxy, Niederalkanoyloxy, Niederalkoxy, Bicycloalkoxycarbonyl substituiert sein kann, oder für 3-Phthalidoxy oder (Niederalkyl, Niederalkoxy, Halogen)-substituiertes 3-Phthalidoxy stehen kann.

Eine gegebenenfalls N-substituierte Carbamoylgruppoe $R^0$ wird durch $COR^6$ oder $COR^7$ dargestellt, worin einer der Reste $R^6$ und $R^7$ oder beide insbesondere Amino; Niederalkylamino; Di-niederalkylamino; Di-niederalkylamino, worin beide Alkylgruppen durch eine Kohlenstoff-Kohlenstoff-Bindung verbunden sind und gemeinsam mit dem Aminostickstoff einen 5-, 6- oder 7-gliedrigen heterocyclischen Ring, z. B. Pyrrolidino, Piperidino oder Perhydroazepino, bilden; (Amino oder Acylamino)-substituiertes Niederalkylamino; $\alpha$-(Carboxy oder Niederalkoxycarbonyl)-substituiertes Niederalkylamino oder Arylsubstituiertes Niederalkylamino, worin Aryl vorzugsweise Phenyl oder Indolyl bedeutet und das an dem $\alpha$-Kohlenstoffatom durch Carboxy oder Niederalkoxycarbonyl substituiert sein kann, bedeuten.

Weiter können die Begriffe verestertes Carboxy und N-substituiertes Carbamoyl, sofern sie in der Definition von $R^1$ als verestertes Carboxy-niederalkyl und N-substituiertes Carbamoyl-niederalkyl erscheinen, irgendeine der für diese Begriffe oben angegebenen Bedeutungen haben.

Die vorliegend verwendeten allgemeinen Definitionen besitzen im Bereich der Erfindung die

folgenden Bedeutungen :

Die Bezeichnung « nieder », die vorstehend und nachfolgend in Verbindung mit organischen Resten bzw. Verbindungen verwendet wird, definiert solche mit bis zu und einschliesslich 7, vorzugsweise bis zu und einschliesslich 4 und insbesondere 1 oder 2 Kohlenstoffatomen.

Niederalkyl enthält 1-7 Kohlenstoffatome, vorzugsweise 1 bis 4 Kohlenstoffatome und bedeutet z. B. Ethyl, Propyl, Butyl oder insbesondere Methyl.

Aryl bedeutet einen carbocyclischen oder heterocyclischen aromatischen Rest, vorzugsweise unsubstituiertes oder durch Niederalkyl, Niederalkoxy, Niederalkylendioxy, Niederalkanoyloxy, Hydroxy, Halogen oder Trifluormethyl mono- oder disubstituiertes Phenyl ; Indolyl, vorzugsweise 3-Indolyl ; oder durch Niederalkyl, Niederalkoxy, Niederalkylendioxy, Niederalkanoyloxy, Hydroxy, Halogen oder Trifluormethyl substituiertes Indolyl, vorzugsweise 3-Indolyl.

Cycloalkyl bedeutet einen gesättigten cyclischen Kohlenwasserstoffrest, der vorzugsweise 3 bis 8 Kohlenstoffatome enthält und beispielsweise Cyclopentyl oder Cyclohexyl ist.

Arylniederalkyl bedeutet vorzugsweise Benzyl, 1- oder 2-Phenylethyl, 1-, 2- oder 3-Phenylpropyl, 1-, 2-, 3- oder 4-Phenylbutyl, worin der Phenylring unsubstituiert oder durch Niederalkyl, Hydroxy, Niederalkoxy, Niederalkylendioxy, Niederalkanoyloxy, Halogen oder Trifluormethyl mono- oder disubstituiert ist ; und auch Indolylmethyl, vorzugsweise 3-Indolylmethyl, 1- oder 2-Indolylethyl, vorzugsweise 2-(3-Indolyl)ethyl.

Cycloalkyl-niederalkyl bedeutet vorzugsweise 1- oder 2-(Cyclopentyl oder Cyclohexyl)ethyl, 1-, 2- oder 3-(Cyclopentyl oder Cyclohexyl)propyl oder 1-, 2-, 3- oder 4-(Cyclopentyl oder Cyclohexyl)butyl.

Niederalkoxy enthält vorzugsweise 1 bis 4 Kohlenstoffatome und ist beispielsweise Methoxy, Propoxy, Isopropoxy oder insbesondere Ethoxy.

Mono-niederalkylamino enthält vorzugsweise 1 bis 4 Kohlenstoffatome in dem Niederalkylteil und ist beispielsweise N-Methylamino, N-Propylamino oder vorzugsweise N-Ethylamino. Di-niederalkylamino enthält vorzugsweise 1 bis 4 Kohlenstoffatome in jedem Niederalkylteil und bedeutet z. B. N,N-Dimethylamino, N-Methyl-N-ethylamino und vorzugsweise N,N-Diethylamino.

Niederalkylthio-niederalkyl bedeutet vorzugsweise (Methyl, Ethyl oder Propyl)-thio-(methyl, ethyl, propyl oder butyl), vorzugsweise 2-(Methylthio)ethyl.

Niederalkanoyloxy bedeutet vorzugsweise Acetoxy, Propionyloxy oder Pivaloyloxy.

Alkylendioxy bedeutet vorzugsweise Ethylendioxy und vorzugsweise Methylendioxy.

Aryl-niederalkoxy bedeutet vorzugsweise Benzyloxy, Benzyloxy substituiert durch Methyl, Methoxy oder Chlor, und Pyridylmethoxy.

Carboxy-niederalkoxy bedeutet vorzugsweise 1-Carboxyethoxy.

Niederalkoxycarbonyl-niederalkoxy bedeutet vorzugsweise 1-(Ethoxycarbonyl)ethoxy.

Amino-niederalkoxy, Mono-niederalkylamino-niederalkoxy und Diniederalkylamino-niederalkoxy bedeuten vorzugsweise Aminoethoxy, Ethylaminoethoxy bzw. Diethylaminoethoxy.

Niederalkanoyloxyalkoxy bedeutet vorzugsweise Pivaloyloxymethoxy.

Bicycloalkyloxycarbonyl-niederalkoxy bedeutet vorzugsweise Bicyclo-[2.2.1]heptyloxycarbonyl-niederalkoxy, unsubstituiert oder substituiert durch Niederalkyl, vorzugsweise Bicyclo[2.2.1]heptyloxycarbonyl-methoxy, z. B. Bornyloxycarbonyl-methoxy.

Amino-niederalkyl und ω-Amino-niederalkyl bedeuten vorzugsweise Amino(ethyl, propyl oder butyl) bzw. ω-Amino(ethyl, propyl oder butyl).

Halogen bedeutet vorzugsweise Chlor, kann jedoch auch Brom, Fluor oder Iod sein.

Acyliertes Hydroxy bedeutet vorzugsweise Niederalkanoyloxy, z. B. Acetyloxy, Benzoyloxy, am Phenylring durch Niederalkyl, Halogen oder Niederalkoxy, z. B. wie durch Methyl, Chlor bzw. Methoxy substituiertes Benzoyloxy, oder Nicotinoyloxy.

Verethertes Hydroxy bedeutet vorzugsweise Niederalkoxy, z. B. Methoxy, Ethoxy oder t-Butoxy, oder Benzyloxy.

Aryloxy bedeutet vorzugweise Phenoxy oder Phenoxy substituiert durch Niederalkyl, Niederalkoxy oder Halogen, wie Methyl, Methoxy bzw. Chlor.

Arylthio bedeutet vorzugsweise Phenylthio oder durch Niederalkyl, Niederalkoxy oder Halogen, wie Methyl, Methoxy bzw. Chlor substituiertes Phenylthio.

Arylsulfinyl und -sulfonyl bedeutet vorzugsweise Phenylsulfinyl und Phenylsulfonyl oder durch Niederalkyl, Niederalkoxy oder Halogen, wie Methyl, Methoxy bzw. Chlor substituiertes Phenylsulfinyl und Phenylsulfonyl.

Arylamino bedeutet vorzugsweise Anilino ; Aryl-N-niederalkylamino bedeutet vorzugsweise N-Methylanilino.

Acylamino-niederalkyl und ω-Acylamino-niederalkyl bedeuten vorzugsweise Acylamino(ethyl, propyl oder butyl) bzw. ω-Acylamino(ethyl, propyl, oder butyl).

Acylamino bedeutet Niederalkanoylamino, Niederalkoxycarbonylamino, Cycloalkylcarbonylamino, Cycloalkyloxycarbonylamino, Cycloalkylniederalkoxycarbonylamino ; auch Aryl-niederalkanoylamino, Aryl-niederalkoxycarbonylamino, Arylsulfonamido, worin Aryl vorzugsweise Phenyl oder vorzugsweise durch Niederalkyl, Niederalkoxy oder Halogen substituiertes Phenyl bedeutet. Acylamino steht weiter für Aroylamino, worin Aroyl vorzugsweise Benzoyl oder vorzugsweise durch Niederalkyl, Niederalkoxy oder Halogen substituiertes Benzoyl bedeutet ; oder für Nicotinoyl.

Aryl-niederalkoxycarbonylamino bedeutet vorzugsweise Aryl-methoxycarbonylamino, insbesondere Benzyloxycarbonylamino, Benzyloxycarbonylamino, welches am Phenylring durch Niederalkyl, Niederalkoxy oder Halogen, wie Methyl, Methoxy bzw. Chlor substituiert ist, oder Pyridylmethoxycarbonylamino.

Die Salze von Verbindungen der Formel I sind von solchen Verbindungen abgeleitet, die salzbildende Eigenschaften haben. Bevorzugt sind die pharmazeutisch verträglichen Salze.

Pharmazeutisch verträgliche Salze sind vorzugsweise Metall- oder Ammoniumsalze der Verbindungen der Formel I, worin $R^0$ Carboxy bedeutet, oder der Formeln IA-IC, worin $COR^6$ und/oder $COR^7$ Carboxy bedeuten, insbesondere Alkali- oder Erdalkalimetallsalze, z. B. Natrium-, Kalium-, Magnesium- oder Calciumsalze; oder vorteilhafterweise leicht kristallisierende Ammoniumsalze, abgeleitet von Ammoniak oder organischen Aminen, wie Mono-, Di- oder Tri-nieder(alkyl, cycloalkyl oder hydroxyalkyl)aminen, Niederalkylendiaminen oder (Hydroxyniederalkyl oder Arylniederalkyl)alkylammonium-Basen, z. B. Methylamin, Diethylamin, Triethylamin, Dicyclohexylamin, Triethanolamin, Ethylendiamin, Tris-(hydroxymethyl)aminomethan oder Benzyltrimethylammoniumhydroxid. Diese verbindungen der Formel I bilden Säureadditionssalze, bei denen es sich vorzugsweise um solche von therapeutisch verträglichen anorganischen oder organischen Säuren handelt, wie starken Mineralsäuren, z. B. Halogenwasserstoffsäuren, z. B. Chlorwasserstoff- oder Bromwasserstoffsäure; Schwefel-, Phosphor-, Salpeter- oder Perchlorsäure; aliphatischen oder aromatischen Carbon- oder Sulfonsäuren, z. B. Ameisen-, Essig-, Propion-, Bernstein-, Glykol-, Milch-, Aepfel-, Wein-, Glucon-, Citronen-, Ascorbin-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoa-, Nicotin-, Methansulfon-, Ethansulfon-, Hydroxyethansulfon-, Benzolsulfon-, p-Toluolsulfon-, Naphthalinsulfon-, Sulfanil- oder Cyclohexylsulfaminsäure.

Die Verbindungen der Formel I zeigen wertvolle pharmakologische Eigenschaften, z. B. kardiovaskuläre Effekte, welche sie unter anderem aufgrund ihrer Hemmwirkung auf die Bildung von Angiotension II hervorrufen. Diese Wirkung entsteht durch selektive Hemmung des Enzyms, welches das Angiotensin in Säugern umwandelt. Die Verbindungen sind daher nützlich für die Behandlung von Krankheiten, welche auf die Hemmung des Enzyms, das Angiotensin in Säugern, einschliesslich Menschen, umwandelt, ansprechen.

Die Verbindungen der Erfindung zeigen in erster Linie hypotensive/antihypertensive Wirkungen und beeinflussen die Herztätigkeit, u. a. aufgrund ihrer hemmenden Wirkung auf das Enzym, welches das Angiotensin umwandelt. Diese Eigenschaften können durch in-vitro- oder in-vivo-Tierversuche, vorzugsweise an Säugetieren, z. B. Ratten, Katzen, Hunden oder deren isolierten Organen, als Testobjekte nachgewiesen werden. Die Tiere können entweder normotensiv oder hypertensiv, z. B. genetisch spontan hypertensive Ratten, oder renal hypertensive Ratten oder Hunde, und Hunde, denen Natrium entzogen ist, sein. Die Verbindungen können den Versuchstieren enteral oder parenteral, vorzugsweise oral oder intravenös, z. B. in Gelatine-Kapseln oder in Form von Stärke enthaltenden Suspensionen bzw. wässrigen Lösungen, verabreicht werden. Die verwendete Dosis kann in einem Bereich von ungefähr zwischen 0,01 und 100 mg/kg/Tag, vorzugsweise zwischen ungefähr 0,05 und 50 mg/kg/Tag, insbesondere zwischen ungefähr 0,1 und 25 mg/kg/Tag liegen.

Die blutdrucksenkende Wirkung in vivo wird entweder direkt mit einem Katheter, der in die femurale Arterie des Versuchstieres eingeführt ist, oder indirekt durch Sphygmomanometrie am Rattenschwanz, und einem Uebertragungsinstrument registriert. Der Blutdruck wird vor und nach der Verabreichung des Wirkstoffes in mm Hg bestimmt.

So sind die antihypertensiven Wirkungen in spontan hypertensiven Ratten durch indirekte Messung des systolischen Blutdrucks nachweisbar. Wache Ratten werden einzeln in sehr engen Käfigen in eine leicht geheizte Kammer gebracht. Ein Puls-Sensor wird innerhalb einer aufblasbaren Manschette am Schwanz jeder Ratte angebracht. Die Manschette wird periodisch aufgeblasen, um die Schwanz-Arterie zu schliessen. Der Druck in der Manschette wird kontinuierlich reduziert, und der systolische Druck entspricht demjenigen Druck in der Manschette, bei dem die Pulswellen wieder auftreten. Nach der Registrierung von Kontrollwerten des Blutdrucks und der Herzfrequenz werden die Testverbindungen einmal täglich an vier aufeinander folgenden Tagen oral verabreicht. Zusätzliche Blutdruckmessungen werden üblicherweise 2,0, 4,0 und 23,5 Stunden nach einer täglichen Dosis vorgenommen. Die Werte werden mit denjenigen von Ratten, welche lediglich mit der Trägersubstanz behandelt worden sind, verglichen.

Die Verbindungen der Erfindung zeigen nach intravenöser oder oraler Verabreichung eine hemmende Wirkung gegen die durch Angiotensin I hervorgerufene Blutdrucksteigerung auch an normotensiven Ratten. Das Angiotensin I wird durch das genannte Umwandlungsenzym zu der stark blutdrucksteigernden Substanz Angiotensin II hydrolysiert. Die Hemmung des genannten Enzyms blockiert die Bildung von Angiotensin II aus Angiotensin I. Auf diese Weise wird die durch Angiotensin I hervorgerufene Blutdrucksteigerung abgeschwächt.

Der entsprechende Test in vivo wird mit männlichen normotensiven Ratten, welche mit dem Natriumsalz der 5-Ethyl-5-(1-methyl-propyl)-2-thiobarbitursäure narkotisiert sind, durchgeführt. Eine femurale Arterie und eine Wadenvene werden mit je einer Hohlnadel für die direkte Messung des Blutdrucks und für die intravenöse Verabreichung des Angiotensins I und einer erfindungsgemässen Verbindung versehen. Nach der Stabilisierung des basalen Blutdrucks wird die Druckänderung nach drei, in 5-minütigen Intervallen, i. v. durchgeführten Reizungen mit 333 ng/kg Angiotensin I bestimmt.

Solche Druckänderungen werden wieder 5, 10, 15, 30 und 60 Minuten nach intravenöser Verabreichung oder 1, 2, 3 und 4 Stunden nach peroraler Verabreichung der zu prüfenden Verbindung bestimmt und mit den Anfangswerten verglichen. Jede durch die erfindunsgemässen Verbindungen hervorgerufene festgestellte Abnahme vom Ansprechen des Blutdrucks ist ein Hinweis auf die Hemmung des Enzyms, welches das Angiotensin I umwandelt.

Die Hemmung des Enzyms, welches das Angiotensin umwandelt, durch die Verbindungen dieser Erfindung in vitro kann analog zu Biochim. Biophys. Acta 293, 451 (1973) nachgewiesen werden. Gemäss dieser Methode werden die genannten Verbindungen in ungefähr 1 mMol-Konzentrationen in Phosphatpuffer gelöst. Zu 100 µl von Lösungen der Testverbindung in Phosphatpuffer, welche man auf die gewünschte Konzentration verdünnt, gibt man zuerst 100 µl von 5 millimolarem Hippuryl-histidyl-leucin in Phosphatpuffer und dann 50 µl des Angiotensin-umwandelnden Enzyms. Dieses Enzym wird aus Lungen von erwachsenen männlichen Kaninchen in Tris-Puffer, der Kalium- und Magnesiumchlorid und auch Rohrzucker enthält, hergestellt. Die genannten Lösungen werden 30 Minuten bei 37 °C inkubiert. Durch Hinzufügen von 0,75 ml einer 0,6-normalen wässerigen Natriumhydroxidlösung wird die Reaktion dann abgebrochen. Man gibt bei Zimmertemperatur 100 µl einer 0,2 %igen Lösung von o-Phthalaldehyd in Methanol und 10 Minuten danach 100 µl 6n-Chlorwasserstoffsäure dazu. Diese Proben werden in einem Spektrophotometer bei 360 nm mit Wasser verglichen, und ihre optische Dichte wird bestimmt. Diese Werte werden durch einen Konversionsfaktor einer Standard-Kurve angepasst, wobei man die während der genannten Inkubation von 30 Minuten gebildete Histidyl-leucin-Menge in Nanomolen erhält. Die Ergebnisse werden, um den $IC_{50}$-Wert zu bestimmen, gegenüber den Wirkstoffkonzentrationen graphisch als Kurve dargestellt. Der $IC_{50}$-Wert bedeutet diejenige Wirkstoff-Konzentration, welche die Hälfte der Aktivität einer Kontrollprobe, die keinen Wirkstoff enthält, ergibt.

Wegen der vorgenannten vorteilhaften Eigenschaften sind die erfindungsgemässen Verbindungen als spezifische therapeutische Mittel für Säuger, inklusive Menschen, sehr nützlich.

Dementsprechend sind die Verbindungen der Erfindung wertvolle antihypertensive Mittel, insbesondere für die Herabsetzung von hohem Blutdruck (ohne Rücksicht auf die Aetiologie) und/oder bei Herzerkrankungen wie Herzinsuffizienz, und/oder anderen Zuständen, die mit Oedemen oder Ascites einhergehen. Sie können auch zur Herstellung von anderen wertvollen Produkten, insbesondere von entsprechenden pharmazeutischen Präparaten verwendet werden.

Erfindungsgemäss können eine oder beide der Carboxylgruppen der Dicarbonsäuren, d. h. der Verbindungen der Formel IA, IB oder IC, worin $R^6$ und $R^7$ Hydroxy sind, in Form der funktionellen Derivate als Ester oder Amide vorliegen. Diese funktionellen Derivate sind vorzugsweise die Mono- oder Bis-niederalkylester, z. B. Methyl-, Ethyl-, n- oder i-Propyl-, Butyl- oder Benzylester; die Mono- oder Bisamide, die mono- oder di-N-alkylierten Amide, z. B. Mono- oder Diethylamide; die mono- oder disubstituierten Niederalkylester, z. B. die ω-(Amino- Mono- oder Dimethylamino, Carboxy oder Carbethoxy)-(ethyl, propyl oder butyl)-ester. Stark bevorzugte funktionelle Derivate sind die Monoester, z. B. solche, worin einer der Reste $R^6$ und $R^7$ Hydroxy und der andere Niederalkoxy bedeutet.

Derivate der erfindungsgemässen Verbindungen, die Wirkstoff-Vorstufen darstellen, z. B. jegliche pharmazeutisch verträglichen Ester und Amide der erfindungsgemässen Mono- oder Dicarbonsäuren, die durch Solvolyse oder unter physiologischen Bedingungen in die vorliegenden Carbonsäuren übergeführt werden können, z. B. die vorstehend genannten Ester und Amide, stellen einen speziellen Gegenstand der Erfindung dar.

Diese Ester sind vorzugsweise z. B. geradkettige oder verzweigte unsubstituierte oder in geeigneter Weise substituierte Niederalkylester, wie die Pivaloyloxymethyl-, Bornyloxycarbonylmethyl-, Benzyl-, Pyridylmethyl-, α-Carboxyethyl- oder in geeigneter Weise veresterte α-Carboxyethylester, und dergleichen.

Diese Amide sind vorzugsweise z. B. einfache primäre und sekundäre Amide und Amide, abgeleitet von Aminosäuren oder deren Derivaten, wie die von Alanin, Phenylalanin und dergleichen abgeleiteten Amide.

Insbesondere betrifft die Erfindung die Verbindungen der Formel IA

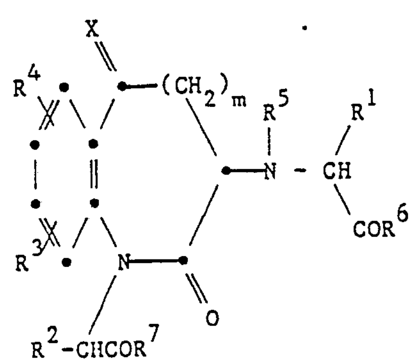

(IA)

worin der carbocyclische Ring auch perhydriert sein kann; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Arylthio-niederalkyl, Aryl-N-niederalkylamino-niederalkyl oder Arylamino-niederalkyl bedeutet; $R^2$ und $R^5$ Wasserstoff oder Niederalkyl bedeuten; $R^3$ und $R^4$ Wasserstoff, Niederalkyl, Niederalkoxy, Niederalkanoyloxy, Hydroxy, Halogen oder Trifluormethyl bedeuten oder $R^3$ und $R^4$ gemeinsam Niederalkylendioxy darstellen; X Oxo, zwei Wasserstoffatome, oder eine Hydroxygruppe oder acylierte Hydroxygruppe und ein Wasserstoff bedeutet; m für 2 oder 3 steht; $R^6$ und $R^7$ unabhängig voneinander Hydroxy, Amino, Mono- oder Di-niederalkylamino, Niederalkoxy, Aryl-niederalkoxy, Niederalkanoyloxymethoxy, (Amino, Mono- oder Di-niederalkylamino, Carboxy oder Niederalkoxycarbonyl)-niederalkoxy bedeuten; oder deren pharmazeutisch verträgliche Salze.

Bevorzugte Ausführungsformen der Erfindung betreffen Verbindungen der Formel IA, worin $R^1$ Wasserstoff, Niederalkyl, Amino-niederalkyl, Aryl, Aryl-niederalkyl, Cycloalkyl-niederalkyl ist; und worin innerhalb der obigen Definitionen Aryl unsubstituiertes oder durch Niederalkyl, Niederalkoxy, Niederalkylendioxy, Niederalkanoyloxy, Hydroxy, Halogen oder Trifluormethyl mono- oder disubstituiertes Phenyl bedeutet; und Cycloalkyl 3 bis 8 Kohlenstoffatome enthält; X, m, $R^2$ bis $R^7$ die obigen Bedeutungen haben; oder ihre pharmazeutisch verträglichen Salze; oder die genannten Verbindungen, worin der carbocyclische Ring perhydriert ist.

Weitere bevorzugte Verbindungen der Erfindung sind diejenigen der Formel IA, worin $R^1$ Aryl-niederalkyl ist, worin Aryl Indolyl bedeutet, und auch für Carboxy-niederalkyl, Niederalkoxycarbonylniederalkyl, Hydroxy-niederalkyl, Niederalkylthio-niederalkyl, Acylamino-niederalkyl; Aryloxy-niederalkyl oder Arylthio-niederalkyl steht; X, m und $R^2$ bis $R^7$ die obigen Bedeutungen haben; oder ihre pharmazeutische verträglichen Salze; oder die genannten Verbindungen, worin der carbocyclische Ring perhydriert ist.

Besonders bevorzugte Verbindungen der Erfindung sind diejenigen der Formel IA, worin $R^1$ Wasserstoff, Niederalkyl, Amino-niederalkyl, Acylamino-niederalkyl, Aryl-niederalkyl, worin Aryl unsubstituiertes Phenyl oder durch Niederalkyl, Hydroxy, Niederalkoxy, Niederalkylendioxy, Niederalkanoyloxy, Halogen oder Trifluormethyl mono- oder disubstituiertes Phenyl ist, bedeutet; $R^3$ und $R^4$ Wasserstoff, Niederalkyl, Niederalkoxy, Halogen oder Trifluormethyl bedeuten oder $R^3$ und $R^4$ gemeinsam für Alkylendioxy stehen; $R^6$ und $R^7$ unabhängig voneinander Hydroxy, Amino, Niederalkoxy, Phenyl-niederalkoxy, Niederalkoxycarbonyl-niederalkoxy bedeuten; $R^2$, $R^5$, X und m die obigen Bedeutungen haben, oder ihre pharmazeutische verträglichen Salze; oder die genannten Verbindungen, worin der carbocyclische Ring perhydriert ist.

Besonders wertvoll sind Verbindungen der Formel IA, worin $R^1$ Wasserstoff, Niederalkyl, Amino-niederalkyl, Aryl-niederalkoxy-carbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ und $R^4$ für Wasserstoff, Niederalkyl, Niederalkoxy, Halogen oder Trifluormethyl stehen, oder $R^3$ und $R^4$ gemeinsam Niederalkylendioxy bedeuten; X für Oxo, zwei Wasserstoffatome, oder eine Hydroxy- oder Niederalkanoyloxygruppe und ein Wasserstoffatom steht; m 2 oder 3 bedeutet; $R^6$ und $R^7$ unabhängig voneinander für Hydroxy, Amino, Niederalkoxy, Phenyl-niederalkoxy oder Niederalkoxycarbonyl-niederalkoxy stehen; oder ihre pharmazeutisch verträglichen Salze; oder die genannten Verbindungen, worin der carbocyclische Ring perhydriert ist.

Insbesondere bevorzugt sind Verbindungen der Formel IA, worin $R^1$ Wasserstoff, Niederalkyl, ω-Amino-niederalkyl, ω-Arylmethoxycarbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ für Wasserstoff steht; $R^4$ Wasserstoff, Niederalkyl, Niederalkoxy, Halogen oder Trifluormethyl bedeutet; X für Oxo, zwei Wasserstoffatome, oder eine Hydroxy- oder Niederalkanoyloxygruppe und ein Wasserstoffatom steht, m 2 oder 3 bedeutet; $R^6$ und $R^7$ unabhängig voneinander für Hydroxy, Amino, Niederalkoxy, Phenylniederalkoxy, Niederalkoxycarbonyl-niederalkoxy stehen; oder ihre pharmazeutisch verträglichen Salze; oder die genannten Verbindungen, worin der carbocyclische Ring perhydriert ist.

Vor allem wertvoll sind Verbindungen der Formel IA, worin $R^1$ die Bedeutung hat von Wasserstoff, Methyl, Ethyl, Isopropyl, ω-Aminopropyl, ω-Aminobutyl, ω-(Benzyloxycarbonylamino)propyl, ω-(Benzyloxycarbonylamino)butyl, Aryl-(methyl, ethyl, propyl), worin Aryl unsubstituiertes Phenyl oder durch Methyl, Hydroxy, Methoxy, Methylendioxy, Acetoxy, Chlor oder Trifluormethyl monosubstituiertes Phenyl bedeutet; $R^2$ und $R^5$ für Wasserstoff oder Methyl stehen; $R^3$ und $R^4$ Wasserstoff, Methoxy, Methyl, Chlor oder Trifluormethyl bedeuten; X für Oxo, zwei Wasserstoffatome, oder eine Hydroxy- oder Acetoxygruppe und ein Wasserstoffatom steht; m 2 oder 3 bedeutet; $R^6$ und $R^7$ unabhängig voneinander Hydroxy, Amino, Ethoxy, Methoxy, Benzyloxy, Ethoxycarbonylmethoxy oder Pivaloyloxymethoxy bedeuten; oder ihre pharmazeutisch verträglichen Salze; oder die genannten Verbindungen, worin der carbocyclische Ring perhydriert ist.

Ueberragend wertvoll sind Verbindungen der Formel IB

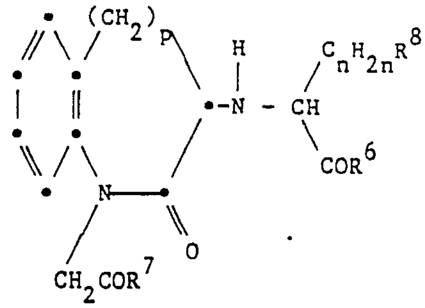

(IB)

worin n eine ganze Zahl von 1 bis 4 bedeutet, p für die ganze Zahl 3 oder 4 steht, $R^8$ Wasserstoff, Amino, Benzyloxycarbonylamino, unsubstituiertes Phenyl oder Phenyl, monosubstituiert durch Niederalkyl, Niederalkoxy, Niederalkanoyloxy, Halogen, Hydroxy oder Trifluormethyl, bedeutet; $R^6$ und $R^7$ unabhängig voneinander Hydroxy, Niederalkoxy mit bis zu 4 Kohlenstoffatomen, Benzyloxy oder Amino bedeuten, oder ihre pharmazeutische verträglichen Salze, oder die genannten Verbindungen, worin der carbocyclische Ring perhydriert ist.

Besonders wertvoll sind Verbindungen der Formel IB, worin $C_nH_{2n}$ Ethylen darstellt, $R^8$ Phenyl oder Phenyl, monosubstituiert durch Niederalkoxy mit bis zu 4 Kohlenstoffatomen, Niederalkyl mit bis zu 4 Kohlenstoffatomen, Halogen oder Trifluormethyl, darstellt, $R^6$ und $R^7$ unabhängig voneinander Hydroxy oder Niederalkoxy mit bis zu 4 Kohlenstoffatomen bedeuten; p die ganze Zahl 3 oder 4 bedeutet, oder ihre pharmazeutisch verträglichen Salze; oder die genannten Verbindungen, worin der carbocyclische Ring perhydriert ist.

Weitere bevorzugte Verbindungen sind solche der Formel IB, worin $C_nH_{2n}$ n-Propylen oder n-Butylen bedeutet; $R^8$ für Amino oder Benzyloxycarbonylamino steht; $R^6$ und $R^7$ unabhängig voneinander Hydroxy oder Niederalkoxy mit bis zu 4 Kohlenstoffatomen bedeuten; p für die ganze Zahl 3 oder 4 steht; oder ihre pharmazeutisch verträglichen Salze; oder die genannten Verbindungen, worin der carbocyclische Ring perhydriert ist.

Es sind auch bevorzugt die obigen Verbindungen der Formel IB, worin $(CH_2)_p$ Propylen bedeutet.

Die vorliegende Erfindung betrifft auch die Stereoisomeren der Verbindungen der Formel I. Eine Anzahl Racemate ist erhältlich, wenn z. B. in der Formel IA zumindest einer der Reste $R^1$ und $R^2$ nicht Wasserstoff und/oder X H(OH) oder H(acyliertes OH) bedeutet. Weiter können die Verbindungen der Erfindung, in welchen der carbocyclische Ring perhydriert ist, je nach der Ringverknüpfung als cis- oder trans-Isomere vorliegen.

Die einzelnen Enantiomeren dieser Racemate können ihrerseits erhalten werden. Bestimmte spezifische Vertreter dieser Isomeren sind als Inhibitoren für das Angiotensin-umwandelnde Enzym bevorzugt.

Bevorzugt sind die oben genannten Verbindungen, in welchen das asymmetrische Ringkohlenstoffatom (in 3-Stellung), welches die substituierte Aminogruppe trägt, die (S)-Konfiguration aufweist. Weiter bevorzugte Verbindungen sind solche, in welchen das asymmetrische Kohlenstoffatom der Seitenkette, welches die Gruppe $COR^6$ trägt, die (S)-Konfiguration hat.

Herausragend sind Verbindungen der Formel IC

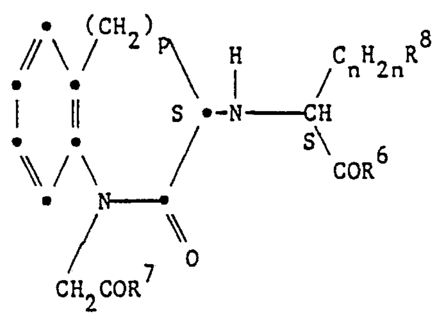

(IC)

worin S die Chiralität bedeutet, n eine ganze Zahl von 1 bis 4 bedeutet; p für die ganze Zahl 3 oder 4 steht, $R^8$ Wasserstoff, Amino, Benzyloxycarbonylamino, unsubstituiertes Phenyl oder durch Niederalkyl, Niederalkoxy, Niederalkanoyloxy, Halogen, Hydroxy oder Trifluormethyl monosubstituiertes Phenyl bedeutet; $R^6$ und $R^7$ unabhängig voneinander Hydroxy, Niederalkoxy mit bis zu 4 Kohlenstoffatomen, Benzyloxy oder Amino bedeuten; oder ihre pharmazeutisch verträglichen Salze.

Bevorzugt sind weiter die genannten Verbindungen der Formeln IB und IC, in welchen $(CH_2)_p$ Propylen bedeutet.

Die Verbindungen der Formel I gemäss der Erfindung können in an sich bekannter Weise hergestellt werden, indem man z. B.

a) in eine Verbindung der Formel

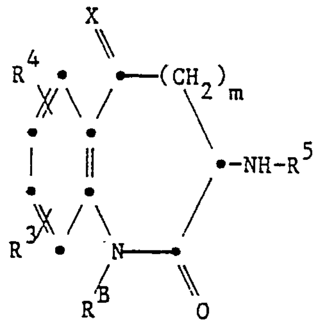

(II)

worin der carbocyclische Ring auch perhydriert sein kann und worin X, $R^B$, $R^3$, $R^4$, $R^5$ und m die vorstehend angegebenen Bedeutungen besitzen, $R^1$ durch Alkylierung mit einer Verbindung der Formel

$$R^A—Z \quad \text{(IIIA)},$$

worin Z eine reaktive veresterte Hydroxygruppe bedeutet und $R^A$ die vorstehend angegebenen Bedeutungen besitzt, oder mit einer Verbindung der Formel

$$R^1—CO—R^0 \quad \text{(IV)},$$

worin $R^1$ und $R^0$ die oben angegebenen Bedeutungen haben, in Gegenwart eines Reduktionsmittels, unter vorübergehendem Schutz etwaiger primärer und sekundärer Aminogruppen und/oder gegebenenfalls Hydroxy- und/oder Oxogruppen, die in irgendeinem der Reste X, $R^B$, $R^3$ und $R^4$ und/oder im Alkylierungsmittel anwesend sein können, einführt, oder

b) eine Verbindung der Formel

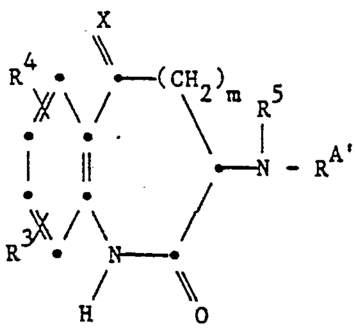

(V)

worin der carbocyclische Ring auch perhydriert sein kann und worin X, $R^3$, $R^4$, $R^5$ und m die vorstehend angegebenen Bedeutungen besitzen und $R^{A'}$ Wasserstoff oder $R^A$ wie vorstehend definiert ist, mit einer Verbindung der Formel

$$R^B—Z \quad \text{(IIIB)},$$

worin Z eine reaktive veresterte Hydroxygruppe bedeutet und $R^B$ die vorstehend angegebenen Bedeutungen besitzt, alkyliert, wobei man vorübergehend etwaige primäre und sekundäre Aminogruppen und/oder gegebenenfalls Hydroxy- und/oder Oxogruppen, die in irgendeinem der Reste X, $R^{A'}$, $R^B$, $R^3$ und $R^4$ anwesend sein können, schützt, oder

c) eine Verbindung der Formel

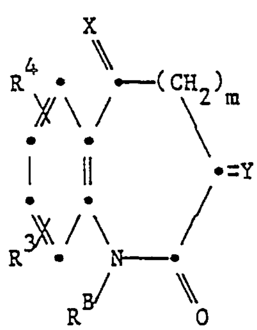

(VI)

worin der carbocyclische Ring auch perhydriert sein kann und worin Y Oxo, Dichloro, oder eine reaktive veresterte Hydroxygruppe Z gemeinsam mit Wasserstoff bedeutet und X, $R^B$, $R^3$, $R^4$ und m die vorstehend angegebenen Bedeutungen besitzen, mit einem Amin der Formel

$$R^A—NH—R^5 \qquad (VII),$$

worin $R^A$ und $R^5$ die vorstehend angegebenen Bedeutungen besitzen, kondensiert, wobei, wenn Y Oxo oder Dichloro bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel

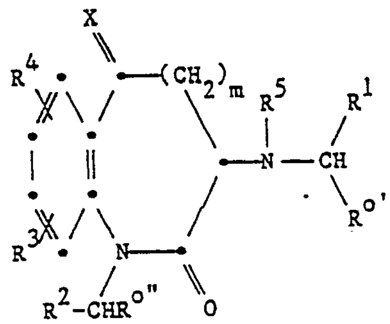

(VIII)

worin der carbocyclische Ring auch perhydriert sein kann und worin X, $R^1$ bis $R^5$ und m die vorstehend angegebenen Bedeutungen besitzen, eines der Symbole $R^{0'}$ und $R^{0''}$ Cyano ist und das andere Cyano oder $R^0$ wie vorstehend definiert ist, die Cyanogruppe(n) einer Solvolyse unterzieht, oder

e) eine Verbindung der Formel

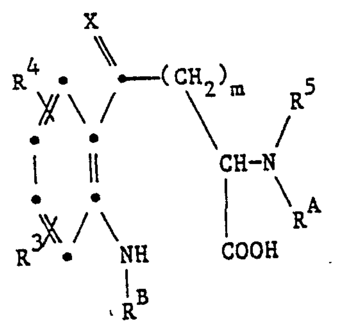

(IX)

worin der carbocyclische Ring auch perhydriert sein kann und worin X, $R^A$, $R^B$, $R^3$, $R^4$, $R^5$ und m die vorstehend angegebenen Bedeutungen besitzen, oder einen Ester hiervon cyclisiert, oder

f) eine Verbindung, die strukturell mit einer Verbindung der vorstehend angegebenen Formel I identisch ist, mit Ausnahme dessen, dass sie eine oder zwei zusätzliche Doppelbindung(en) im heterocyclischen Ring und/oder eine zusätzliche Doppelbindung zwischen dem exocyclischen Stickstoffatom und dem C-3-Kohlenstoffatom oder dem benachbarten Kohlenstoffatom innerhalb der Gruppe $R^A$ aufweist, mit einem Reduktionsmittel behandelt, um diese Doppelbindung(en) zu sättigen, und gewünschtenfalls eine erhaltene Verbindung der Formel I in eine andere Verbindung der Formel I umwandelt und/oder, gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt, und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

Eine reaktive veresterte Hydroxygruppe Z ist eine mit einer starken organischen Säure veresterte Hydroxygruppe, z. B. einer aliphatischen oder aromatischen Sulfonsäure (wie einer Niederalkansulfonsäure, insbesondere Methansulfonsäure, Trifluormethansulfonsäure, insbesondere Benzolsulfonsäure, p-Toluolsulfonsäure, p-Brombenzolsulfonsäure und p-Nitrobenzolsulfonsäure) oder mit einer starken anorganischen Säure, wie insbesondere Schwefelsäure, oder einer Halogenwasserstoffsäure, wie Chlorwasserstoffsäure oder am meisten bevorzugt Iodwasserstoffsäure oder Bromwasserstoffsäure, veresterte Hydroxygruppe.

Eine substitutive Alkylierung gemäss der vorliegenden Erfindung wird unter herkömmlichen allgemeinen Bedingungen bei Temperaturen im Bereich von etwa 0 °C bis zur Siedetemperatur der Reaktionsmischung, vorzugsweise bei Temperaturen zwischen Raumtemperatur und etwa 100 °C,

durchgeführt. Die Umsetzung findet vorteilhaft in Gegenwart eines Lösungsmittels, das im Hinblick auf die Reaktanten inert ist, statt, wie in Gegenwart eines chlorierten Niederalkans (z. B. Chloroform oder Methylenchlorid), eines acyclischen oder cyclischen Ethers (z. B. Diethylether, 1,2-Dimethoxyethan, Dioxan oder Tetrahydrofuran) und insbesondere eines tertiären Amids mit niedrigem Molekulargewicht (z. B. N,N-Dimethylformamid, N,N-Dimethylacetamid, N-Methylpyrrolidon, N-Ethylpiperidon und Hexamethylphosphorsäuretrisamid). Vorteilhafterweise wird die während der Reaktion freigesetzte starke Säure HZ durch Zugabe eines säurebindenden Mittels gebunden, wie vorzugsweise eines anorganischen Säurefängers, wie eines Alkalimetallbicarbonats, -carbonats oder -hydroxids, eines organischen quaternären Ammoniumsalzes (z. B. eines Tetrabutylammoniumsalzes) oder einer organischen tertiären Base, wie Triethylamin, N-Ethylpiperidin, Pyridin oder Chinolin.

Eine erfindungsgemässe Alkylierung kann auch unter an sich bekannten und im Stand der Technik verwendeten Bedingungen einer reduktiven Alkylierung durchgeführt werden. Bei Durchführung dieser Alkylierung werden die Ausgangsstoffe gleichzeitig oder in einem nachfolgenden Schritt mit einem Reduktionsmittel umgesetzt. Unter Reduktionsmitteln, die gleichzeitig mit dem Alkylierungsmittel verwendet werden, sollen Ameisensäure und komplexe Metallhydride, wie Natriumcyanborhydrid, erwähnt werden ; unter den Reduktionsmitteln, die überwiegend in einer getrennten nachfolgenden Arbeitsstufe, d. h. zur Reduktion eines vorher gebildeten Imins (Schiff'sche Base) verwendet werden, sollen Diboran und komplexe Metallhydride, wie Natriumborhydrid und Natriumcyanborhydrid, erwähnt werden, die vorteilhaft dem primären Reaktionsgemisch ohne Isolierung eines Zwischenprodukts, z. B. des Imins, zugegeben werden. In diesem Fall wird die Alkylierung vorteilhaft in einem gegenüber dem Reduktionsmittel inerten organischen Lösungsmittel durchgeführt, wie in einem aliphatischen oder cyclischen Ether (wie Diethylether, Diisopropylether, 1,2-Dimethoxyethan, Dioxan oder Tetrahydrofuran) oder in einem aliphatischen Alkohol (wie Methanol, Ethanol, Isopropylalkohol, Glykol, Glykolmonomethylether oder Diethylenglykol), vorzugsweise bei etwa 0 bis 80 °C. Ein wesentliches Reduktionsmittel, das sowohl gleichzeitig als auch anschliessend verwendet werden kann, ist Wasserstoff, insbesondere katalytisch aktivierter Wasserstoff. Die Katalysatoren sind diejenigen, die herkömmlich als Hydrierungskatalysatoren eingesetzt werden, vorzugsweise diejenigen der Klasse der Edelmetalle (wie Palladium, Platin und Rhodium) auf einem Träger (wie Calciumcarbonat, Aluminiumoxid oder Bariumsulfat), in feindisperser Suspension ohne Träger oder in Form von Komplexen in homogener Phase. Auch feindispergierte Uebergangsmetalle, z. B. Raney-Metalle, insbesondere Raney-Nickel, sind sehr geeignete Katalysatoren für die reduktive Alkylierung. Die speziellen Reaktionsbedingungen hängen in grossem Ausmass von dem jeweiligen Hydrierungskatalysator und dessen exakter Aktivität ab und weichen von den allgemein für die Hydrierung bekannten nicht ab. Temperaturen im Bereich von Raumtemperatur bis zu etwa 150 °C und Wasserstoffdrücke im Bereich von Atmosphärendruck bis zu etwa 300 Atmosphären sind gemäss den Standardverfahren des Standes der Technik anwendbar. Zusätzlich zu den vorstehend im Zusammenhang mit der Hydridreduktion erwähnten inerten Lösungsmitteln können auch niedermolekulare Amide, insbesondere tertiäre Amide (wie N,N-Dimethylformamid, N,N-Dimethylacetamid, N-Methylpyrrolidon, N-Ethylpiperidon, Hexamethylphosphorsäure-trisamid), auch Formamid und Acetamid als geeignete Lösungsmittel verwendet werden. Spezielle Massnahmen müssen bei Ausgangsmaterialien angewandt werden, die eine leicht reduzierbare funktionelle Gruppe, wie die Oxogruppe (X = O), aufweisen ; um diese Gruppen zu erhalten, müssen selektive Reduktionsbedingungen, wie sie aus dem Stand der Technik bekannt sind, angewandt werden, oder wenn eine gleichzeitige Reduktion dieser Gruppen erwünscht oder erforderlich ist, werden dementsprechend energische Reagentien und/oder Bedingungen angewandt.

Die oben genannten im voraus gebildeten Imine werden vorzugsweise durch Kondensation entsprechender Ausgangsstoffe in einem inerten Lösungmittel, z. B. Toluol oder Methylenchlorid, in erster Linie in Gegenwart von Dehydratisierungskatalysatoren, z. B. Bortrifluoridetherat, p-Toluolsulfonsäure oder Molekularsieben, hergestellt.

Im Falle der Reaktanten der Formeln IIIA, IIIB, IV und VII, welche eine freie Carboxylgruppe $R^0$ enthalten, kann ein geeignetes Carboxylatsalz, vorzugsweise in situ, vor der Kondensation mit den nachfolgend ausführlich genannten, gewünschten Zwischenprodukten hergestellt werden.

In jedem der Alkylierungsverfahren müssen primäre und sekundäre Aminogruppen in den Ausgangsmaterialien, mit Ausnahme der zu alkylierenden Aminogruppe, in einer vorübergehend geschützten Form während der Alkylierung vorliegen. Geeignete Schutzgruppen sowie Verfahren zu deren Einführung und Abspaltung sind aus dem Stand der Technik, der bis in die Einzelheiten ausgearbeitet ist, insbesondere als allgemeine Methoden für die Synthese von Peptiden, bekannt, vergl. Houben-Weyl : Methoden der Organischen Chemie, 4. Ausgabe, Band 15/I und II, E. Wünsch (Herausgeber) : Synthese von Peptiden (Georg Thieme-Verlag, Stuttgart, 1974). Die nähere Auswahl der Schutzgruppen hängt von dem speziellen Zweck ab, wobei es erforderlich ist, insbesondere die speziellen Eigenschaften der jeweiligen Ausgangsmaterialien und die Reaktionsbedingungen der jeweiligen Verfahrens mit zu berücksichtigen. Im Fall von verschiedenen zu schützenden funktionellen Gruppen können vorteilhafte Kombinationen ausgewählt werden. Vorzugsweise werden z. B. ähnliche oder besser identische Aminoschutzgruppen sowohl in den Resten $R^0$ als auch im Rest $R^1$ verwendet und gleichzeitig im Anschluss an die Alkylierung abgespalten.

Geeignet als Aminoschutzgruppen sind insbesondere Aminoschutzgruppen, die durch Reduktion

entfernt werden können, wie z. B. insbesondere diejenigen vom Benzyloxycarbonyl-Typ, bei denen die Benzyloxycarbonylgruppe an dem aromatischen Teil durch Halogenatome, Niederalkoxygruppen und/ oder Niederalkylreste und insbesondere durch Nitrogruppen, substituiert sein kann, wie die p-Chlor- und p-Brombenzyloxycarbonyl-, p-Methoxybenzyloxycarbonyl-, p-Methylbenzyloxycarbonyl- und insbesondere p-Nitrobenzyloxycarbonylgruppe, oder alternativ die Isonicotinyloxycarbonylgruppe. Eine vorteilhafte Aminoschutzgruppe ist eine Ethoxycarbonylgruppe, die in der β-Stellung eine durch drei Kohlenwasserstoffreste substituierte Silylgruppe enthält, wie Triphenylsilyl, Dimethyl-tert.-butylsilyl oder insbesondere Trimethylsilyl. Eine β-(Trihydrocarbylsilyl)-ethoxycarbonylgruppe dieses Typs, wie eine β-(Tri-niederalkylsilyl)-ethoxycarbonylgruppe, z. B. insbesondere β-(Trimethylsilyl)-ethoxycarbonyl, bildet mit der zu schützenden Aminogruppe eine entsprechende β-Trihydrocarbylsilylethoxycarbonylaminogruppe (z. B. die β-Trimethylsilylethoxycarbonylaminogruppe), die unter sehr speziellen, sehr milden Bedingungen durch Einwirken von Fluorid-Ionen abgespalten werden kann.

Es ist auch möglich, Gruppen zu verwenden, die durch Acidolyse abgespalten werden können, wie die tert.-Butoxycarbonylgruppen und analoge Gruppen, ebenso diejenigen des Aralkyl-Typs, wie Benzhydryl, Di-(4-Methoxy)-benzhydryl und Triphenylmethyl (Trityl), oder bestimmte Aralkoxycarbonylgruppen vom 2-(p-Biphenylyl)-2-propoxycarbonyl-Typ, die in der CH-PS 509 266 beschrieben werden. Es sei bemerkt, dass sich von Estern der Kohlensäure ableitende Schutzgruppen in den meisten Fällen auch durch basische Hydrolyse abspaltbar sind.

Für den fakultativen vorübergehenden Schutz der Hydroxygruppen können mit Vorteil Schutzgruppen verwendet werden, die durch Reduktion abgespalten werden können, vgl. den vorstehend zitierten Text (Houben-Weyl), und auch Gruppen, die durch Acidolyse entfernt werden können, wie 2-Tetrahydropyranyl, tert.-Butoxycarbonyl und tert.-Butyl. Bevorzugte Hydroxyschutzgruppen, die durch Reduktion abgespalten werden können, sind biespielsweise Benzylgruppen, die an dem aromatischen Teil durch Halogen, Niederalkyl, Niederalkoxy und/oder insbesondere Nitro, -insbesondere die 4-Nitrobenzylgruppe-, substituiert sein können. Es ist auch möglich, Acylgruppen, die unter schwach basischen Bedingungen abgespalten werden können, wie Formyl oder Trifluoracetyl, zu verwenden.

Für den fakultativen Schutz der Oxogruppen werden diese vorzugsweise als Ketale, insbesondere als Ketale abgeleitet von Niederalkanolen, wie Methanol oder Ethanol, oder vorteilhaft von Ethylenglykol, oder als entsprechende Thioketale, vorzugsweise als solche von 1,2-Ethandithiol, geschützt. Alle diese Gruppen können unter den nachstehend angegebenen Bedingungen Oxogruppen freisetzen.

Die anschliessende Abspaltung der Schutzgruppen gemäss der Erfindung hängt von deren Natur ab und wird in jedem Fall in an sich bekannter herkömmlicher Weise durchgeführt, wobei die allgemeinen Eigenschaften des sich ergebenden Produkts in Betracht gezogen werden. Wenn die Schutzgruppen für Amino, Hydroxy und Oxo so ausgewählt worden sind, dass sie unter ähnlichen Bedingungen abgespalten werden können (insbesondere bevorzugt sind die durch Acidolyse ober bei Amino und Hydroxy durch Reduktion abspaltbaren Gruppen, die bereits im einzelnen erwähnt worden sind), werden alle diese Schutzgruppen mit Vorteil in einem einzigen Arbeitsgang abgespalten, in speziellen Fällen ist es jedoch möglich, verschiedene Typen von Gruppen zu verwenden und jede von Ihnen einzeln abzuspalten.

Die Gruppen, die durch Reduktion abgespalten werden können, insbesondere diejenigen, die halogenierte Niederalkylreste (z. B. 2,2,2-Trichlorethylreste), Isonicotinylreste (z. B. Isonicotinyloxycarbonyl), und insbesondere substituierte Benzylreste, vor allem 4-Nitrobenzylreste jeglicher Art enthalten, werden vorzugsweise durch Reduktion mit Zink, gewöhnlich in Gegenwart einer Säure, vorzugsweise Essigsäure, und mit oder ohne Zugabe eines inerten organischen Lösungsmittels, gewöhnlich bei Raumtemperatur abgespalten. Die Abspaltung einer Schutzgruppe durch saure Hydrolyse (Acidolyse) wird im Fall von Gruppen des tert.-Butyl-Typs mit Hilfe von Chlorwasserstoff, Fluorwasserstoff oder Trifluoressigsäure und im Fall von säureempfindlichen Schutzgruppen hauptsächlich mit Hilfe einer niederen aliphatischen Carbonsäure, wie Ameisensäure und/oder Essigsäure, in Gegenwart von Wasser und gegebenenfalls eines polyhalogenierten Niederalkanols oder Niederalkanons, wie 1,1,1,3,3,3-Hexafluorpropan-2-ol oder Hexafluoraceton, durchgeführt. Auf diese Weise ist es beispielsweise möglich, eine N-Tritylgruppe durch eine organische Säure, wie Ameisensäure, Essigsäure, Chloressigsäure oder Trifluoressigsäure, in wässrigem oder absolutem Trifluorethanol als Lösungsmittel (vgl. DE-OS 23 46 147) oder durch wässrige Essigsäure zu spalten, die tert.-Butoxycarbonylgruppe durch Trifluoressigsäure oder Chlorwasserstoffsäure abzuspalten und die 2-(p-Biphenylyl)-isopropoxycarbonylgruppe durch wässrige Essigsäure oder beispielsweise durch ein Gemisch von Eisessig, Ameisensäure (Konzentration von 82,8 %) und Wasser (7 : 1 : 2) oder gemäss dem Verfahren in der DE-OS 23 46 147 zu entfernen. Die β-Silylethylestergruppen werden vorzugsweise durch Fluorid-Ionen-abgebende Reagentien, beispielsweise Fluoride von quaternären organischen Basen, wie Tetraethylammoniumfluorid, abgespalten.

Ketalisierte und thioketalisierte Oxogruppen werden durch Acidolyse mit üblichen starken anorganischen Säuren oder mit Oxalsäure in Gegenwart von Wasser, die letztgenannten vorteilhaft durch Behandlung mit einem schwefelbindenden Mittel, z. B. einem Quecksilber (II)-Salz und/oder Cadmiumcarbonat in freie Oxogruppen übergeführt.

Schutzgruppen, die gegenüber basischen Bedingungen instabil sind, beispielsweise Formyl, Trifluoracetyl und Kohlensäureestergruppen, können vorsichtig durch Einwirken einer wässrigen Natrium- oder Kaliumbicarbonat- oder -carbonatlösung oder auch durch Einwirken von wässrigem Ammoniak in einem organischen Lösungsmittel, gewöhnlich bei Raumtemperatur, entfernt werden. Die Schutzgruppen

werden vorzugsweise unter den Reaktionsbedingungen der Beispiele oder unter analogen Bedingungen abgespalten.

Verfahren a) Die Kondensation der Amine der Formel II mit den bekannten α-Ketosäure-Derivaten der Formel IV (vgl. Chem. Ber. 31, 551, 3133) durch reduktive N-Alkylierung wird unter aus dem Stand der Technik bekannten Bedingungen, z. B. durch katalytische Hydrierung mit Wasserstoff in Gegenwart von Platin-, Palladium- oder Nickel-Katalysatoren oder mit chemischen Reduktionsmitteln, wie einfachen oder komplexen Leichtmetallhydriden, vorteilhaft einem Alkalimetallcyanborhydrid, wie Natriumcyanborhydrid, durchgeführt. Die reduktive Aminierung mit einem Alkalimetallcyanborhydrid wird vorzugsweise in einem inerten Lösungsmittel, z. B. Methanol oder Acetonitril, vorteilhaft in Gegenwart einer Säure, z. B. Chlorwasserstoffsäure oder Essigsäure, bei einer Temperatur zwischen etwa 0 und 50 °C, vorzugsweise Raumtemperatur, durchgeführt.

Die Alkylierung der Amine der Formel II mit einem Reagens der Formel IIIA, wie es aus dem Stand der Technik gut bekannt ist, wird mit oder ohne basische Katalysatoren, wie Triethylamin oder Kaliumcarbonat in einem inerten Lösungsmittel durchgeführt.

Die Ausgangsmaterialien der Formeln IIIA und IV sind bekannt, oder wenn sie unbekannt sind, können sie auf einfache Weise nach herkömmlichen Syntheseverfahren hergestellt werden. Die Ausgangsmaterialien der Formel II können nach herkömmlichen Syntheseverfahren erhalten werden und mit Vorteil in der Weise, die nachstehend eingehender für die speziellen Zwischenprodukte beschrieben und veranschaulicht ist.

Verbindungen der Formel II können durch Kondensation, unter basischer Katalyse, einer Verbindung der Formel

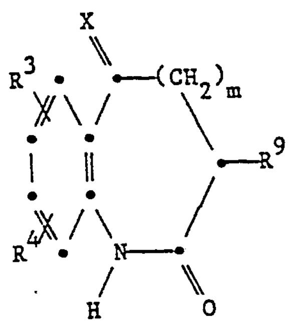

(X)

oder eines Perhydro-Derivates davon, worin $R^3$, $R^4$ und m die vorher angegebenen Bedeutungen haben, und $R^9$ Amino, Niederalkylamino, Azido oder Acylamino, z. B. Niederalkanoylamino oder -alkoxycarbonylamino, bedeutet, mit einer Verbindung der Formel

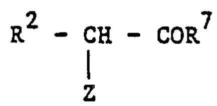

(III'B),

worin $R^2$ und $R^7$ die vorher angegebenen Bedeutungen haben; und Z eine reaktive veresterte Hydroxygruppe bedeutet; und gegebenenfalls Reduktion, Hydrogenolyse, Hydrolyse oder Alkylierung des erhaltenen Zwischenprodukts, erhalten werden.

Die Verbindung der Formel X werden aus den entsprechenden gegebenenfalls substituierten und/oder derivatisierten 3,4,5,6-Tetrahydro-1-benzazocin-2-1H-on- oder aus entsprechenden Benzazonin-Derivaten erhalten. Diese ihrerseits werden aus dem entsprechenden Cyclopent-[b] indol oder 1,2,3,4-Tetrahydrocarbazol durch Periodat-Oxidation (J. Chem. Soc. 1975, 1280; J. Am. Chem. Soc. 88, 1049 (1966)) hergestellt. Die Keto-Funktion kann zum Alkohol, z. B. mit Natriumborhydrid oder durch katalytische Hydrierung, reduziert werden. Die Alkohol-Funktion kann durch Ueberführung in einen Ester, z. B. in ein O-Acetyl-Derivat oder in ein Addukt mit Dicyclohexylcarbodiimid und nachfolgende Hydrogenolyse bei erhöhter Temperatur und erhöhtem Druck, eliminiert werden.

Die genannten Tetrahydro-1-benzazocin-2-1H-one und Hexahydro-benzazocin-2-one werden in die 3-Halogen-, z. B. 3-Brom-Derivate, z. B. durch Behandlung mit Phosphorpentachlorid und anschliessende Hydrierung oder unter den in den Beispielen gezeigten Bedingungen umgewandelt. Die Substitution dieser Halogen-Derivate mit einem Metallazid, z. B. Natriumazid, und nachfolgende Reduktion oder Substitution mit Ammoniak oder einem Niederalkylamin und etwaige Acylierung ergibt Verbindungen der Formel X.

Alternativ werden die Verbindungen der Formel X, worin $R^9$ Amino, Alkylamino oder Acylamino bedeutet, durch Reduktion und Cyclisierung der geeignet substituierten und/oder derivatisierten (5 oder 6)-o-Nitrophenyl-2-amino(pentan oder hexan)-carbonsäure und gegebenenfalls anschliessende N-Alkylierung oder N-Acylierung erhalten.

Verbindungen der Formel X, worin $R^9$ Amino bedeutet, können aus Verbindungen der Formel X, worin $R^9$ für Phthalimido steht, z. B. durch Umsetzung mit Hydrazin hergestellt werden. Die genannten Phthalimido-Ausgangsstoffe, z. B. für Verbindungen, in welchen $m = 2$, können durch Cyclisierung der 2-Phthalimido-5-(o-aminophenyl)-pentansäure, z. B. mit Dicyclohexylcarbodiimid oder mit einem anderen an sich bekannten Cyclisierungsmittel cyclisiert werden. Die 5-(o-Aminophenyl)-2-phthalimidopentansäure oder ein Ester davon wird z. B. durch palladiumkatalysierte Kupplung von 2-Iodanilin mit N-Phthalylallylglycin-benzylester und nachfolgende Hydrierung erhalten.

Das Verfahren b) wird in üblicher Weise, unter den Bedingungen der vorher beschriebenen substitutiven Alkylierung, vorzugsweise in Gegenwart von sehr starken Basen, wie Alkalimetallhydriden (z. B. Natrium- oder Kaliumhybrid), -alkoholaten (z. B. Natrium-methylat oder -ethylat, Kalium-tert.-butylat) oder -amiden (z. B. Lithiumdiisopropylamid) durchgeführt, wobei die vorstehend erwähnten Ether und Amide als Lösungsmittel bevorzugt sind. Bei einer speziellen Ausführungsform des Verfahrens b) werden Ausgangsmaterialien verwendet, worin $R^{A'}$ Wasserstoff bedeutet, und es werden zumindest zwei Aequivalente der Reaktante IIIB verwendet. In dem erhaltenen Produkt sind beide Reste $R^A$ und $R^B$ identisch und liegen im Bereich der Bedeutungen von $R^B$.

Die Ausgangsmaterialien der Formel IIIB sind bekannt, oder wenn sie unbekannt sind, können sie auf einfache Weise nach herkömmlichen Syntheseverfahren hergestellt werden. Die Ausgangsmaterialien der Formel V können nach herkömmlichen Syntheseverfahren erhalten werden und mit Vorteil in der Weise, die nachstehend eingehender für die speziellen Zwischenprodukte beschrieben und veranschaulicht ist.

Verbindungen der Formel V können durch Kondensation unter den Bedingungen der reduktiven Alkylierung einer Verbindung der Formel

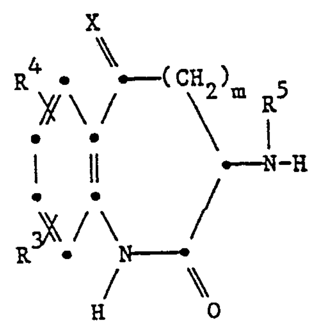

(XI)

oder ihres Perhydro-Derivates, worin $R^3$, $R^4$, m und X die oben angegebenen Bedeutungen haben, und $R^5$ Wasserstoff oder Niederalkyl bedeutet, mit einer Verbindung der Formel IV'

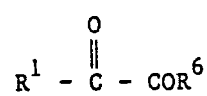

(IV')

worin $R^1$ und $R^6$ die vorher definierten Bedeutungen haben, oder unter Alkylierungsbedingungen mit einer Verbindung der Formel III'A

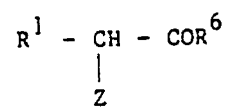

(III'A)

worin $R^1$, $R^6$ und Z die vorher angegebenen Bedeutungen haben, erhalten werden.

Das Verfahren c), das auch eine Alkylierungsreaktion darstellt, wird entsprechend den gleichen allgemeinen Gesichtspunkten und unter den gleichen experimentellen Bedingungen durchgeführt, wie im einzelnen vorstehend beschrieben (substitutive oder reduktive Alkylierung). Die Ausgangsmaterialien der Formel VI können mit Hilfe an sich bekannter herkömmlicher Verfahren hergestellt werden, z. B. in der nachstehend eingehender beschriebenen Weise.

Die Ausgangsmaterialien der Formel VII oder VII' sind Aminosäuren und deren Derivate, die aus dem Stand der Technik bekannt sind oder nach bekannten Methoden hergestellt werden können. Es ist erwähnenswert, dass die optisch aktiven erfindungsgemässen Verbindungen ausgehend von einer optisch aktiven Verbindung der Formel VII oder VII', z. B. von L-α-Aminophenylbuttersäure, L-Phenylalanin, L-Tryptophan, L-Methionin, L-Asparaginsäure, L-Threonin, L-Glutaminsäure, L-Lysin, L-Ornithin, und deren Derivaten, hergestellt werden können.

Das Verfahren d) wird auch in herkömmlicher Weise unter den allgemeinen Bedingungen der Solvolyse durchgeführt, die dafür bekannt sind, dass sie Cyanide (Nitrile) in die freien Carbonsäuren oder

ihre Salze, Ester oder Amide überführen. Für die Umwandlung in eine freie Säure wird die Hydrolyse mit Wasser vorteilhaft in einem inerten organischen Lösungsmittel, das zumindest teilweise mit Wasser mischbar ist, wie Ether (z. B. Diethyl- oder Diisopropylether, 1,2-Dimethoxyethan oder insbesondere Dioxan oder Tetrahydrofuran), oder einem Niederalkanol (z. B. Methanol, Ethanol, Isopropylalkohol, einem Butylalkohol, insbesondere tert.-Butylalkohol) durchgeführt, wobei eine grössere Menge Wasser in den letztgenannten Fällen erforderlich ist, um eine Alkoholyse zu verhindern. Die Hydrolyse kann sowohl durch starke Säuren, insbesondere anorganische Säuren, wie Schwefelsäure, oder vorzugsweise Halogenwasserstoffsäuren (z. B. Bromwasserstoffsäure oder als erste Wahl Chlorwasserstoffsäure), als auch durch Basen, insbesondere anorganische Basen, wie Hydroxide und Carbonate der Alkalimetalle, z. B. Natrium- und Kaliumhydroxid, katalysiert werden. Die Basen werden gewöhnlich in zumindest stöchiometrischen Anteilen verwendet, wobei Carbonsäuresalze als primäre Produkte entstehen. Die sauren Katalysatoren werden zur Erzielung des besten Resultats vorteilhaft in Form einer verdünnten wässrigen Lösung angewandt. Die Endprodukte der Formel I, worin $R^0$ eine veresterte Carboxylgruppe bedeutet, können erhalten werden, indem man die Solvolyse des Nitrils mit dem entsprechenden Alkohol (Alkoholyse) in Gegenwart einer katalytische Menge einer wasserfreien starken Säure, vorteilhaft von gasförmigem Chlorwasserstoff, durchführt. Gewöhnlich wird ein Ueberschuss an Alkohol als Lösungsmittel verwendet, jedoch können inerte organische Lösungsmittel, wie acyclische und cyclische Ether (insbesondere die vorstehend erwähnten), und/oder halogenierte Niederalkane (insbesondere Chloroform und Dichlormethan) zugegeben werden. Wird die Alkoholyse unter streng wasserfreien Bedingungen durchgeführt, muss das primäre Produkt (Imidoester), vorteilhaft durch Zugabe von Wasser, hydrolysiert werden. Andererseits wird, wenn man die Alkoholyse in Gegenwart eines annähernd stöchiometrischen Aequivalents von Wasser durchführt, der gewünschte Ester direkt erhalten. Um ein entsprechendes Amid (eine Verbindung der Formel I, worin $R^0$ Carbamoyl bedeutet) zu erhalten, kann ein entsprechendes Nitril der Formel VIII vorzugsweise einer alkalischen Hydrolyse in Gegenwart von Wasserstoffperoxid unterzogen werden.

Die Ausgangsmaterialien der Formel VIII können mit Hilfe an sich bekannter herkömmlicher Methoden, z. B. durch eine Kondensation analog derjenigen des Verfahrens c), erhalten werden, wobei ein Ausgangsmaterial der Formel VI mit einem Amin der Formel

$$R^5-NH-\overset{\overset{R^1}{|}}{CH}-CN \qquad (VII')$$

worin $R^1$ und $R^5$ die vorstehend angegebenen Bedeutungen besitzen, behandelt wird. Auch die Verfahren a) und b) können in analoger Weise zur Herstellung von Nitrilen der Formel VIII verwendet werden.

Die Cyclisierung entsprechend der Verfahrensvariante e) kann auch in an sich bekannter Weise, z. B. durch Dehydratation, durchgeführt werden. Besonders geeignete allgemeine Methoden für diesen Zweck sind die im Zusammenhang mit der Bildung der Amidbindung in Peptiden entwickelten, wie sie in Sammelwerken, z. B. Houben-Weyl, Band 15/I und Band 15/II, wie vorstehend zitiert, angegeben werden. Gemäss einer bevorzugten Abwandlung wird die zu cyclisierende Aminogruppe durch Protonierung (d. h. in Form eines Säureadditionssalzes) inaktiv gemacht und die Carboxylgruppe in einen aktivierten Ester, wie einen solchen mit 2,4,5-Trichlorphenol, Pentachlorphenol, Pentafluorphenol, 2-Nitrophenol oder insbesondere 4-Nitrophenol, oder mit einer N-Hydroxyverbindung, wie N-Hydroxysuccinimid, 1-Hydroxybenztriazol oder N-Hydroxypiperidin, oder alternativ mit einem N,N'-disubstituierten Isoharnstoff, wie insbesondere N,N'-Dicyclohexylisoharnstoff, oder einem analogen allgemein bekannten aktivierenden Mittel, übergeführt. Die Cyclisierung wird durchgeführt, indem man vorzugsweise durch Zugabe einer organischen Base, z. B. eines quaternären Ammoniumsalzes oder insbesondere eines tertiären Amins, wie Triethylamin, N-Ethylmorpholin oder N-Methylpiperidin, basisch macht, um die zu cyclisierende Aminogruppe durch Umwandlung in die unprotonierte Form zu reaktivieren. Die Reaktionstemperatur beträgt gewöhnlich von —20 bis +50 °C, vorzugsweise annähernd Raumtemperatur, und es werden übliche Lösungsmittel verwendet, z. B. Dioxan, Tetrahydrofuran, Acetonitril, Pyridin, Dimethylformamid, Dimethylacetamid, Dimethylsulfoxid, N-Methylpyrrolidon, Hexamethylphosphorsäuretrisamid, sowie Chloroform und Methylenchlorid und bewährte Mischungen derselben. Bei einer speziellen Variante des Verfahrens kann die Carboxygruppe direkt in situ durch Einwirken der freien Säure mit einem Carbodiimid, wie N,N'-Dicyclohexylcarbodiimid (gegebenenfalls unter Zusatz von N-Hydroxysuccinimid, eines unsubstituierten oder beispielsweise Halogen-, Methyl- oder Methoxy-substituierten 1-Hydroxybenztriazols oder von 4-Hydroxybenzo-1,2,3-triazin-3-oxid oder N-Hydroxy-5-norbornen-2,3-dicarboximid) oder mit N,N'-Carbonyldiimidazol aktiviert werden.

Die Ausgangsmaterialien der Formel IX können nach an sich bekannten allgemeinen Methoden, z. B. wie sie nachstehend anhand spezieller Beispiele erörtert werden, hergestellt werden.

Auch die Reduktion entsprechend dem Verfahren f) kann in einer Weise durchgeführt werden, wie sie an sich allgemein für die Sättigung derartiger Doppelbindungen bekannt ist. Die Sättigung der Doppelbindungen wird vorteilhaft durch katalytische Hydrierung, beispielsweise unter den im einzelnen

vorstehend erörterten bevorzugten Bedingungen, sowie auch durch Metallreduktion, wie Zinkreduktion, in neutralem oder saurem Medium oder insbesondere im Fall der C-N-Doppelbindung durch Diboran oder komplexe Hydride, wie Natriumborhydrid, wie vorstehend erwähnt, durchgeführt. Die ungesättigten Ausgangsmaterialien für diese Verfahrensvariante werden nach allgemein bekannten Methoden, z. B. den bei den Verfahren a) und c) und/oder in einer speziellen Form, wie nachstehend erörtert, erhalten.

Bei der Durchführung der fakultativen Umwandlung eines erhaltenen Endprodukts der Formel I in eine andere Verbindung der Formel I werden Umwandlungen wie die folgenden durchgeführt: eine Aminogruppe wird alkyliert, und/oder eine Oxogruppe, insbesondere diejenige des Symbols X, wird in eine Hydroxygruppe plus Wasserstoff oder in zwei Wasserstoffatome durch Reduktion umgewandelt, und/oder Hydroxy wird in Oxo umgewandelt durch Oxidation oder in Wasserstoff durch Reduktion, und/oder eine freie Hydroxy- oder Carboxygruppe wird aus ihrer veresterten Form durch Hydrolyse oder Hydrogenolyse freigesetzt, und/oder eine Hydroxy- oder Aminogruppe wird acyliert, und/oder ein freies Carboxyl wird verestert, und/oder der aromatische carbocyclische Ring in der Formel I wird perhydriert, und/oder der perhydrierte carbocyclische Ring wird dehydriert zu dem aromatischen carbocyclischen Ring. Alle diese fakultativen Umwandlungen werden nach gut bekannten herkömmlichen Methoden durchgeführt.

Durch die Alkylierungsreaktion kann z. B. das durch $R^5$ dargestellte Niederalkyl in das Endprodukt der Formel I eingeführt werden, worin $R^5$ Wasserstoff bedeutet, wobei sämtliche der im einzelnen vorher erörterten Abwandlungen angewandt werden können. Sowohl die substitutive als auch die reduktive Alkylierung können angewandt werden, die erstgenannte mit Alkylhalogeniden, die letztgenannte mit niedrigen aliphatischen Aldehyden und Ketonen und katalytisch aktiviertem Wasserstoff oder im Fall von Formaldehyd vorteilhaft mit Ameisensäure als Reduktionsmittel. Durch die substitutive Alkylierung können Niederalkylgruppen auch in eine durch das Symbol $R^0$ dargestellte Carbamoylgruppe eingeführt werden.

Die Verbindungen der Formel I oder IA und die Zwischenprodukte hierfür, worin X Oxo bedeutet, können in die entsprechenden Verbindungen, worin X ein Wasserstoffatom und eine Hydroxygruppe bedeutet, durch Reduktion, z. B. katalytische Hydrierung, z. B. mit Wasserstoff in Gegenwart eines Platinkatalysators, oder mit einem Metallhydrid-Reduktionsmittel, wie Natriumborhydrid, oder gemäss der Meerwein-Ponndorf-Methode oder einer Abwandlung derselben unter Verwendung eines Alkanols, insbesondere Isopropylalkohol, sowohl als Lösungsmittel als auch als Reduktionsmittel und eines Metallalkoholats, vorzugsweise eines dem reduzierenden Alkohol entsprechenden, wie Aluminiumisopropylat, als Katalysator, übergeführt werden. Die Reduktion der Oxogruppe zu zwei Wasserstoffatomen kann vorteilhaft z. B. durch Behandlung mit amalgamiertem Zink und Chlorwasserstoffsäure oder durch Raney-Nickel-Desulfurierung eines entsprechenden Dithioketals bewirkt werden. Erhaltene Verbindungen, worin X ein Wasserstoffatom und eine Hydroxygruppe bedeutet, können in die Verbindungen, worin X zwei Wasserstoffatome bedeutet, z. B. durch katalytische Hydrierung des Addukts eines Carbodiimids, beispielsweise des Addukts durch Kondensation einer Verbindung, worin X ein Wasserstoffatom und eine Hydroxygruppe bedeutet, mit Dicyclohexylcarbodiimid in Gegenwart von Cuprochlorid gemäss der allgemeinen, in Chem. Ber. 107, 1353 (1974) beschriebenen Methode übergeführt werden.

Alternativ können die Verbindungen, worin X ein Wasserstoffatom und eine Hydroxygruppe bedeutet, zuerst in die entsprechenden Verbindungen, worin X ein Wasserstoffatom und eine acylierte Hydroxygruppe (oder Acyloxy z. B. Acetoxy) bedeutet, übergeführt und anschliessend z. B. durch katalytische Hydrierung in Gegenwart eines Palladiumkatalysators, zu Verbindungen reduziert werden, worin X zwei Wasserstoffatome bedeutet. Die Oxidation von Hydroxy zur Bildung von Oxo kann vorzugsweise mit einem Derivat des sechswertigen Chroms, wie Chromsäure und deren Salze, mit einem Permanganatsalz (insbesondere Kaliumpermanganat) oder unter den Bedingungen der Oppenauer-Oxidation mit Aceton oder Cyclohexanon als Oxidationsmittel und Aluminiumisopropylat als Katalysator durchgeführt werden. Veresterte Hydroxygruppen werden insbesondere mit Hilfe der vorstehend im einzelnen im Zusammenhang mit der Abspaltung der Hydroxyschutzgruppen erörterten Methoden freigesetzt. Die Acylierung von Hydroxygruppen wird auf übliche Weise, vorzugsweise unter Verwendung eines entsprechenden Säureanhydrids oder Halogenids, durchgeführt.

Der aromatische carbocyclische Ring in Verbindungen der Formel I oder in den Zwischenprodukten für die Herstellung von Verbindungen der Formel I wird in die Perhydro-Form z. B. durch Hydrierung bei atmosphärischem oder höherem Druck, in Gegenwart eines Katalysators (wie Platin oder Rhodium), bei Zimmertemperatur oder erhöhter Temperatur, in einem polaren Lösungsmittel, z. B. Ethanol, umgewandelt.

Verbindungen der Formel I oder IA, worin $R^1$ Amino-niederalkyl bedeutet, können in die Verbindungen, worin $R^1$ für Acylaminoniederalkyl steht, oder umgekehrt, gemäss den aus dem Stand der Technik bekannten und vorher im Zusammenhang mit den Schutzgruppen beschriebenen Methoden, übergeführt werden.

Die freien Carbonsäuren der Formel I oder IA, worin $R^6$ und/oder $R^7$ Hydroxy bedeuten oder deren Salze, können mit geeigneten aus dem Stand der Technik gut bekannten Alkoholen oder deren reaktiven Derivaten, oder mit einem Diazoalkan, insbesondere Diazomethan, verestert werden, um die entsprechenden Mono- oder Di-ester, nämlich die Verbindungen der Formel I oder IA zu ergeben, worin $R^6$ und/oder $R^7$ Niederalkoxy, Aryl-niederalkoxy, Niederalkanoyloxymethoxy oder Niederalkoxycarbonyl-niederalkoxy

bedeuten. Alternativ kann die Carboxylgruppe in ein reaktives Derivat derselben, wie einen aktivierten Ester, z. B. einen solchen mit 2,4,5-Trichlorphenol, Pentachlorphenol, Pentafluorphenol, 2-Nitrophenol oder insbesondere 4-Nitrophenol, oder mit einer N-Hydroxy-Verbindung, z. B. N-Hydroxysuccinimid, 1-Hydroxybenztriazol oder N-Hydroxypiperidin, oder alternativ mit einem N,N'-disubstituierten Isoharnstoff, wie insbesondere N,N'-Dicyclohexylisoharnstoff, oder in ein gemischtes Anhydrid, z. B. mit einem Säurehalogenid (z. B. insbesondere Säurechlorid) umgewandelt werden. Dieses aktivierte Zwischenprodukt wird dann mit dem gewünschten Alkohol umgesetzt. Weiter kann die freie Carbonsäure über reaktionsfähige Zwischenprodukte in die Mono- oder Di-amide der Formel I, worin $R^6$ und/oder $R^7$ Amino, Mono- oder Di-niederalkylamino bedeuten, umgewandelt werden.

Die freie Carboxylgruppe kann aus einem veresterten Carboxyl in allgemein bekannter Weise, insbesondere durch Basen-katalysierte Hydrolyse, freigesetzt werden. Von speziellem Interesse sind jedoch Methoden, nach denen es möglich ist, selektiv eine spezielle durch die Symbole $—COR^6$ und $—COR^7$ wiedergegebene Carboxylgruppe freizusetzen. In einem derartigen Fall kann von einer geeigneten Kombination von Estergruppen Gebrauch gemacht werden, die im Stand der Technik insbesondere als Carboxylschutzgruppen bekannt und in grosser Vielfalt insbesondere für die Synthese von Peptiden entwickelt worden sind, vgl. Houben-Weyl, Band 15/I und Band 15/II wie vorstehend zitiert. Für die selektive Abspaltung unter Freisetzung der Carboxylgruppe geeignete Reste sind Ester, die sich z. B. von Alkoholen, die durch Acidolyse entfernbare Reste ergeben, ableiten, wie Cyanmethylalkohol, Benzoylmethylalkohol oder tert.-Butylalkohol, jedoch insbesondere von Alkoholen, die Reste ergeben, die abgespalten werden können durch Reduktion, wie 2,2,2-Trichlorethanol, Benzylalkohol, und insbesondere 4-Nitrobenzylalkohol, oder alternativ Isonicotinalkohol. Eine besonders bevorzugte Klasse von substituierten Alkanolen sind Ethylalkohole, die in β-Stellung eine trisubstituierte Silylgruppe enthalten, wie Triphenylsilyl, Dimethyl-tert.-butylsilyl oder insbesondere Trimethylsilyl. Wie beispielsweise in der BE-PS 851 576 beschrieben, sind diese Alkohole besonders zur selektiven Entfernung geeignet, da die entsprechenden β-Sylilethylester, beispielsweise β-(Trimethylsilyl)-ethylester, die Stabilität herkömmlicher Alkylester besitzen, jedoch selektiv unter milden Bedingungen durch Einwirken von Fluorid-Ionen unter Erhalt anderer veresterter Carboxylgruppen, z. B. von Alkoxycarbonylgruppen, abgespalten werden können.

Die Abspaltung der veresternden Gruppen hängt von ihrer Natur ab und wird in jedem Fall in herkömmlicher, an sich bekannter Weise durchgeführt, wobei man die Eigenschaften der anderen beteiligten Reste mit berücksichtigt. Die Gruppen, die durch Reduktion abgespalten werden können, insbesondere diejenigen, die halogenierte Niederalkylreste (beispielsweise 2,2,2-Trichlorethylreste), Isonicotinylreste (z. B. Isonicotinyloxycarbonyl) und gegebenenfalls substituierte Benzylreste, vor allem 4-Nitrobenzylreste jeglicher Art enthalten, werden bevorzugt durch Reduktion mit Zink, gewöhnlich in Gegenwart einer Säure, vorzugsweise Essigsäure, und mit oder ohne Zusatz eines inerten organischen Lösungsmittels, gewöhnlich bei Raumtemperatur abgespalten, diejenigen des Benzyl-Typs, insbesondere unsubstituierte Benzylester, werden auch durch Hydrogenolyse-Techniken, wie sie üblicherweise für Benzylgruppen angewandt werden, z. B. mit Wasserstoff in Gegenwart einer Katalysators, z. B. Palladium, entfernt.

Die Umwandlung der Verbindungen der Formel I oder IA, worin $R^6$ und/oder $R^7$ Niederalkoxy, Arylniederalkoxy, Amino, Mono- oder Di-(niederalkyl)-amino bedeuten, in die Verbindungen der Formel I oder IA, worin $R^6$ und/oder $R^7$ Hydroxy bedeuten, wird vorteilhaft durchgeführt durch Hydrolyse mit anorganischen Säuren, wie Halogenwasserstoffsäuren, Trifluoressigsäure oder Schwefelsäure. Die β-Silylethylestergruppen werden vorzugsweise durch Fluorid-Ionen erzeugende Reagentien, z. B. Fluoride von quaternären organischen Basen, wie Tetraethylammoniumfluorid, abgespalten. Gruppen $COR^6$ und $COR^7$, die Basen-instabil sind, können vorsichtig durch rasches Einwirken einer wässrigen Natrium- oder Kaliumbicarbonatlösung oder vorzugsweise von wässrigem Ammoniak in einem organischen Lösungsmittel, gewöhnlich bei Raumtemperatur, und/oder mit wässrigen Alkalien, vorzugsweise Alkalimetallhydroxiden, wie Lithium- oder Natriumhydroxid, entfernt werden. Die Estergruppen werden vorzugsweise unter den Reaktionsbedingungen der Beispiele oder unter analogen Bedingungen abgespalten.

Eine geeignete Kombination der Estergruppen kann bei den früheren Synthesestufen oder durch eine geeignete Wahl an Ausgangsmaterialien und Reaktanten ausgewählt werden, wobei z. B. eine selektiv abspaltbare Estergruppe mit einem Carboxyl eingeführt wird, das in der letzten Stufe freigesetzt wird.

Die Verbindungen der Formel I oder IA, worin $R^6$ und/oder $R^7$ Niederalkoxy bedeuten, können mit Ammoniak, Mono- oder Di-niederalkylaminen amidiert werden, um Verbindungen der Formel I oder IA zu ergeben, worin $R^6$ und/oder $R^7$ unsubstituiertes oder Mono- oder Di-niederalkyl-Amino bedeuten.

Die Verbindungen der Formel I oder IA, worin weder $R^6$ noch $R^7$ Hydroxy bedeuten, können in Monocarbonsäuren der Formel I oder IA übergeführt werden, worin einer der Reste $R^6$ und $R^7$ Hydroxy bedeutet. Eine derartige Umwandlung wird durch selektive hydrolytische oder hydrogenolytische Verfahren, die aus dem Stand der Technik gut bekannt sind und sich nach dem chemischen Charakter der $R^6$- und $R^7$-Substituenten richten, durchgeführt.

Die vorstehenden Reaktionen werden nach Standard-Methoden in Gegenwart oder Abwesenheit von Verdünnungsmitteln, vorzugsweise solchen, die gegenüber den Reagentien inert sind und deren Lösungsmittel darstellen, von Katalysatoren, Kondensationsmitteln bzw. den anderen Mitteln und/oder in

inerter Atmosphäre, bei niedrigen Temperaturen, Raumtemperatur oder erhöhten Temperaturen, vorzugsweise beim Siedepunkt der verwendeten Lösungsmittel, bei atmosphärischem oder überatmosphärischem Druck, durchgeführt.

Die Erfindung umfasst weiterhin jegliche Variante der vorliegenden Verfahren, bei der ein bei irgendeiner Stufe derselben erhältliches Zwischenprodukt als Ausgangsmaterial verwendet wird und die verbliebenen Stufen ausgeführt werden, oder das Verfahren bei irgendeiner Stufe desselben unterbrochen wird oder bei der die Ausgangsmaterialien unter Reaktionsbedingungen gebildet werden oder bei der die Reaktionskomponenten in Form ihrer Salze oder optisch reinen Antipoden verwendet werden. Hauptsächlich diejenigen Ausgangsmaterialien sollten bei diesen Reaktionen verwendet werden, die zur Bildung der vorstehend als besonders wertvoll angegebenen Verbindungen führen.

Die Erfindung betrifft auch neue Ausgangsmaterialien und Verfahren zu deren Herstellung.

In Abhängigkeit von der Wahl der Ausgangsmaterialien und Methoden können die neuen Verbindungen in Form eines der möglichen Isomeren oder von deren Gemischen vorliegen, beispielsweise in Abhängigkeit von der Anzahl der asymmetrischen Kohlenstoffatome, als reine optische Isomere, wie als Antipoden, oder als Gemische von optischen Isomeren, wie als Racemate, oder als Gemische von Diastereoisomeren.

Erhaltene Gemische der Diastereoisomeren und Gemische der Racemate können auf Basis der physiko-chemischen Unterschiede der Bestandteile in bekannter Weise in die reinen Isomeren, Diastereoisomeren oder Racemate getrennt werden, z. B. durch Chromatographie und/oder fraktionierte Kristallisation.

Die erhaltenen Racemate können weiterhin in die optischen Antipoden nach an sich bekannten Methoden, beispielsweise durch Umkristallisieren aus einem optisch aktiven Lösungsmittel, mit Hilfe von Mikroorganismen oder durch Umsetzung eines sauren Endprodukts mit einer optisch aktiven Base, die Salze mit der racemischen Säure bildet, und Trennung der auf diese Weise erhaltenen Salze, beispielsweise auf Basis ihrer unterschiedlichen Löslichkeiten, in die Diastereoisomeren, aus denen die Antipoden durch Einwirken geeigneter Mittel freigesetzt werden können, aufgetrennt werden. Basische racemische Produkte können gleichfalls in die Antipoden, z. B. durch Trennung von deren diastereoisomeren Salzen, z. B. durch fraktionierte Kristallisation von deren d- oder ℓ-Tartraten, getrennt werden. Irgendwelche racemischen Zwischenprodukte oder Ausgangsmaterialien können in ähnlicher Weise getrennt werden.

Zweckmässigerweise wird der aktivere der beiden Antipoden isoliert.

Schliesslich werden die erfindungsgemässen Verbindungen entweder in freier Form oder in Form von deren Salzen erhalten. Irgendeine erhaltene Base kann in ein entsprechendes Säureadditionssalz, vorzugsweise unter Verwendung einer pharmazeutisch verträglichen Säure oder eines Anionenaustausch-Präparates, übergeführt werden, oder erhaltene Salze können in die entsprechenden freien Basen, beispielsweise unter Verwendung einer stärkeren Base, wie eines Metall- oder Ammoniumhydroxids oder eines basischen Salzes, z. B. eines Alkalimetallhydroxids oder -carbonats, oder eines Kationenaustausch-Präparats, übergeführt werden. Eine Verbindung der Formel I, worin $R^0$ Carboxy bedeutet, oder der Formel IA, worin $COR^6$ und/oder $COR^7$ Carboxy bedeuten, kann somit auch in die entsprechenden Metall- oder Ammoniumsalze übergeführt werden. Diese oder andere Salze, beispielsweise die Pikrate, können auch für die Reinigung der erhaltenen Basen verwendet werden. Die Basen werden in die Salze übergeführt, die Salze werden getrennt, und die Basen werden aus den Salzen freigesetzt. Im Hinblick auf die enge Verwandtschaft zwischen den freien Verbindungen und den Verbindungen in Form ihrer Salze ist, wenn immer in diesem Zusammmenhand auf eine Verbindung Bezug genommen wird, auch ein entsprechendes Salz mit umfasst, vorausgesetzt, dass dies unter den gegebenen Umständen möglich oder sinnvoll ist.

Die Verbindungen einschliesslich ihrer Salze können auch in Form ihrer Hydrate erhalten werden oder andere für die Kristallisation verwendete Lösungsmittel enthalten.

Die erfindungsgemässen pharmazeutischen Präparate sind solche, die für die enterale, wie die orale oder rektale, und die parenterale Verabreichung an Säuger, einschliesslich des Menschen, zur Behandlung oder Verhinderung von Erkrankungen geeignet sind, die auf die Inhibierung des Angiotensin-umwandelnden Enzyms ansprechen, z. B. von kardiovaskularen Erkrankungen, wie der Hypertension, und von kongestiver Herzinsuffizienz und umfassen eine wirksame Menge einer pharmakologisch aktiven Verbindung der Formel I oder eines pharmazeutisch verträglichen Salzes derselben, allein oder in Kombination mit einem oder mehreren pharmazeutisch verträglichen Trägern.

Die pharmakologisch aktiven Verbindungen der Erfindung sind bei der Herstellung von pharmazeutischen Zusammensetzungen verwendbar, die eine wirksame Menge derselben in Verbindung oder Bemischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z. B. Lactose, Dextrose, Sucrose, Mannit, Sorbit, Cellulose und/oder Glycin, b) Gleitmitteln, z. B. Siliciumdioxid, Talk, Stearinsäure, deren Magnesium- oder Calciumsalz und/oder Polyethylenglykol, für Tabletten auch c) Bindemitteln, z. B. Magnesiumaluminiumsilikat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z. B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvantien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Ueberzugsmethoden hergestellt und enthalten etwa 0,1 bis 75 %, vorzugsweise etwa 1 bis 50 %, des aktiven Bestandteils. Eine Einheitsdosis für einen Säuger von etwa 50 bis 70 kg kann zwischen etwa 10 und 200 mg aktiven Bestandteil enthalten.

Die folgenden Beispiele sollen die Erfindung erläutern und keine Einschränkungen darstellen. Die Temperaturen sind in Celsiusgraden angegeben, und sämtliche Teile sind in Form von Gewichtsteilen angegeben. Wenn nicht anders erwähnt, werden sämtliche Verdampfungen unter vermindertem Druck, vorzugsweise zwischen etwa 15 und 100 mg Hg, durchgeführt.

Im Fall der Verbindungen der Formel I oder IA, worin mehr als ein Asymmetrie-Zentrum vorliegt, werden die erhaltenen diastereoisomeren Verbindungen als A, B etc. in den betreffenden Beispielen bezeichnet. Die jeweiligen diastereoisomeren Verbindungen werden durch die physikalischen Eigenschaften, z. B. den Schmelzpunkt, die relative Wanderung bei der Chromatographie sowie die spektralen Eigenschaften bei der Infrarot- oder kernmagnetischen Resonanz-Messung charakterisiert.

Im Fall der Verbindungen der Formel I oder IA, worin X zwei Wasserstoffatome bedeutet und ein Asymmetrie-Zentrum in der Seitenkette an dem das Stickstoff tragenden Kohlenstoffatom vorliegt, wurden die Symbole A und B wie folgt den jeweiligen Isomeren auf Basis ihrer relativen Wanderung bei der Chromatographie zugeordnet. Auf Basis der Wanderung bei der Dünnschichtchromatographie und Normalphasen-Hochdruck-Flüssigkeits-Chromatographie unter Verwendung von Siliciumdioxidgel als stationäre Phase wird das sich schnell bewegende Isomere als Isomer A und das sich langsam bewegende Isomere als Isomer B bezeichnet. Auf Basis der Wanderung bei der Hochdruck-Flüssigkeits-Chromatographie mit reverser Phase wird das sich langsam bewegende Isomere als Isomer A und das sich schnell bewegende Isomer als Isomer B bezeichnet.

### Beispiel 1

a) Eine Lösung von 1,0 g 3-Amino-1-ethoxycarbonylmethyl-3,4,5,6-tetrahydro-1-benzazocin-2-1H-on in 40 ml Methanol und 1,4 ml 10 %iger Natronlauge wird unter Stickstoff 2 Stunden bei Raumtemperatur gehalten. Der Ansatz wird im Hochvakuum zur Trockene eingeengt und ergibt das Natriumsalz der Aminosäure, Fp. 248-250° (Zers.). Dieses Material und 2,2 g Benzylbrenztraubensäureethylester werden in 60 ml Methanol/Essigester (60 : 40) gelöst. Eine Lösung von 0,3 g Natriumcyanborhydrid in 10 ml Methanol wird während 2 Stunden tropfenweise zugegeben, der Ansatz wird während 65 Stunden bei Raumtemperatur gerührt. 2 ml konz. Salzsäure werden zugegeben, und der Ansatz 1 Stunde gerührt. Die Lösungsmittel werden unter vermindertem Druck abgezogen, 50 ml Wasser werden zugesetzt, und der pH wird durch Zugabe von 2N Natronlauge auf 12 eingestellt. Die wässerige Lösung wird mit Ether extrahiert (2 × 75 ml), mit konz. Salzsäure auf pH 4 eingestellt und mit Dichlormethan (2 × 100 ml) extrahiert. Die vereinigten Dichlormethan-Lösungen werden über Natriumsulfat getrocknet; nach dem Abziehen des Lösungsmittels unter vermindertem Druck bleibt ein weisser Schaum zurück. Dieser wird in 25 ml Dichlormethan gelöst, danach 5 Minuten lang HCl-Gas eingeleitet. Die Lösung wird zur Trockene eingedampft, der Rückstand mit Essigsäureethylester gekocht, wobei man 1-Carboxymethyl-3-(1-ethoxycarbonyl-3-phenylpropylamino)-3,4,5,6-tetrahydro-1-benzazocin-2-1H-on-Hydrochlorid, Fp. 147-149° (Zers.), erhält.

b) Ueber Nacht wird die Substanz mit einem Gemisch aus verdünnter Natronlauge und Methanol bei Raumtemperatur behandelt, anschliessend angesäuert, und man erhält 1-Carboxymethyl-3-(1-carboxy-3-phenylpropylamino)-3,4,5,6-tetrahydro-1-benzazocin-2-1H-on.

Das Ausgangsmaterial wird wie folgt hergestellt:

DL-α-Allylglycin (30 g) wird unter Rühren zu einer Lösung von 37,0 g wasserfreiem Natriumcarbonat in 1 400 ml Wasser gegeben. Man fügt N-Ethoxycarbonylphthalimid (78,1 g) zur gerührten Lösung und rührt weitere 30 Minuten. Das Reaktionsgemisch wird filtriert und durch Zugabe von 2N Natronlauge stark basisch gemacht. Die Lösung wird mit Methylenchlorid (4 × 500 ml) extrahiert und dann mit 6N HCl bis auf pH 2 angesäuert. Die Lösung wird mit Methylenchlorid (4 × 500 ml) extrahiert und die vereinigten Extrakte über Natriumsulfat getrocknet. Das Lösungsmittel wird unter vermindertem Druck abgezogen, der Rückstand mit Ether verrieben, wobei man N-Phthalylallylglycin vom Fp. 107-109° erhält, das ohne Reinigung weiterverwendet wird.

Der pH-Wert einer Lösung von N-Phthalylallylglycin (45 g) in Methanol (400 ml) und Wasser (40 ml) wird durch Zugabe einer 20 % wässerigen Cäsiumcarbonat-Lösung auf 7,0 eingestellt. Die Lösungsmittel werden unter vermindertem Druck abgezogen und der Rückstand in Dimethylformamid (225 ml) gelöst. Die Lösung wird unter vermindertem Druck eingedampft, dann wird weiteres Dimethylformamid (225 ml) hinzugegeben und wiederum eingedampft. Der Rückstand wird in Dimethylformamid (360 ml) gelöst, Benzylbromid (35 g) hinzugegeben und das Reaktionsgemisch 65 Stunden bei Raumtemperatur gerührt. Das Lösungsmittel wird bei vermindertem Druck abgezogen, Essigsäureethylester (1 000 ml) und Wasser

(500 ml) hinzugegeben und die Phasen getrennt. Die organische Phase wird mit 2N Natronlauge (200 ml), dann mit Wasser (500 ml) gewaschen und über Natriumsulfat getrocknet. Das Lösungsmittel wird bei vermindertem Druck abgezogen, der Rückstand mit Ether verrieben, wobei man den N-Phthalylallylglycinbenzylester erhält, der ohne Reinigung weiterverwendet wird.

Ein Gemisch aus N-Phthalylallylglycinbenzylester (52,8 g), 2-Iodanilin (27,7 g), Palladium(II)-acetat (2,9 g), Tri(o-tolyl)-phosphin (7,75 g), Triethylamin (22,2 ml) und Acetonitril (264 ml) wird in einem Autoklav 18 Stunden unter Stickstoff bei 100° gehalten. Das Lösungsmittel wird unter vermindertem Druck abgezogen, zum Rückstand Wasser (500 ml) und Methylenchlorid (1 500 ml) hinzugegeben und die Phasen getrennt. Die wässerige Phase wird mit weiterem Methylenchlorid (500 ml) extrahiert und die vereinigten Methylenchlorid-Extrakte über Natriumsulfat getrocknet. Das Lösungsmittel wird unter vermindertem Druck abgezogen und der Rückstand über Kieselgel chromatographiert, wobei man als Hauptfraktion ein Oel erhält, das als 5-(2-Aminophenyl)-2-phthalimido-4-pentensäurebenzylester identifiziert und ohne Reinigung weiterverwendet wird.

Eine Lösung von 5-(2-Aminophenyl)-2-phthalimido-4-pentensäurebenzylester (10,7 g) in Ethanol (500 ml) wird bei atmosphärischem Druck mit 10 % Palladium-auf-Kohle (2 g) bis zum Ende der Wasserstoffaufnahme hydriert. Der Katalysator wird abfiltriert und das Filtrat unter vermindertem Druck eingedampft. Der Rückstand wird mit Essigsäureethylester verrieben, und man erhält 5-(2-Aminophenyl)-2-phthalimidopentansäure vom Fp. 258-260°, die ohne Reinigung weiterverwendet wird.

Eine Lösung von 5-(2-Aminophenyl)-2-phthalimidopentansäure (3,5 g) und 1-(3-Dimethylaminopropyl)-3-ethylcarbodiimid-Hydrochlorid (1,9 g) in Dimethylformamid (85 ml) wird unter Stickstoff 65 Stunden bei Raumtemperatur gerührt. Das Lösungsmittel wird im Hochvakuum abgezogen, zum Rückstand Essigsäureethylester (250 ml) und Wasser (150 ml) gegeben und die Phasen getrennt. Die organische Phase wird mit 2N Natronlauge (100 ml), dann mit Wasser (100 ml) gewaschen und über Natriumsulfat getrocknet. Das Lösungsmittel wird unter vermindertem Druck abgezogen und der Rückstand aus Methanol/Essigsäureethylester kristallisiert, wobei man 3-Phthalimido-3,4,5,6-tetrahydro-1-benzazocin-2-1H-on vom Fp. 229-230° erhält, das ohne Reinigung weiterverwendet wird.

Eine Lösung von 3-Phthalimido-3,4,5,6-tetrahydro-1-benzazocin-2-1H-on (1,7 g) in Dimethylformamid (10 ml) wird bei 0° unter Stickstoff gerührt. Eine Lösung von Kalium-tert.-butoxid (0,75 g) in Dimethylformamid (6 ml) wird hinzugegeben und das Reaktionsgemisch bei 0° 5 Minuten lang gerührt. Ferner wird eine Lösung von Bromessigsäureethylester (1,0 g) in Dimethylformamid (5 ml) hinzugegeben und das Reaktionsgemisch 1-1 1/2 Stunden lang bei 0° gerührt. Nach Zugabe von Wasser (3 ml) wird der Ansatz im Hochvakuum eingedampft. Zum Rückstand wird Essigsäureethylester (100 ml) und Wasser (50 ml) gegeben und die Phasen getrennt. Die organische Phase wird mit Wasser (50 ml) gewaschen und über Natriumsulfat getrocknet. Der Rückstand wird aus Methanol/Ether umkristallisiert, und man erhält 1-Ethoxycarbonylmethyl-3-phthalimido-3,4,5,6-tetrahydro-1-benzazocin-2-1H-on vom Fp. 79-81°, das direkt weiterverwendet wird.

Eine Lösung von 1-Ethoxycarbonylmethyl-3-phthalimido-3,4,5,6-tetrahydro-1-benzazocin-2-1H-on (1,4 g) und Hydrazinhydrat (0,19 g) in Ethanol (400 ml) wird 4 Stunden unter Rückfluss gekocht. Das Lösungsmittel wird unter vermindertem Druck abgezogen, zum Rückstand Essigsäureethylester (200 ml) und 2N Salzsäure (125 ml) gegeben und die Phasen getrennt. Die organische Phase wird mit 2N Salzsäure (100 ml) gewaschen. Die vereinigten Salzsäure-Extrakte werden mit 2N Natronlauge stark basisch gemacht, mit Essigsäureethylester (2 × 200 ml) extrahiert und über Natriumsulfat getrocknet. Das Lösungsmittel wird unter vermindertem Druck abgezogen und der Rückstand aus Ethanol/Ether kristallisiert, wobei man 3-Amino-1-ethoxycarbonylmethyl-3,4,5,6-tetrahydro-1-benzazocin-2-1H-on als Oel erhält, das ohne Reinigung weiterverwendet wird.

3-Phthalimido-3,4,5,6-tetrahydro-1-benzazocin-2-1H-on kann auch wie folgt hergestellt werden.

Eine Lösung von 6-Acetoxy-3,4,5,6-tetrahydro-1-benzazocin-2-1H-on (7,4 g) in Ethanol (250 ml) wird bei einem Druck von 3 at bei 70° unter Verwendung von 10 % Palladium-auf-Kohle (1,5 g) als Katalysator hydriert. Der Katalysator wird abfiltriert und das Lösungsmittel bei vermindertem Druck abgezogen. Der Rückstand wird aus Methanol/Essigsäureethylester umkristallisiert, und man erhält 3,4,5,6-Tetrahydro-1-benzazocin-2-1H-on.

Zu einer Lösung von 4,7 g 3,4,5,6-Tetrahydro-1-benzazocin-2-1H-on [J. Chem. Soc. (C) 3176 (1969)] in Chloroform (75 ml) wird Phosphorpentachlorid (5,9 g portionsweise hinzugegeben, wobei die Temperatur bei 0-5° gehalten wird. Nach erfolgter Zugabe wird Iod (60 mg) hinzugegeben, ferner Brom (4,5 g), letzteres tropfenweise innerhalb von 5 Minuten. Das Gemisch wird 4 Stunden unter Rückfluss gekocht. Das Chloroform wird abgedampft, zum Rückstand sowohl ein Eis/Wasser-Gemisch (60 ml) sowie Dichlormethan (100 ml) gegeben und die Phasen getrennt. Die organische Phase wird über Magnesiumsulfat getrocknet und unter vermindertem Druck eingedampft. Das zurückbleibende Rohmaterial wird durch Chromatographie über Kieselgel gereinigt, wobei die Elution mit Ether/Hexan-Mischungen erfolgt. Nach Einengung der entsprechenden Fraktionen erhält man 3-Brom-3,4,5,6-tetrahydro-1-benzazocin-2-1H-on.

Eine Lösung von 3-Brom-3,4,5,6-tetrahydro-1-benzazocin-2-1H-on (3,0 g) und Kalium-phthalimid (2,3 g) in Dimethylformamid (150 ml) wird 18 Stunden bei 90° gehalten. Das Reaktionsgemisch wird auf Raumtemperatur abgekühlt und in Wasser gegeben (450 ml). Man rührt noch 2 Stunden, filtriert das 3-Phthalimido-3,4,5,6-tetrahydro-1-benzazocin-2-1H-on ab und trocknet es.

Beispiel 2

Eine Lösung des Natriumsalzes von 3-Amino-1-carboxymethyl-1,3,4,5,6,7-hexahydro-1-benzazonin-2-on (13,2 g) und von Benzylbrenztraubensäureethylester (30 g) in Essigsäure (100 ml) und Methanol (75 ml) wird unter trockenem Stickstoff 1 Stunde lang bei Raumtemperatur gerührt. Dann wird eine Lösung von Natriumcyanborhydrid (3,5 g) in Methanol (30 ml) tropfenweise während 4 Stunden hinzugegeben. Das Reaktionsgemisch wird 18 Stunden bei Raumtemperatur gerührt. Dann wird konz. Salzsäure (10 ml) tropfenweise hinzugegeben und das Reaktionsgemisch eine weitere Stunde bei Raumtemperatur gerührt. Die Lösungsmittel werden unter vermindertem Druck abgezogen, zum Rückstand Wasser (400 ml) und Ether (100 ml) gegeben und die Phasen getrennt. Der pH-Wert wird durch Zugabe von 40 % wässeriger Natronlauge auf 9 eingestellt und die etherische Phase verworfen. Durch Zugabe von konz. Salzsäure wird der pH-Wert der wässerigen Phase nun auf 4,3 eingestellt und die Lösung mit Essigsäureethylester (3 × 100 ml) extrahiert.

Die vereinigten Essigsäureethylester-Extrakte werden über Magnesiumsulfat getrocknet und das Lösungsmittel unter vermindertem Druck abgezogen. Der Rückstand wird in Dichlormethan (150 ml) gelöst und HCl-Gas 5 Minuten lang in die Lösung eingeleitet. Das Lösungsmittel wird verdampft und der Rückstand umkristallisiert, wobei man 1-Carboxymethyl-3(1-ethoxycarbonyl-3-phenylpropylamino)-1,3,4,5,6,7-hexahydro-1-benzazonin-2-on erhält.

Das Ausgangsmaterial wird wie folgt hergestellt :

Eine Lösung von 3,4,5,6-Tetrahydro-1H-1-benzazonin-2,7-dion (14,2 g) [J. Am. Chem. Soc. 88, 1049 (1966)] und Natriumborhydrid (1,4 g) in Ethanol (720 ml) wird 18 Stunden bei Raumtemperatur gerührt. Das Ethanol wird unter vermindertem Druck abgezogen und der Rückstand in Dichlormethan (350 ml) aufgenommen. Die Lösung wird nacheinander mit 2N Salzsäure (2 × 200 ml) und gesättigter Kochsalzlösung (100 ml) extrahiert und über Natriumsulfat getrocknet. Nach Verdampfen des Lösungsmittels unter vermindertem Druck erhält man 7-Hydroxy-1,3,4,5,6,7-hexahydro-1-benzazonin-2-on.

Eine Lösung von 7-Hydroxy-1,3,4,5,6,7-hexahydro-1-benzazonin-2-on (9,2 g) in Acetanhydrid (200 ml) wird 3 Stunden bei 80° gehalten. Das Reaktionsgemisch wird auf Raumtemperatur abgekühlt und die Lösungsmittel unter vermindertem Druck verdampft. Ether (300 ml) wird hinzugegeben, die Lösung mit Wasser (150 ml) gewaschen und über Magnesiumsulfat getrocknet. Nach Verdampfen des Lösungsmittels unter vermindertem Druck erhält man 7-Acetoxy-1,3,4,5,6,7-hexahydro-1-benzazonin-2-on.

Eine Lösung von 7-Acetoxy-1,3,4,5,6,7-hexahydro-1-benzazonin-2-on (7,4 g) in Ethanol (250 ml) wird bei einem Druck von 3 at bei 70° unter Verwendung von 10 % Palladium-auf-Kohle (1,5 g) als Katalysator hydriert. Der Katalysator wird abfiltriert und das Lösungsmittel unter vermindertem Druck abgedampft. Der Rückstand wird kristallisiert und man erhält 1,3,4,5,6,7-Hexahydro-1-benzazonin-2-on.

Zu einer Lösung von 1,3,4,5,6,7-Hexahydro-1-benzazonin-2-on (4,7 g) in Chloroform (75 ml) wird Phosphorpentachlorid (5,9 g) portionsweise gegeben, wobei die Temperatur bei 0-5° gehalten wird. Nach erfolgter Zugabe wird Iod (60 mg) hinzugefügt, gefolgt von Brom (4,5 g), letzteres tropfenweise innerhalb von 5 Minuten. Das Gemisch wird 4 Stunden unter Rückfluss gekocht. Das Chloroform wird verdampft, zum Rückstand sowohl ein Eis/Wasser-Gemisch (60 ml) als auch Dichlormethan (100 ml) gegeben und die Phasen getrennt. Die organische Phase wird über Magnesiumsulfat getrocknet und das Lösungsmittel unter vermindertem Druck abgedampft. Das zurückbleibende Rohmaterial wird durch Chromatographie über Kieselgel gereinigt, wobei als Elutionsmittel Ether/Hexan-Mischungen dienen. Nach Einengung der entsprechenden Fraktionen erhält man 3-Brom-1,3,4,5,6,7-Hexahydro-1-benzazonin-2-on.

Eine Lösung von 3-Brom-1,3,4,5,6,7-Hexahydro-1-benzazonin-2-on (14,2 g) und Natriumazid (7,1 g) in Dimethylsulfoxid (400 ml) wird 3 Stunden unter Stickstoff bei 80° gehalten. Das Reaktionsgemisch wird auf Eis/Wasser (1 200 ml) gegossen und die Suspension 30 Minuten gerührt. Die festen Bestandteile werden abfiltriert, mit Wasser (300 ml) gewaschen und getrocknet, wobei man 3-Azido-1,3,4,5,6,7-hexahydro-1-benzazonin-2-on erhält.

Eine Lösung von 3-Azido-1,3,4,5,6,7-hexahydro-1-benzazonin-2-on (8,9 g) in trockenem Dimethylformamid (100 ml) wird innerhalb von 30 Minuten zu einer Lösung von Kalium-tert.-butoxid (4,5 g) in trockenem Dimethylformamid (100 ml), die bei 0° und unter trockenem Stickstoff gehalten wird, gegeben. Der Ansatz wird 2 Stunden bei 0° gerührt, und dann eine Lösung von Bromessigsäureethylester (5,8 g) in Dimethylformamid (10 ml) innerhalb von 5 Minuten hinzugefügt. Das Reaktionsgemisch wird 2 Stunden bei 0° gerührt und dann unter vermindertem Druck eingedampft. Zum Rückstand wird Essigsäureethylester (250 ml) und Wasser (150 ml) gegeben, die Phasen werden getrennt, die Essigsäureethylester-Phase wird mit Wasser (100 ml) gewaschen und über Natriumsulfat getrocknet. Das Lösungsmittel wird unter vermindertem Druck abgedampft, und man erhält 3-Azido-1-ethoxycarbonylmethyl-1,3,4,5,6,7-hexahydro-1-benzazonin-2-on.

Eine Lösung von 3-Azido-1-ethoxycarbonylmethyl-1,3,4,5,6,7-hexahydro-1-benzazonin-2-on (5,0 g) in Ethanol (500 ml) wird bei atmosphärischem Druck unter Verwendung von 10 % Palladium-auf-Kohle (0,7 g) als Katalysator hydriert. Der Katalysator wird abfiltriert und das Lösungsmittel unter vermindertem Druck verdampft, wobei man 3-Amino-1-ethoxycarbonylmethyl-1,3,4,5,6,7-hexahydro-1-benzazonin-2-on erhält.

Eine Lösung von Natriumhydroxid (2,0 g) in Wasser (5 ml) wird zu einer Lösung von 3-Amino-1-ethoxycarbonylmethyl-1,3,4,5,6,7-hexahydro-1-benzazonin-2-on (15,0 g) in Methanol (150 ml) bei Raumtemperatur gegeben und das Gemisch 2 Stunden gerührt. Die Lösungsmittel werden abgedampft und der Rückstand gründlich getrocknet, wobei man das Natriumsalz von 3-Amino-1-carboxymethyl-1,3,4,5,6,7-hexahydro-1-benzazonin-2-on erhält.

## Beispiel 3

Gemäss den in den vorherigen Beispielen beschriebenen Verfahren wird 6-Acetoxy-3,4,5,6-tetrahydro-1-benzazocin-2-1H-on in 6-Acetoxy-1-carboxymethyl-3-(1-ethoxycarbonyl-3-phenylpropylamino)-3,4,5,6-tetrahydro-1-benzazocin-2-1H-on umgewandelt.

Die Ausgangsverbindung wird wie folgt hergestellt :

Eine Lösung von 4,5-Dihydro-1-benzazocin-2,6-1H,3H-dion (14,2 g) [Tetrahedron Letters 4079-4082 (1976)] und Natriumborhydrid (1,4 g) in Ethanol (720 ml) wird 18 Stunden bei Raumtemperatur gerührt. Das Ethanol wird unter vermindertem Druck abgedampft und der Rückstand in Dichlormethan (350 ml) gelöst. Die Lösung wird mit 2N Salzsäure (2 × 200 ml) extrahiert, dann mit gesättigter Kochsalzlösung (100 ml) gewaschen und über Natriumsulfat getrocknet. Nach Verdampfen des Lösungsmittels unter vermindertem Druck erhält man 6-Hydroxy-3,4,5,6-tetrahydro-1-benzazocin-2-1H-on.

Eine Lösung von 6-Hydroxy-3,4,5,6-tetrahydro-1-benzazocin-2-1H-on (9,2 g) in Acetanhydrid (200 ml) wird 3 Stunden bei 80° gehalten. Das Reaktionsgemisch wird auf Raumtemperatur abgekühlt und die Lösungsmittel unter vermindertem Druck verdampft. Man gibt Ether (300 ml) hinzu, wäscht die Lösung mit Wasser (150 ml) und trocknet sie über Magnesiumsulfat. Das Lösungsmittel wird unter vermindertem Druck verdampft, und man erhält 6-Acetoxy-3,4,5,6-tetrahydro-1-benzazocin-2-1H-on.

## Beispiel 4

a) Eine Lösung von 3-[(5-Benzyloxycarbonylamino-1-(S)-methoxycarbonylpentyl) amino]-1-ethoxycarbonylmethyl-3,4,5,6-tetrahydro-1-benzazocin-2-1H-on (Isomer B) (3,2 g) in Methanol (20 ml) wird zu einer Lösung von Natriumhydroxid (1,0 g) in Wasser (3 ml) gegeben. Das Reaktionsgemisch wird 2 Stunden bei Raumtemperatur gerührt und dann durch Zugabe von 2N Salzsäure angesäuert.

Die Lösung wird unter vermindertem Druck eingedampft, und man erhält 3-[(5-Benzyloxycarbonylamino-1-(S)-carboxypentyl) amino]-1-carboxymethyl-3,4,5,6-tetrahydro-1-benzazocin-2-1H-on (Isomer B).

b) Eine Lösung von 3-[(5-Benzyloxycarbonylamino-1-(S)-methoxycarbonylpentyl) amino]-1-ethoxycarbonylmethyl-3,4,5,6-tetrahydro-1-benzazocin-2-1H-on (Isomer A) (2,6 g) in Methanol (15 ml) wird zu einer Lösung von Natriumhydroxid (0,8 g) in Wasser (2,5 ml) gegeben. Das Reaktionsgemisch wird 2 Stunden bei Raumtemperatur gerührt und dann durch Zugabe von 2N Salzsäure angesäuert. Die Lösung wird unter vermindertem Druck eingedampft, und man erhält 3-[(5-Benzyloxycarbonylamino-1-(S)-carboxypentyl) amino]-1-carboxymethyl-3,4,5,6-tetrahydro-1-benzazocin-2-1H-on (Isomer A).

Die Ausgangsmaterialien werden wie folgt hergestellt: 3-Amino-1-ethoxycarbonylmethyl-3,4,5,6-tetrahydro-1-benzazocin-2-1H-on (8,0 g), Essigsäure (0,4 ml) und tert.-Butylnitrit (4,2 ml) werden in Chloroform (150 ml) gelöst, 2 Stunden unter Rückfluss gekocht und dann auf Raumtemperatur abgekühlt. m-Chlorperbenzoesäure (5,7 g) wird portionsweise hinzugegeben und die Lösung 30 Minuten bei Raumtemperatur gerührt. Sie wird mit gesättigter Natriumhydrogencarbonat-Lösung (120 ml), dann mit Wasser (75 ml), mit 2N Salzsäure (50 ml), wieder mit Wasser (50 ml), 2N Salzsäure (50 ml) und schliesslich mit Wasser (50 ml) gewaschen. Die Lösung wird über Magnesiumsulfat getrocknet, das Lösungsmittel unter vermindertem Druck verdampft, und man erhält 1-Ethoxycarbonylmethyl-5,6-dihydro-1-benzazocin-2,3-1H,4H-dion.

Eine Lösung von 1-Ethoxycarbonylmethyl-5,6-dihydro-1-benzazocin-2,3-1H,4H-dion (4,2 g), ε-Benzyloxycarbonyllysinmethylester (4,8 g) und Dibutylzinndichlorid (0,32 g) in Dichlormethan (100 ml) wird mit 4 Å Molekularsieb (45 g) 48 Stunden unter Rückfluss gekocht. Das Reaktionsgemisch wird auf Raumtemperatur abgekühlt, filtriert und das Lösungsmittel unter vermindertem Druck abgedampft, wobei man ein Produkt erhält, das in Methanol (100 ml) und Essigsäure (5 ml) gelöst wird. Natriumcyanborhydrid (0,36 g) wird nach 10 Minuten hinzugegeben und das Reaktionsgemisch bei Raumtemperatur 65 Stunden lang gerührt. Dann wird der Ansatz mit konz. Salzsäure angesäuert und unter vermindertem Druck eingedampft. Zum Rückstand wird Essigsäureethylester (150 ml) und 2N Salzsäure gegeben, die Phasen werden getrennt, die wässerige Phase wird basisch gemacht und mit Dichlormethan (2 × 100 ml) extrahiert. Die Extrakte werden über Magnesiumsulfat getrocknet und das Lösungsmittel unter vermindertem Druck verdampft, wobei man ein Oel erhält, das in 2 diskrete Fraktionen, nämlich das Isomer A und das Isomer B von 3-[(5-Benzyloxycarbonylamino-1-(S)-methoxycarbonylpentyl) amino]-1-ethoxycarbonylmethyl-3,4,5,6-tetrahydro-1-benzazocin-2-1H-on, aufgetrennt wird.

## Beispiel 5

a) Eine Lösung von 3-[(5-Benzyloxycarbonylamino-1-(S)-carboxypentyl) amino]-1-carboxymethyl-3,4,5,6-tetrahydro-1-benzazocin-2-1H-on (Isomer B) (2,1 g) in Ethanol (300 ml) wird bei atmosphärischem Druck unter Verwendung von 5 % Palladium-auf-Kohle (0,5 g) als Katalysator hydriert. Der Katalysator

wird abfiltriert, das Lösungsmittel unter vermindertem Druck verdampft, und man erhält 3-[(5-Amino-1-(S)-carboxypentyl) amino]-1-carboxymethyl-3,4,5,6-tetrahydro-1-benzazocin-2-1H-on (Isomer B).

b) In ähnlicher Weise wird 3-[(5-Benzyloxycarbonylamino-1-(S)-carboxypentyl) amino]-1-carboxymethyl-3,4,5,6-tetrahydro-1-benzazocin-2-1H-on (Isomer A) zu 3-[(5-Amino-1-(S)-carboxypentyl) amino]-1-carboxymethyl-3,4,5,6-tetrahydro-1-benzazocin-2-1H-on (Isomer A) hydriert.

Beispiel 6

Gemäss den in den vorangehenden Beispielen beschriebenen Verfahren können die folgenden Verbindungen der Formel Ia und Derivate davon, in denen $R^2$ und $R^5$ Wasserstoff bedeuten, X für 2 Wasserstoffe steht, $R^6$ für Hydroxy oder Ethoxy steht, $R^7$ Hydroxy ist und m für 2 oder 3 steht, hergestellt werden, vorteilhaft als S,S-Isomere.

| Verbindungen | $R^3$ | $R^4$ | $R^1$ | m | Derivat |
|---|---|---|---|---|---|
| 6a | $OCH_3$ | $OCH_3$ | $CH_2CH_2C_6H_5$ | 3 | - |
| 6b | H | H | $(CH_2)_3NHCOOCH_2C_6H_5$ | 2,3 | - |
| 6c | H | H | $(CH_2)_4NHCOOCH_2C_6H_5$ | 3 | - |
| 6d | H | H | $CH_2CH_2C_6H_5$ | 2,3 | Perhydro |
| 6e | H | Cl | $CH_2CH_2C_6H_5$ | 2 | - |
| 6f | H | H | $CH_2COOEt$ | 2,3 | - |
| 6g | H | H | n-Propyl | 2,3 | - |
| 6h | H | H | $(CH_2)_4NHCOOCH_2C_6H_5$ | 2,3 | Perhydro |
| 6i | H | H | Phenoxyethyl | 2,3 | - |
| 6j | H | H | Phenylthioethyl | 2,3 | - |

Ausgangsverbindungen für

6a — beschrieben in J. Chem. Soc. Perkin II, 733 (1978)

6b — L-Ornithin

6c — L-Lysin

6d, 6h — 3-Amino-1-carboxymethyl-3,4,5,6-tetrahydro-1-benzazocin-2-1H-on wird mit Rutheniumauf-Aluminium-Katalysator in Eisessig bei 120° und einem Druck von 100 at hydriert, wobei man 3-Amino-1-carboxymethylperhydro-1-benzazocin-2-on erhält; 3-Amino-1-carboxymethylperhydrobenzazonin-2-on kann auf ähnliche Weise hergestellt werden.

6e — beschrieben in J. Chem. Soc. (C) 2176 (1969)

6f — L-Asparaginsäure

6g — 2-Oxopentansäureethylester

6i — 4-Phenoxy-2-aminobutansäureethylester

6j — 4-Phenylthio-2-aminobutansäureethylester

Beispiel 7

a) Zu 1-Ethoxycarbonylmethyl-3-(1-ethoxycarbonyl-3-phenyl-(1S)-propylamino)-3,4,5,6-tetrahydro-(3S)-1-benzazocin-2-1H-on (0,56 g) in 40 ml Methanol wird bei Raumtemperatur eine Lösung von Natriumhydroxid (0,11 g) in 5 ml Wasser gegeben. Das Gemisch wird 2 Stunden bei Raumtemperatur gerührt. Der Ansatz wird eingedampft, in 5 ml Wasser gelöst und mit 2 × 50 ml Diethylether gewaschen. Die wässerige Phase wird mit 6N Salzsäure auf pH 2 eingestellt, wobei ein Niederschlag ausfällt. Der Feststoff wird abfiltriert, im Vakuum bei 50° getrocknet, und man erhält 1-Carboxymethyl-3-(1-carboxy-3-phenyl-(1S)-propylamino)-3,4,5,6-tetrahydro-(3S)-1-benzazocin-2-1H-on-Hydrochlorid, Fp. 148-150°, $\alpha_D = -24°$ (c = 1.0, Methanol).

b) Verwendet man als Ausgangsmaterial 1-Ethoxycarbonylmethyl-3-(1-ethoxycarbonyl-3-phenyl-(1S)-propylamino)-3,4,5,6-tetrahydro-(3R)-1-benzazocin-2-1H-on (1,0 g) in 50 ml Methanol und Natriumhydroxid in 5 ml Wasser und folgt dem unter a) beschriebenen Verfahren, so erhält man 1-Carboxymethyl-3-(1-carboxy-3-phenyl-(1S)-propylamino)-3,4,5,6-tetrahydro-(3R)-1-benzazocin-2-1H-on-Hydrochlorid, Fp. 169-171°, $\alpha_D = +104°$ (c = 1.0, Methanol).

Die Ausgangsverbindungen werden wie folgt hergestellt:

Zu 3,4,5,6-Tetrahydro-1-benzazocin-2-1H-on (12,0 g), hergestellt wie von R. Huisgen und L. Krause in Ann. Chem. 574, 171 (1951) beschrieben, in 150 ml Chloroform bei 0° werden nacheinander Phosphorpentachlorid (15,0 g) und Iod (0,15 g) gegeben. Man rührt 30 Minuten lang und gibt dann Brom (12,0 g) innerhalb von 5 Minuten hinzu. Der Ansatz wird 4 Stunden unter Rückfluss gekocht, dann auf Raumtemperatur abgekühlt und auf ein Eis/Wasser-Gemisch gegeben. Die Chloroformphase wird mit 50 ml Wasser gewaschen, über Magnesiumsulfat getrocknet und das Lösungsmittel verdampft, wobei man 3-Brom-3,4,5,6-tetrahydro-1-benzazocin-2-1H-on erhält, das gemäss Dünnschichtchromatographie auf Kieselgel homogen ist ($R_f = 0,6$ in 1 : 1 Essigsäureethylester/Toluol) und folgende physikalischen Daten aufweist : NMR ($CDCl_3$) 8.56 (1H, breit), 6.69-7.94 (4H, m), 4.34 (1H,t) 1.02-3.33 (6H, m) ; MS (m/e) 253.

Die beschriebene Bromverbindung (1,0 g) und Natriumazid (0,30 g) werden in 20 ml Dimethylsulfoxid 5 Stunden bei 60° gerührt. Das Gemisch wird in 125 ml kaltes Wasser gegossen, wobei man einen Feststoff erhält, der abfiltriert und im Vakuum bei 60° getrocknet wird. Er entspricht 3-Azido-3,4,5,6-tetrahydro-1-benzazocin-2-1H-on mit folgenden Daten : IR (Nujol) 2110, 1650 $cm^{-1}$-NMR (DMSO-$d_6$) 10.2 (1H, breit), 7.02-7.61 (4H, m), 3.36 (1H, m), 1.18-2.95 (6H, m) ; MS (m/e) 188 (M-28), 187, 132.

Das beschriebene Azid (5,0 g), gelöst in 250 ml Tetrahydrofuran, wird bei Raumtemperatur mit Kalium-tert.-butoxid (2,9 g) versetzt. Man lässt 1 Stunde rühren und gibt dann Bromessigsäureethylester (4,3 g), gelöst in 10 ml Tetrahydrofuran, hinzu. Der Ansatz wird 36 Stunden bei Raumtemperatur gerührt und dann eingedampft. Der Rückstand wird mit 50 ml Wasser versetzt und mit 3 × 50 ml Methylenchlorid extrahiert. Die organischen Extrakte werden über Magnesiumsulfat getrocknet, eingedampft, und man erhält 1-Ethoxycarbonylmethyl-3-oxo-3,4,5,6-tetrahydro-1-benzazocin-2-1H-on, IR ($CHCl_3$) 1640, 1720, 1750 $cm^{-1}$ ; MS (m/e) 274, 247, 202, 174.

Dieses Keton (2,75 g) und 2-Amino-4-phenyl-(2S)-butansäureethylester (2,1 g), zusammen mit 200 ml Chloroform, das Di-n-butylzinndichlorid (0,15 g enthält, werden 24 Stunden unter Rückfluss gekocht, wobei ein Wasserabscheider verwendet wird. Das Reaktionsgemisch wird eingedampft, und man erhält das rohe Imin, das direkt weiterverwendet wird.

Zum erwähnten Imin (4,6 g), gelöst in 75 ml Methanol und 15 ml Essigsäure, wird Natriumcyanborhydrid (0,85 g) gegeben. Der Ansatz wird 20 Stunden bei Raumtemperatur gerührt. 2,5 ml 12N Salzsäure werden hinzugefügt und das Reaktionsgemisch eingedampft. Der Rückstand wird mit 50 ml 10 % wässeriger Natriumcarbonatlösung versetzt und mit 3 × 75 ml Methylenchlorid extrahiert. Die vereinigten organischen Extrakte werden über Magnesiumsulfat getrocknet und das Lösungsmittel verdampft, wobei man ein Oel erhält. Die diastereomeren Amine werden durch Blitz-Säulenchromatographie getrennt (Kieselgel, 3 : 1 Toluol/Essigsäureethylester), und man erhält 1-Ethoxycarbonylmethyl-3-(1-ethoxycarbonyl-3-phenyl-(1S)-propylamino)-3,4,5,6-tetrahydro-(3R)-1-benzazocin-2-1H-on, $R_f = 0.5$ in 1 : 1 Essigsäureethylester/Toluol, NMR ($CDCl_3$) 6.93-7.42 (9H, m), 4.41 (2H, s), 4.13 (4H, q, J = 7), 1.22 (3H, t, J = 7) und 1-Ethoxycarbonylmethyl-3-(1-ethoxycarbonyl-3-phenyl)-(1S)-propylamino)-3,4,5,6-tetrahydro-(3S)-1-benzazocin-2-1H-on, $R_f = 0.48$ in 1 : 1 Essigsäureethylester/Toluol, NMR ($CDCl_3$), 7.24 (9H, m), 4.44 (2H, s), 4.19 (4H, q, J = 7), 1.26 (3H, t, J = 7), 1.02 (3H, t, J = 7).

## Beispiel 8

a) 1-Benzyloxycarbonylmethyl-3-(1-ethoxycarbonyl-3-phenyl-(1S)-propylamino)-3,4,5,6-tetrahydro-(3S)-1-benzazocin-2-1H-on (1,0 g) in 150 ml Ethanol wird bei einem Druck von 1 at in Gegenwart von 0,5 g 10 % Palladium-auf-Kohle hydriert. Das Reaktionsgemisch wird filtriert und eingedampft. HCl-Gas wird durch eine Lösung des Rückstandes in 100 ml Diethylether geleitet. Durch Filtration erhält man 1-Carboxymethyl-3-(1-ethoxycarbonyl-3-phenyl-(1S)-propylamino)-3,4,5,6-tetrahydro-(3S)-1-benzazocin-2-1H-on-Hydrochlorid, Fp. 96-98°, $\alpha_D$ = — 15° (c = 1.0, in Methanol).

b) Geht man von 1-Benzyloxycarbonylmethyl-3-(1-ethoxycarbonyl-3-phenyl-(1S)-propylamino)-3,4,5,6-tetrahydro-(3R)-1-benzazocin-2-1H-on (2,2 g) aus und verfährt wie unter a) angegeben, so erhält man 1-Carboxymethyl-3-(1-ethoxycarbonyl-3-phenyl-(1S)-propylamino)-3,4,5,6-tetrahydro-(3R)-1-benzazocin-2-1H-on-Hydrochlorid.

Die Ausgangsverbindungen werden wie folgt hergestellt :

Zu 3-Azido-3,4,5,6-tetrahydro-1-benzazocin-2-1H-on (4,0 g), gelöst in 250 ml Tetrahydrofuran, wird Kalium-tert.-butoxid (2,3 g) gegeben. Man lässt 1 Stunde bei Raumtemperatur rühren und gibt dann Bromessigsäurebenzylester (4,7 g), gelöst in 10 ml Tetrahydrofuran, hinzu. Der Ansatz wird 36 Stunden bei Raumtemperatur gerührt und dann eingedampft. Man nimmt den Rückstand in 50 ml Wasser auf und extrahiert mit 3 × 50 ml Methylenchlorid. Die organischen Extrakte werden über Magnesiumsulfat getrocknet, eingedampft, und man erhält 1-Benzyloxycarbonylmethyl-3-oxo-3,4,5,6-tetrahydro-1-benzazocin-2-1H-on, IR (reine Substanz) 1660, 1720, 1750 $cm^{-1}$.

Das soeben beschriebene Keton (5,9 g) und 2-Amino-4-phenyl-(2S)-butansäureethylester (3,8 g) werden in 200 ml Chloroform, das Di-n-butylzinndichlorid (0,15 g) enthält, 24 Stunden unter Rückfluss gekocht, wobei ein Wasserabscheider verwendet wird. Das Reaktionsgemisch wird eingedampft und man erhält das rohe Imin, welches direkt weiterverwendet wird.

Zu dem oben genannten Imin (9,0 g), gelöst in 80 ml Methanol und 15 ml Essigsäure, wird Natriumcyanborhydrid (1,4 g) gegeben. Das Reaktionsgemisch wird 18 Stunden bei Raumtemperatur

gerührt. Man gibt 3,0 ml 12N Salzsäure hinzu und dampft den Ansatz ein. Der Rückstand wird in 75 ml 10 % wässeriger Natriumcarbonat-Lösung aufgenommen und mit 3 × 75 ml Methylenchlorid extrahiert. Die vereinigten organischen Extrakte werden über Magnesiumsulfat getrocknet und eingedampft, wobei man ein Oel erhält. Die diastereomeren Amine werden durch Blitz-Säulenchromatographie (Kieselgel, 3 : 1 Toluol/Essigsäureethylester) getrennt, und man erhält 1-Benzyloxycarbonylmethyl-3-(1-ethoxycarbonyl-3-phenyl-(1S)-propylamino)-3,4,5,6-tetrahydro-(3R)-1-benzazocin-2-1H-on, $R_f$ = 0,53 in 1 : 1 Essigsäureethylester/Toluol ; NMR ($CDCl_3$) 7.31 (5H, s), 7.18 (9H, m), 5.07 (2H, s), 4.36 (2H, s), 4.02 (2H, q, J = 7), 1.11 (3H, t, J = 7), sowie 1-Benzyloxycarbonylmethyl-3-(1-ethoxycarbonyl-3-phenyl-(1S)-propylamino)-3,4,5,6-tetrahydro-(3S)-1-benzazocin-2-1H-on, $R_f$ = 0.50 in 1 : 1 Essigsäureethylester/Toluol ; NMR ($CDCl_3$) 7.35 (5H, s), 7.21 (9H, m), 5.18 (2H, s), 4.02 (2H, q, J = 7), 1.01 (3H, t, J = 7).

## Beispiel 9

a) Ausgehend von 1,3,4,5,6,7-Hexahydro-1-benzazonin-2-on [Ann. Chem. 586, 30 (1954)] kann man 1-Carboxymethyl-3-(1-carboxy-3-phenyl-(1S)-propylamino)-1,3,4,5,6,7-hexahydro-(3S)-1-benzazonin-2-on-Hydrochlorid gemäss dem in Beispiel 7a) beschriebenen Verfahren herstellen.

b) 1-Carboxymethyl-3-(1-carboxy-3-phenyl)-(1S)-propylamino)-1,3,4,5,6,7-hexahydro-(3R)-1-benzazonin-2-on-Hydrochlorid wird gemäss dem in Beispiel 7b) beschriebenen Verfahren hergestellt.

c) 1-Carboxymethyl-3-(1-ethoxycarbonyl-3-phenyl-(1S)-propylamino)-1,3,4,5,6,7-hexahydro-(3S)-1-benzazonin-2-on-Hydrochlorid wird gemäss dem in Beispiel 8a) beschriebenen Verfahren hergestellt.

d) 1-Carboxymethyl-3-(1-ethoxycarbonyl-3-phenyl-(1S)-propylamino)-1,3,4,5,6,7-hexahydro-(3R)-1-benzazonin-2-on-Hydrochlorid wird gemäss dem in Beispiel 8b) beschriebenen Verfahren hergestellt.

## Beispiel 10

Eine Lösung von 1-Ethoxycarbonylmethyl-3,4,5,6-tetrahydro-1-benzazocin-2,3-1H-dion (7,0 g, 25,5 mmol) und ε-N-Benzyloxycarbonyl-L-lysinethylester (7,9 g, 25 mmol) in 300 ml Chloroform wird unter Verwendung eines Wasserabscheiders in Gegenwart von Dibutylzinndichlorid (0,40 g) unter Rückfluss gekocht. Nach 2 Stunden wird der Ansatz eingedampft, und man erhält das rohe Imin (15,0 g), das ohne Reinigung weiterverwendet wird.

Zum genannten Imin (15,0 g), gelöst in 8 ml Essigsäure und 100 ml Methanol, wird Natriumcyanborhydrid (3,5 g) gegeben. Der Ansatz wird über Nacht bei Raumtemperatur gerührt und dann die Reaktion durch Zugabe von 5 ml 12N Salzsäure abgebrochen. Nach 1-stündigem Rühren wird das Reaktionsgemisch eingedampft. Der Rückstand wird mit 2N Natronlauge auf pH 9 eingestellt und mit 3 × 150 ml Essigsäureethylester extrahiert. Die vereinigten organischen Extrakte werden über Magnesiumsulfat getrocknet, eingedampft, und man erhält 3-[(5-Benzyloxycarbonylamino-1-ethoxycarbonylpentyl)-amino]-1-ethoxycarbonylmethyl-3,4,5,6-tetrahydro-1-benzazocin-2-1H-on als Diastereomerengemisch.

Dieses Diastereomerengemisch wird durch Blitz-Säulenchromatographie (Kieselgel, 1 : 1 Toluol/Essigsäureethylester) getrennt, und man erhält das SR-Isomer ($R_f$ = 0.30) und das SS-Isomer ($R_f$ = 0.25).

## Beispiel 11

Eine Lösung von 3-[(5-Benzyloxycarbonylamino-1-(S)-ethoxycarbonylpentyl)amino]-1-ethoxycarbonylmethyl-3,4,5,6-tetrahydro-(3S)-1-benzazocin-2-1H-on (1,7 g) in 60 ml Methanol und Natronlauge, hergestellt aus 0,30 g Natriumhydroxid und 5 ml Wasser, wird 18 Stunden bei Raumtemperatur gerührt und dann eingedampft. Der Rückstand wird in 50 ml Wasser aufgenommen und mit 150 ml Ether gewaschen. Die Etherphase wird verworfen. Die wässerige Phase wird mit 2N Salzsäure auf pH 2,5 eingestellt, auf 15 ml eingeengt, wobei ein Feststoff ausfällt, der abfiltriert wird. Er entspricht 3-[(5-Benzyloxycarbonylamino-1-(S)-carboxypentyl)amino]-1-carboxymethyl-3,4,5,6-tetrahydro-(3S)-1-benzazocin-2-1H-on, Fp. 122-125°, $\alpha_D$ = —70° (c = 1.0, Methanol). Die SR-Disäure, $\alpha_D$ = + 101° (c = 1.0, Ethanol), wird in ähnlicher Weise hergestellt.

## Beispiel 12

Eine Lösung von 3-[(5-Benzyloxycarbonylamino-1-(S)-carboxypentyl)amino]-1-carboxymethyl-3,4,5,6-tetrahydro-(3S)-1-benzazocin-2-1H-on (0,60 g) in 20 ml Wasser wird bei atmosphärischem Druck unter Verwendung von 0,8 g 10 % Palladium-auf-Kohle hydriert. Das Reaktionsgemisch wird filtriert, das Filtrat eingedampft, und man erhält 0,33 g 3-[(5-Amino-1-(S)-carboxypentyl)amino]-1-carboxymethyl-3,4,5,6-tetrahydro-(3S)-1-benzazocin-2-1H-on. Die entsprechende SR-Disäure wird in ähnlicher Weise hergestellt.

Beispiel 13

Herstellung von 10 000 Tabletten mit einem Gehalt von je 10 mg an 1-Carboxymethyl-3-(1-ethoxycarbonyl-3-phenylpropylamino)-3,4,5,6-tetrahydro-1-benzazocin-2-1H-on-Hydrochlorid :

Bestandteile :

| | |
|---|---|
| 1-Carboxymethyl-3-(1-ethoxycarbonyl-3-phenylpropylamino)-3,4,5,6-tetrahydro-1-benzazocin-2-1H-on-Hydrochlorid | 100 g |
| Milchzucker | 1 157 g |
| Maisstärke | 75 g |
| Polyethylenglykol 6000 | 75 g |
| Talkpulver | 75 g |
| Magnesiumstearat | 18 g |
| gereinigtes Wasser | q.s. |

Verfahren

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyethylenglykol in 150 ml Wasser gegeben. Die erhaltene Paste wird zu den Pulvern gegeben und gegebenenfalls unter Zugabe von mehr Wasser granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Beispiel 14

Herstellung einer injizierbaren Formulierung mit einem Gehalt von je 25 mg an 1-Carboxymethyl-3-(1-ethoxycarbonyl-3-phenylpropylamino)-3,4,5,6-tetrahydro-1-benzazocin-2-1H-on-Hydrochlorid :

Bestandteile :

| | |
|---|---|
| 1-Carboxymethyl-3-(1-ethoxycarbonyl-3-phenylpropylamino)-3,4,5,6-tetrahydro-1-benzazocin-2-1H-on-Hydrochlorid | 25 g |
| Propylparaben | 1 g |
| Wasser zur Injektion q.s. | 5 000 ml |

Der Wirkstoff und das Konservierungsmittel werden in 3 500 ml Wasser zur Injektion gelöst und die Lösung auf 5 000 ml verdünnt. Sie wird durch ein steriles Filter filtriert und unter keimfreien Bedingungen in Injektionsampullen abgefüllt, wobei in jede Ampulle 5 ml der Lösung gegeben werden.

Beispiel 15

Herstellung von 10 000 Kapseln mit einem Gehalt von je 20 mg an 1-Carboxymethyl-3-(1-ethoxycarbonyl-3-phenylpropylamino)-3,4,5,6-tetrahydro-1-benzazocin-2-1H-on-Hydrochlorid :

Bestandteile :

| | |
|---|---|
| 1-Carboxymethyl-3-(1-ethoxycarbonyl-3-phenylpropylamino)-3,4,5,6-tetrahydro-1-benzazocin-2-1H-on-Hydrochlorid | 200 g |
| Milchzucker | 1 700 g |
| Talkpulver | 100 g |

Verfahren

Sämtliche pulverigen Bestandteile werden mit einem Sieb von 0,6 mm Maschenweite gesiebt. Dann wird der Wirkstoff zuerst mit Talk und nachher mit Milchzucker in einem geeigneten Mischer homogenisiert. Kapseln Nr. 3 werden mit je 200 mg der erhaltenen Mischung in einer Füllmaschine abgefüllt.

In analoger Weise werden auch Tabletten, injizierbare Formulierungen und Kapseln von anderen Verbindungen der Erfindung, z. B. den in den Beispielen beschriebenen, hergestellt.

**Patentansprüche** (für die Vertragsstaaten : BE, CH, DE, FR, GB, IT, LI, LU, NL, SE)

1. Verbindungen der allgemeinen Formel I

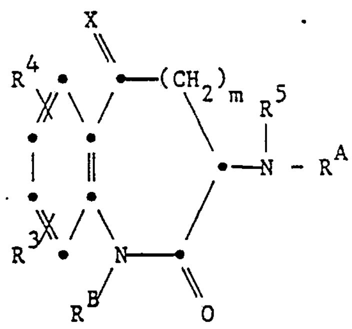

(I)

worin $R^A$ und $R^B$ Reste der Formel

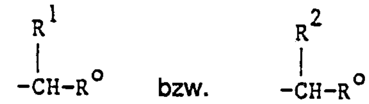

darstellen, worin $R^0$ Carboxy oder ein funktionell modifiziertes Carboxy darstellt; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Aryl-(thio-, sulfinyl- oder sulfonyl-)-niederalkyl, Aryl-N-niederalkylamino-niederalkyl oder Arylamino-niederalkyl bedeutet; $R^2$ für Wasserstoff oder Niederalkyl steht; $R^3$ und $R^4$ unabhängig voneinander Wasserstoff, Niederalkyl, Niederalkoxy, Niederalkanoyloxy, Hydroxy, Halogen oder Trifluormethyl bedeuten, oder $R^3$ und $R^4$ gemeinsam für Niederalkylendioxy stehen; $R^5$ Wasserstoff oder Niederalkyl bedeutet; m für 2 oder 3 steht; und X Oxo, zwei Wasserstoffatome oder Hydroxy oder acyliertes Hydroxy zusammen mit einem Wasserstoffatom bedeutet; und worin der carbocyclische Ring auch perhydriert sein kann; Salze und Stereoisomere von allen diesen Verbindungen.

2. Verbindungen der Formel IA

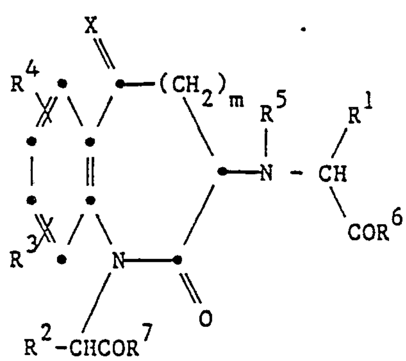

(IA)

worin der carbocyclische Ring auch perhydriert sein kann; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Arylthio-niederalkyl, Aryl-N-niederalkylamino-niederalkyl oder Arylamino-niederalkyl bedeutet; $R^2$ und $R^5$ Wasserstoff oder Niederalkyl bedeuten; $R^3$ und $R^4$ Wasserstoff, Niederalkyl, Niederalkoxy, Niederalkanoyloxy, Hydroxy, Halogen oder Trifluormethyl bedeuten oder $R^3$ und $R^4$ gemeinsam Niederalkylendioxy darstellen; X Oxo, zwei Wasserstoffatome, oder eine Hydroxygruppe oder acylierte Hydroxygruppe und ein Wasserstoff bedeutet; m für 2 oder 3 steht; $R^6$ und $R^7$ unabhängig voneinander Hydroxy, Amino, Mono- oder Di-niederalkylamino, Niederalkoxy, Aryl-niederalkoxy, Niederalkanoyloxymethoxy (Amino, Mono- oder Di-niederalkylamino, Carboxy oder Niederalkoxycarbonyl)-niederalkoxy bedeuten; oder deren Salze.

3. Verbindungen der im Anspruch 2 gezeigten Formel IA, worin $R^1$ Wasserstoff, Niederalkyl, Amino-niederalkyl, Acylamino-niederalkyl, Aryl-niederalkyl, worin Aryl unsubstituiertes Phenyl oder durch Niederalkyl, Hydroxy, Niederalkoxy, Niederalkylendioxy, Niederalkanoyloxy, Halogen oder Trifluormethyl

mono- oder disubstituiertes Phenyl ist, bedeutet; $R^3$ und $R^4$ Wasserstoff, Niederalkyl, Niederalkoxy, Halogen oder Trifluormethyl bedeuten oder $R^3$ und $R^4$ gemeinsam für Alkylendioxy stehen; $R^6$ und $R^7$ unabhängig voneinander Hydroxy, Amino, Niederalkoxy, Phenyl-niederalkoxy, Niederalkoxycarbonyl-niederalkoxy bedeuten; $R^2$, $R^5$, X und m die obigen Bedeutungen haben, oder ihre Salze; oder die genannten Verbindungen, worin der carbocyclische Ring perhydriert ist.

4. Verbindungen der im Anspruch 2 gezeigen Formel IA, worin $R^1$ Wasserstoff, Niederalkyl, ω-Amino-niederalkyl, ω-Arylmethoxycarbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ für Wasserstoff steht; $R^4$ Wasserstoff, Niederalkyl, Niederalkoxy, Halogen oder Trifluormethyl bedeutet; X für Oxo, zwei Wasserstoffatome, oder eine Hydroxy- oder Niederalkanoyloxygruppe und ein Wasserstoffatom steht; m 2 oder 3 bedeutet; $R^6$ und $R^7$ unabhängig voneinander für Hydroxy, Amino, Niederalkoxy, Phenyl-niederalkoxy, Niederalkoxycarbonyl-niederalkoxy stehen; oder ihre Salze; oder die genannten Verbindungen, worin der carbocyclische Ring perhydriert ist.

5. Verbindungen der Formel IB

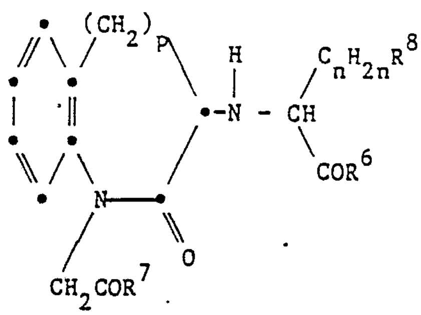

(IB)

worin n eine ganze Zahl von 1 bis 4 bedeutet, p für die ganze Zahl 3 oder 4 steht, $R^8$ Wasserstoff, Amino, Benzyloxycarbonylamino, unsubstituiertes Phenyl oder Phenyl, monosubstituiert durch Niederalkyl, Niederalkoxy, Niederalkanoyloxy, Halogen, Hydroxy oder Trifluormethyl, bedeutet; $R^6$ und $R^7$ unabhängig voneinander Hydroxy, Niederalkoxy mit bis zu 4 Kohlenstoffatomen, Benzyloxy oder Amino bedeuten, oder ihre Salze; oder die genannten Verbindungen, worin der carbocyclische Ring perhydriert ist.

6. Verbindungen der Formel IC

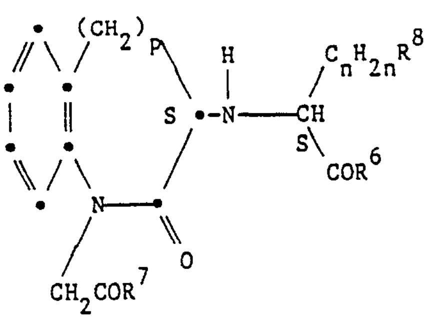

(IC)

worin S die Chiralität bedeutet, n eine ganze Zahl von 1 bis 4 bedeutet; p für die ganze Zahl 3 oder 4 steht, $R^8$ Wasserstoff, Amino, Benzyloxycarbonylamino, unsubstituiertes Phenyl oder durch Niederalkyl, Niederalkoxy, Niederalkanoyloxy, Halogen, Hydroxy oder Trifluormethyl monosubstituiertes Phenyl bedeutet; $R^6$ und $R^7$ unabhängig voneinander Hydroxy, Niederalkoxy mit bis zu 4 Kohlenstoffatomen, Benzyloxy oder Amino bedeuten; oder ihre Salze.

7. 1-Carboxymethyl-3-(1-ethoxycarbonyl-3-phenylpropylamino)-3,4,5,6-tetrahydro-1-benzazocin-2-1H-on und seine Salze.

8. Eine Verbindung aus der Gruppe 1-Carboxymethyl-3-(1-carboxy-3-phenylpropylamino)-3,4,5,6-tetrahydro-1-benzazocin-2-1H-on und 1-Carboxymethyl-3-(1-ethoxycarbonyl-3-phenylpropylamino)-1,3,4,5,6,7-hexahydro-1-benzazonin-2-on und deren Salzen.

9. Eine Verbindung aus der Gruppe 1-Carboxymethyl-3-(1-carboxy-3-phenyl-(1S)-propylamino)-3,4,5,6-tetrahydro-(3R)-1-benzazocin-2-1H-on, 1-Carboxymethyl-3-(1-ethoxycarbonyl-3-phenyl-(1S)-propylamino)-3,4,5,6-tetrahydro-(3R)-1-benzazocin-2-1H-on, 3-[(5-Benzyloxycarbonylamino-1-ethoxycarbonylpentyl)amino]-1-ethoxycarbonylmethyl-3,4,5,6-tetrahydro-1-benzazocin-2-1H-on und 3-[(5-Benzyloxycarbonylamino-1-(S)-carboxypentyl)amino]-1-carboxymethyl-3,4,5,6-tetrahydro-(3S)-1-benzazocin-2-1H-on und deren Salzen.

10. 1-Carboxymethyl-3-(1-carboxy-3-phenyl-(1S)-propylamino)-3,4,5,6-tetrahydro-(3S)-1-benzazocin-2-1H-on und seine Salze.

11. 1-Carboxymethyl-3-(1-ethoxycarbonyl-3-phenyl-(1S)-propylamino)-3,4,5,6-tetrahydro-(3S)-1-benzazocin-2-1H-on und seine Salze.

12. Pharmazeutische Präparate enthaltend eine Verbindung gemäß einem der Ansprüche 1 bis 8 oder ein pharmazeutisch annehmbares Salz davon zusammen mit einem pharmazeutisch geeigneten Trägermaterial.

13. Pharmazeutische Präparate enthaltend eine Verbindung gemäß einem der Ansprüche 9 bis 11 oder ein pharmazeutisch annehmbares Salz davon zusammen mit einem pharmazeutisch geeigneten Trägermaterial.

14. Die in den Ansprüchen 1 bis 8 genannten Verbindungen zur Anwendung in einem Verfahren zur therapeutischen Behandlung des menschlichen oder tierischen Körpers.

15. Die in den Ansprüchen 9 bis 11 genannten Verbindungen zur Anwendung in einem Verfahren zur therapeutischen Behandlung des menschlichen oder tierischen Körpers.

16. Die in den Ansprüchen 1 bis 8 genannten Verbindungen als Hemmer des Angiotensin-umwandelnden Enzyms.

17. Die in den Ansprüchen 9 bis 11 genannten Verbindungen als Hemmer des Angiotensin-umwandelnden Enzyms.

18. Verwendung der in den Ansprüchen 1 bis 8 genannten Verbindungen zur Herstellung von pharmazeutischen Präparaten.

19. Verwendung der in den Ansprüchen 9 bis 11 genannten Verbindungen zur Herstellung von pharmazeutischen Präparaten.

20. Verfahren zur Herstellung von den in Anspruch 1 genannten Verbindungen der Formel I, in der alle Symbole die in Anspruch 1 angegebenen Bedeutungen haben, und deren Salzen und Stereoisomeren, dadurch gekennzeichnet, dass man

a) in eine Verbindung der Formel

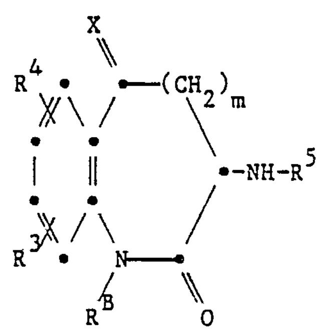

(II)

worin der carbocyclische Ring auch perhydriert sein kann und worin X, $R^B$, $R^3$, $R^4$, $R^5$ und m die vorstehend angegebenen Bedeutungen besitzen, $R^A$ durch Alkylierung mit einer Verbindung der Formel

$$R^A—Z \quad (IIIA),$$

worin Z eine reaktive veresterte Hydroxygruppe bedeutet und $R^A$ die vorstehend angegebenen Bedeutungen besitzt, oder mit einer Verbindung der Formel

$$R^1—CO—R^0 \quad (IV),$$

worin $R^1$ und $R^0$ die oben angegebenen Bedeutungen haben, in Gegenwart eines Reduktionsmittels, unter vorübergehendem Schutz etwaiger primärer und sekundärer Aminogruppen und/oder gegebenenfalls Hydroxy- und/oder Oxogruppen, die in irgendeinem der Reste X, $R^B$, $R^3$ und $R^4$ und/oder im Alkylierungsmittel anwesend sein können, einführt, oder

b) eine Verbindung der Formel

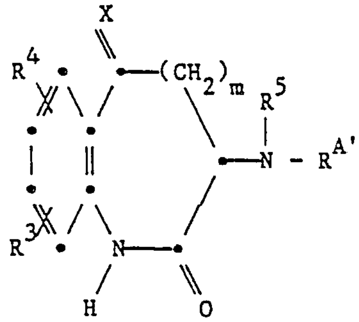

(V)

worin der carbocyclische Ring auch perhydriert sein kann und worin X, $R^3$, $R^4$, $R^5$ und m die vorstehend angegebenen Bedeutungen besitzen und $R^{A'}$ Wasserstoff oder $R^A$ wie vorstehend definiert ist, mit einer Verbindung der Formel

$$R^B—Z \qquad \text{(IIIB)},$$

worin Z eine reaktive veresterte Hydroxygruppe bedeutet und $R^B$ die vorstehend angegebenen Bedeutungen besitzt, alkyliert, wobei man vorübergehend etwaige primäre und sekundäre Aminogruppen und/oder gegebenenfalls Hydroxy- und/oder Oxogruppen, die in irgendeinem der Reste X, $R^{A'}$, $R^B$, $R^3$ und $R^4$ anwesend sein können, schützt, oder

c) eine Verbindung der Formel

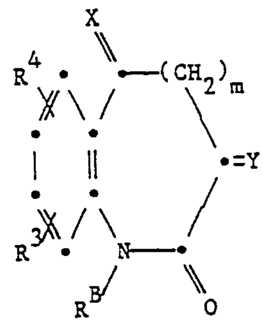

(VI)

worin der carbocyclische Ring auch perhydriert sein kann und worin Y Oxo, Dichloro, oder eine reaktive veresterte Hydroxygruppe Z gemeinsam mit Wasserstoff bedeutet und X, $R^B$, $R^3$, $R^4$ und m die vorstehend angegebenen Bedeutungen besitzen, mit einem Amin der Formel

$$R^A—NH—R^5 \qquad \text{(VII)},$$

worin $R^A$ und $R^5$ die vorstehend angegebenen Bedeutungen besitzen, kondensiert, wobei, wenn Y Oxo oder Dichloro bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel

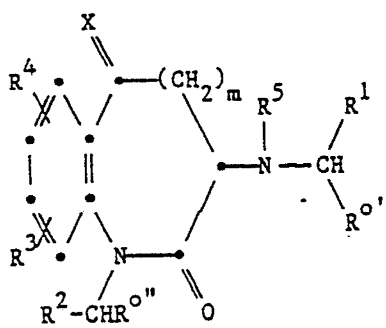

(VIII)

worin der carbocyclische Ring auch perhydriert sein kann und worin X, $R^1$ bis $R^5$ und m die vorstehend angegebenen Bedeutungen besitzen, eines der Symbole $R^{0'}$ und $R^{0''}$ Cyano ist und das andere Cyano oder $R^0$ wie vorstehend definiert ist, die Cyanogruppe(n) einer Solvolyse unterzieht, oder

e) eine Verbindung der Formel

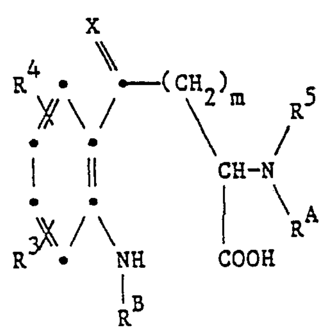

(IX)

worin der carbocyclische Ring auch perhydriert sein kann und worin X, $R^A$, $R^B$, $R^3$, $R^4$, $R^5$ und m die vorstehend angegebenen Bedeutungen besitzen, oder einen Ester hiervon cyclisiert, oder

f) eine Verbindung, die strukturell mit einer Verbindung der vorstehend angegebenen Formel I identisch ist, mit Ausnahme dessen, dass sie eine oder zwei zusätzliche Doppelbindung(en) im heterocyclischen Ring und/oder eine zusätzliche Doppelbindung zwischen dem exocyclischen Stickstoffatom und dem C-3-Kohlenstoffatom oder dem benachbarten Kohlenstoffatom innerhalb der Gruppe $R^A$ aufweist, mit einem Reduktionsmittel behandelt, um diese Doppelbindung(en) zu sättigen, und gewünschtenfalls eine erhaltene Verbindung der Formel I in eine andere Verbindung der Formel I umwandelt und/oder, gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt, und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

21. Die nach dem Verfahren des Anspruchs 20 erhältlichen Verbindungen.

**Patentansprüche** (für den Vertragsstaat AT)

1. Verfahren zur Herstellung von Benzazocinon- und Benzazocinon-Derivaten der allgemeinen Formel

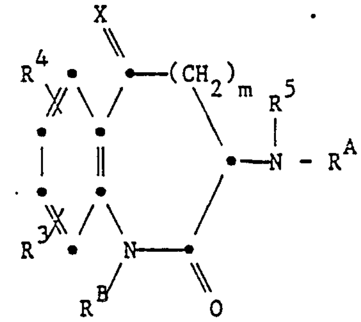

(I)

worin $R^A$ und $R^B$ Reste der Formel

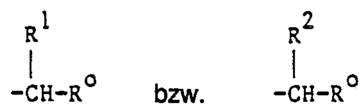

darstellen, worin $R^0$ Carboxy oder ein funktionell modifiziertes Carboxy darstellt; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Aryl-(thio-, sulfinyl- oder sulfonyl-)-niederalkyl, Aryl-N-niederalkylamino-niederalkyl oder Arylamino-niederalkyl bedeutet; $R^2$ für Wasserstoff oder Niederalkyl steht; $R^3$ und $R^4$ unabhängig voneinander Wasserstoff, Niederalkyl, Niederalkoxy, Niederalkanoyloxy, Hydroxy, Halogen oder Trifluormethyl bedeuten, oder $R^3$ und $R^4$ gemeinsam für Niederalkylendioxy stehen; $R^5$ Wasserstoff oder Niederalkyl bedeutet; m für 2 oder 3 steht; und X Oxo, zwei Wasserstoffatome oder Hydroxy oder acyliertes Hydroxy zusammen mit einem Wasserstoffatom bedeutet; und worin der carbocyclische Ring auch perhydriert sein kann; Salzen und Stereoisomeren von allen diesen Verbindungen, dadurch gekennzeichnet, dass man

a) in eine Verbindung der Formel

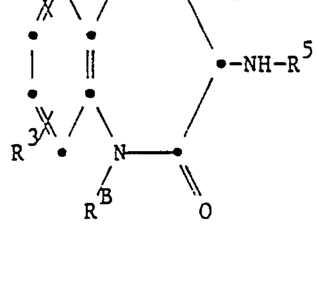

(II)

worin der carbocyclische Ring auch perhydriert sein kann und worin X, $R^B$, $R^3$, $R^4$, $R^5$ und m die vorstehend angegebenen Bedeutungen besitzen, $R^A$ durch Alkylierung mit einer Verbindung der Formel

$R^A—Z$ (IIIA),

worin Z eine reaktive veresterte Hydroxygruppe bedeutet und $R^A$ die vorstehend angegebenen Bedeutungen besitzt, oder mit einer Verbindung der Formel

$R^1—CO—R^0$ (IV),

worin $R^1$ und $R^0$ die oben angegebenen Bedeutungen haben, in Gegenwart eines Reduktionsmittels, unter vorübergehendem Schutz etwaiger primärer und sekundärer Aminogruppen und/oder gegebenenfalls Hydroxy- und/oder Oxogruppen, die in irgendeinem der Reste X, $R^B$, $R^3$ und $R^4$ und/oder im Alkylierungsmittel anwesend sein können, einführt, oder

b) eine Verbindung der Formel

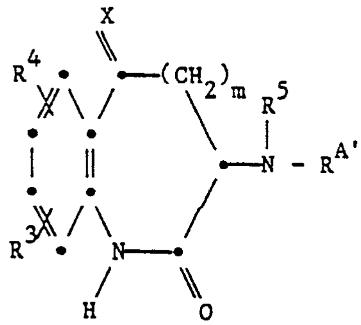

(V)

worin der carbocyclische Ring auch perhydriert sein kann und worin X, $R^3$, $R^4$, $R^5$ und m die vorstehend angegebenen Bedeutungen besitzen und $R^{A'}$ Wasserstoff oder $R^A$ wie vorstehend definiert ist, mit einer Verbindung der Formel

$R^B—Z$ (IIIB),

worin Z eine reaktive veresterte Hydroxygruppe bedeutet und $R^B$ die vorstehend angegebenen Bedeutungen besitzt, alkyliert, wobei man vorübergehend etwaige primäre und sekundäre Aminogruppen und/oder gegebenenfalls Hydroxy- und/oder Oxogruppen, die in irgendeinem der Reste X, $R^{A'}$, $R^B$, $R^3$ und $R^4$ anwesend sein können, schützt, oder

c) eine Verbindung der Formel

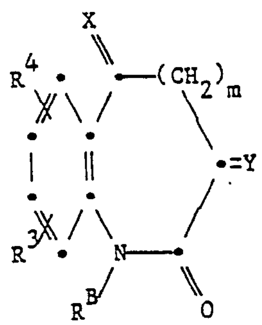

(VI)

worin der carbocyclische Ring auch perhydriert sein kann und worin Y Oxo, Dichloro, oder eine reaktive veresterte Hydroxygruppe Z gemeinsam mit Wasserstoff bedeutet und X, $R^B$, $R^3$, $R^4$ und m die vorstehend angegebenen Bedeutungen besitzen, mit einem Amin der Formel

$R^A—NH—R^5$ (VII),

worin $R^A$ und $R^5$ die vorstehend angegebenen Bedeutungen besitzen, kondensiert, wobei, wenn Y Oxo oder Dichloro bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel

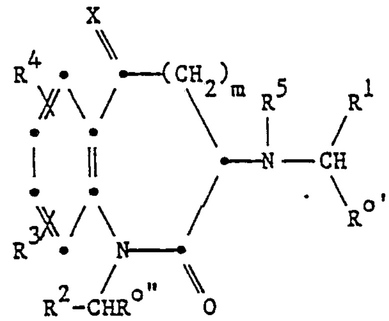

(VIII)

worin der carbocyclische Ring auch perhydriert sein kann und worin X, $R^1$ bis $R^5$ und m die vorstehend angegebenen Bedeutungen besitzen, eines der Symbole $R^{0'}$ und $R^{0''}$ Cyano ist und das andere Cyano oder $R^0$ wie vorstehend definiert ist, die Cyanogruppe(n) einer Solvolyse unterzieht, oder

e) eine Verbindung der Formel

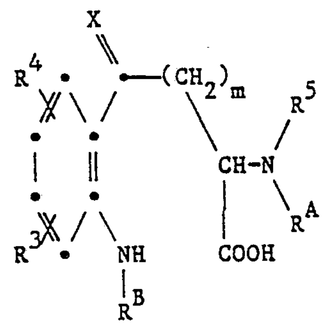

(IX)

worin der carbocyclische Ring auch perhydriert sein kann und worin X, $R^A$, $R^B$, $R^3$, $R^4$, $R^5$ und m die vorstehend angegebenen Bedeutungen besitzen, oder einen Ester hiervon cyclisiert, oder

f) eine Verbindung, die strukturell mit einer Verbindung der vorstehend angegebenen Formel I identisch ist, mit Ausnahme dessen, dass sie eine oder zwei zusätzliche Doppelbindung(en) im heterocyclischen Ring und/oder eine zusätzliche Doppelbindung zwischen dem exocyclischen Stickstoffatom und dem C-3-Kohlenstoffatom oder dem benachbarten Kohlenstoffatom innerhalb der Gruppe $R^A$ aufweist, mit einem Reduktionsmittel behandelt, um diese Doppelbindung(en) zu sättigen, und gewünschtenfalls eine erhaltene Verbindung der Formel I in eine andere Verbindung der Formel I umwandelt und/oder, gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt, und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

2. Verfahren nach Anspruch 1, dadurch gekennzeichnet, dass man Verbindungen der Formel

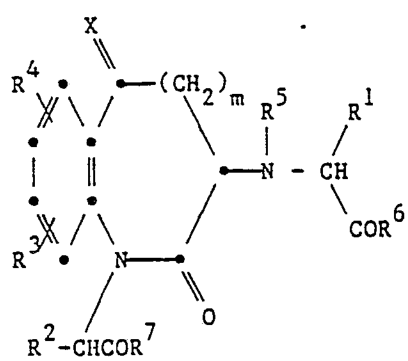

(IA)

worin der carbocyclische Ring auch perhydriert sein kann; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Arylthio-niederalkyl, Aryl-N-niederalkylamino-niederalkyl oder Arylamino-niederalkyl bedeutet ; $R^2$ und $R^5$ Wasserstoff oder Niederalkyl bedeuten ; $R^3$ und $R^4$ Wasserstoff, Niederalkyl, Niederalkoxy, Niederalkanoyloxy, Hydroxy, Halogen oder Trifluormethyl bedeuten oder $R^3$ und $R^4$ gemeinsam Niederalkylendioxy darstellen ; X Oxo, zwei Wasserstoffatome, oder eine Hydroxygruppe oder acylierte Hydroxygruppe und ein Wasserstoff bedeutet ; m für 2 oder 3 steht ; $R^6$ und $R^7$ unabhängig voneinander Hydroxy, Amino, Mono- oder Diniederalkylamino, Niederalkoxy, Aryl-niederalkoxy, Niederalkanoyloxymethoxy, (Amino, Mono- oder Diniederalkylamino, Carboxy oder Niederalkoxycarbonyl)-niederalkoxy bedeuten ; oder deren Salze herstellt.

3. Verfahren nach Anspruch 1, dadurch gekennzeichnet, dass man Verbindungen der Formel IA, worin $R^1$ Wasserstoff, Niederalkyl, Amino-niederalkyl, Acylamino-niederalkyl, Aryl-niederalkyl, worin Aryl unsubstituiertes Phenyl oder durch Niederalkyl, Hydroxy, Niederalkoxy, Niederalkylendioxy, Niederalkanoyloxy, Halogen oder Trifluormethyl mono- oder disubstituiertes Phenyl ist, bedeutet ; $R^3$ und $R^4$ Wasserstoff, Niederalkyl, Niederalkoxy, Halogen oder Trifluormethyl bedeuten oder $R^3$ und $R^4$ gemeinsam für Alkylendioxy stehen ; $R^6$ und $R^7$ unabhängig voneinander Hydroxy, Amino, Niederalkoxy, Phenylniederalkoxy, Niederalkoxycarbonyl-niederalkoxy bedeuten ; $R^2$, $R^5$, X und m die obigen Bedeutungen haben, oder ihre Salze ; oder die genannten Verbindungen, worin der carbocyclische Ring perhydriert ist, herstellt.

4. Verfahren nach Anspruch 1, dadurch gekennzeichnet, dass man Verbindungen der Formel IA, worin $R^1$ Wasserstoff, Niederalkyl, ω-Amino-niederalkyl, ω-Arylmethoxycarbonylamino-niederalkyl oder Arylniederalkyl bedeutet, worin Aryl für unsubstituiertes Phenyl oder durch Niederalkyl, Hydroxy, Niederalkoxy, Niederalkanoyloxy, Halogen oder trifluormethyl monosubstituiertes Phenyl steht ; $R^2$ und $R^5$ Wasserstoff oder Niederalkyl bedeuten ; $R^3$ für Wasserstoff steht ; $R^4$ Wasserstoff, Niederalkyl, Niederalkoxy, Halogen oder Trifluormethyl bedeutet ; X für Oxo, zwei Wasserstoffatome, oder eine Hydroxy- oder Niederalkanoyloxygruppe und ein Wasserstoffatom steht ; m 2 oder 3 bedeutet ; $R^6$ und $R^7$ unabhängig voneinander für Hydroxy, Amino, Niederalkoxy, Phenyl-niederalkoxy, Niederalkoxycarbonylniederalkoxy stehen ; oder ihre Salze ; oder die genannten Verbindungen, worin der carbocyclische Ring perhydriert ist, herstellt.

5. Verfahren nach Anspruch 1, dadurch gekennzeichnet, dass man Verbindungen der Formel

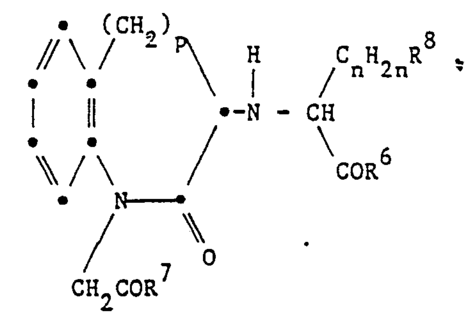

(IB)

worin n eine ganze Zahl von 1 is 4 bedeutet, p für die ganze Zahl 3 oder 4 steht, $R^8$ Wasserstoff, Amino, Benzyloxycarbonylamino, unsubstituiertes Phenyl oder Phenyl, monosubstituiert durch Niederalkyl, Niederalkoxy, Niederalkanoyloxy, Halogen, Hydroxy oder Trifluormethyl, bedeutet ; $R^6$ und $R^7$ unabhängig voneinander Hydroxy, Niederalkoxy mit bis zu 4 Kohlenstoffatomen, Benzyloxy oder Amino bedeuten, oder ihre Salze ; oder die genannten Verbindungen, worin der carbocyclische Ring perhydriert ist, herstellt.

6. Verfahren nach Anspruch 1, dadurch gekennzeichnet, dass man Verbindungen der Formel

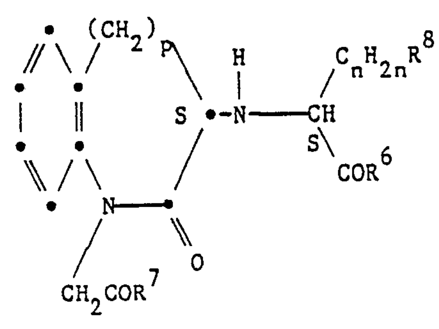

(IC)

worin S die Chiralität bedeutet, n eine ganze Zahl von 1 bis 4 bedeutet ; p für die ganze Zahl 3 oder 4 steht, $R^8$ Wasserstoff, Amino, Benzyloxycarbonylamino, unsubstituiertes Phenyl oder durch Niederalkyl, Niederalkoxy, Niederalkanoyloxy, Halogen, Hydroxy oder Trifluormethyl monosubstituiertes Phenyl

bedeutet, $R^6$ und $R^7$ unabhängig voneinander Hydroxy, Niederalkoxy mit bis zu 4 Kohlenstoffatomen, Benzyloxy oder Amino bedeuten ; oder ihre Salze herstellt.

7. Verfahren nach Anspruch 1, dadurch gekennzeichnet, dass man 1-Carboxymethyl-3-(1-carboxy-3-phenyl-(1S)-propylamino)-3,4,5,6-tetrahydro-(3S)-1-benzazocin-2-1H-on oder ein Salz davon herstellt.

8. Verfahren nach Anspruch 1, dadurch gekennzeichnet, dass man 1-Carboxymethyl-3-(1-ethoxycarbonyl-3-phenyl-(1S)-propylamino)-3,4,5,6-tetrahydro-(3S)-1-benzazocin-2-1H-on oder ein Salz davon herstellt.

9. Verfahren zur Herstellung von den in Anspruch 1 genannten Verbindungen der Formel I, in der alle Symbole die in Anspruch 1 angegebenen Bedeutungen haben, und deren Stereoisomeren und Salzen, dadurch gekennzeichnet, dass man die Verfahrensvarianten a) bis e) nach Anspruch 1 ausführt, oder f) eine Verbindung, die mit einer Verbindung der Formel I identisch ist, ausser dass sie eine zusätzliche Doppelbindung am Kohlenstoffatom C-3 oder zwischen dem Stickstoffatom und dem benachbarten Kohlenstoffatom in der Gruppe $R^A$ aufweist, mit einem Reduktionsmittel behandelt, um diese Doppelbindung zu sättigen, und gewünschtenfalls eine erhaltene Verbindung der Formel I in eine andere Verbindung der Formel I umwandelt und/oder, gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt, und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

10. Verfahren nach Anspruch 9, dadurch gekennzeichnet, daß man 1-Carboxymethyl-3-(1-ethoxycarbonyl-3-phenyl-propylamino)-3,4,5,6-tetrahydro-1-benzazocin-2-1H-on oder ein Salz davon herstellt.

11. Verfahren nach Anspruch 9, dadurch gekennzeichnet, dass man Verbindungen der in den Ansprüchen 2 bis 6 gezeigten Formeln IA, IB oder IC herstellt.

12. Verfahren zur Herstellung eines pharmazeutischen Präparates, gekennzeichnet durch die Verarbeitung eines erfindungsgemäss hergestellten Wirkstoffes nach einem der Ansprüche 1 bis 8 mit einem pharmazeutischen Trägermaterial.

13. Verfahren zur Herstellung eines pharmazeutischen Präparates, gekennzeichnet durch die Verarbeitung eines erfindungsgemäss hergestellten Wirkstoffes nach einem der Ansprüche 9 bis 11 mit einem pharmazeutischen Trägermaterial.

**Claims** (for the Contracting States : BE, CH, DE, FR, GB, IT, LI, LU, NL, SE)

1. Compounds of the general formula I

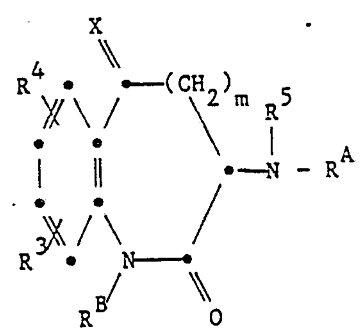

(I)

wherein $R^A$ and $R^B$ are radicals of the formula

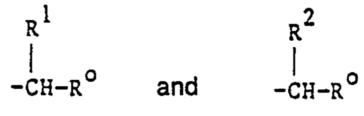

respectively, in which $R^0$ is carboxy or a functionally modified carboxy ; $R^1$ is hydrogen, lower alkyl, amino-lower alkyl, aryl, aryl-lower alkyl, cycloalkyl, cycloalkyl-lower alkyl, acylamino-lower alkyl, mono- or di-lower alkylamino-lower alkyl, lower alkylthio-lower alkyl, carboxy-lower alkyl, esterified carboxy-lower alkyl, carbamoyl-lower alkyl, N-substituted carbamoyl-lower alkyl, hydroxy-lower alkyl, etherified or acylated hydroxy-lower alkyl, aryloxy-lower alkyl, aryl-(thio-, sulfinyl-, or sulfonyl-)lower alkyl, aryl-N-lower alkylamino-lower alkyl or arylamino-lower alkyl ; $R^2$ is hydrogen or lower alkyl ; $R^3$ and $R^4$, each independently, represent hydrogen, lower alkyl, lower alkoxy, lower alkanoyloxy, hydroxy, halogen or trifluoromethyl, or $R^3$ and $R^4$ taken together represent lower alkylenedioxy ; $R^5$ is hydrogen or lower alkyl ; m is 2 or 3 ; and X represents oxo, two hydrogen atoms or hydroxy or acylated hydroxy together

with one hydrogen atom; and wherein the carbocyclic ring may also be perhydrogenated; salts and stereoisomers of all these compounds.

2. Compounds of the formula IA

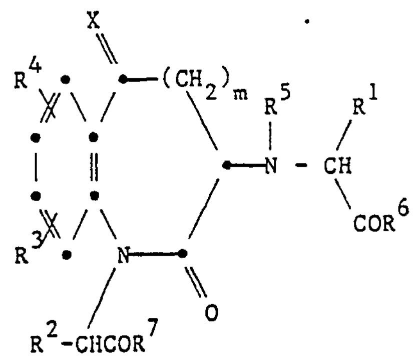

(IA)

wherein the carbocyclic ring may also be perhydrogenated; $R^1$ is hydrogen, lower alkyl, amino-lower alkyl, aryl, aryl-lower alkyl, cycloalkyl, cycloalkyl-lower alkyl, acylamino-lower alkyl, mono- or di-lower alkylamino-lower alkyl, lower alkylthio-lower alkyl, carboxy-lower alkyl, esterified carboxy-lower alkyl, carbamoyl-lower alkyl, N-substituted carbamoyl-lower alkyl, hydroxy-lower alkyl, etherified or acylated hydroxy-lower alkyl, aryloxy-lower alkyl, arylthio-lower alkyl, aryl-N-lower alkylamino-lower alkyl or arylamino-lower alkyl; $R^2$ and $R^5$ represent hydrogen or lower alkyl; $R^3$ and $R^4$ represent hydrogen, lower alkyl, lower alkoxy, lower alkanoyloxy, hydroxy, halogen or trifluoromethyl; or $R^3$ and $R^4$ taken together represent lower alkylenedioxy; X represents oxo, two hydrogen atoms, or one hydroxy or acylated hydroxy group and one hydrogen atom; m is 2 or 3; $R^6$ and $R^7$ independently represent hydroxy, amino, mono- or di-lower alkylamino, lower alkoxy, aryl-lower alkoxy, lower alkanoyloxymethoxy, (amino, mono- or di-lower alkylamino, carboxy or lower alkoxycarbonyl)-lower alkoxy; or salts thereof.

3. Compounds of the formula IA shown in claim 2, wherein $R^1$ is hydrogen, lower alkyl, amino-lower alkyl, acylamino-lower alkyl, aryl-lower alkyl where aryl is phenyl unsubstituted or mono- or di-substituted by lower alkyl, hydroxy, lower alkoxy, lower alkylenedioxy, lower alkanoyloxy, halogen or by trifluoromethyl; $R^3$ and $R^4$ are hydrogen, lower alkyl, lower alkoxy, halogen or trifluoromethyl; or $R^3$ and $R^4$ taken together represent alkylenedioxy; $R^6$ and $R^7$ independently represent hydroxy, amino, lower alkoxy, phenyl-lower alkoxy, lower alkoxycarbonyl-lower alkoxy, $R^2$, $R^5$, X and m as defined above; or salts thereof, or said compounds wherein the carbocyclic ring is perhydrogenated.

4. Compounds of the formula IA shown in claim 2, wherein $R^1$ is hydrogen, lower alkyl, ω-amino-lower alkyl, ω-arylmethoxycarbonylamino-lower alkyl, or aryl-lower alkyl where aryl is phenyl unsubstituted or mono-substituted by lower alkyl, hydroxy, lower alkoxy, lower alkanoyloxy, halogen or by trifluoromethyl; $R^1$ and $R^5$ are hydrogen or lower alkyl; $R^3$ is hydrogen; $R^4$ is hydrogen, lower alkyl, lower alkoxy, halogen or trifluoromethyl; X represents oxo, two hydrogen atoms, or one hydroxy or lower alkanoyloxy group and one hydrogen atom; m is 2 or 3; $R^6$ and $R^7$ independently represent hydroxy, amino, lower alkoxy, phenyl-lower alkoxy, lower alkoxycarbonyl-lower alkoxy; or salts thereof; or said compounds wherein the carbocyclic ring is perhydrogenated.

5. Compounds of the formula IB

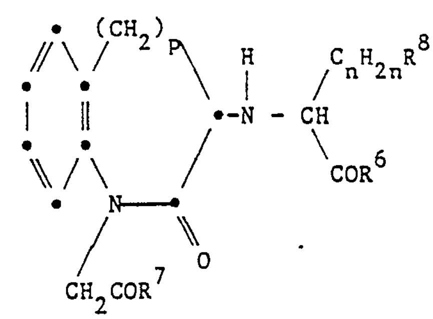

(IB)

wherein n represents an integer from 1 to 4; p is the integer 3 or 4; $R^8$ is hydrogen, amino, benzyloxycarbonylamino, phenyl unsubstituted or monosubstituted by lower alkyl, lower alkoxy, lower alkanoyloxy, halogen, hydroxy or by trifluoromethyl; $R^6$ and $R^7$ independently represent hydroxy, lower alkoxy of up to 4 carbon atoms, benzyloxy or amino; or salts thereof; or said compounds wherein the carbocyclic ring is perhydrogenated.

6. Compounds of the formula IC

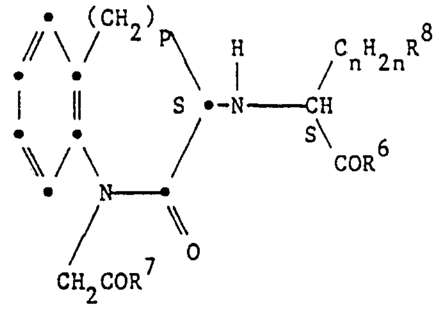

(IC)

wherein S represents the chirality, n represents an integer from 1 to 4, p is the integer 3 or 4; $R^8$ is hydrogen, amino, benzyloxycarbonylamino, phenyl unsubstituted or monosubstituted by lower alkyl, lower alkoxy, lower alkanoyloxy, halogen, hydroxy or by trifluoromethyl; $R^6$ and $R^7$ independently represent hydroxy, lower alkoxy of up to 4 carbon atoms, benzyloxy or amino; or salts thereof.

7. 1-Carboxymethyl-3-(1-ethoxycarbonyl-3-phenylpropylamino)-3,4,5,6-tetrahydro-1-benzazocin-2-1H-one and salts thereof.

8. A compound selected from the group consisting of 1-carboxymethyl-3-(1-carboxy-3-phenylpropylamino)-3,4,5,6-tetrahydro-1-benzazocin-2-1H-one and 1-carboxymethyl-3-(1-ethoxycarbonyl-3-phenylpropylamino)-1,3,4,5,6,7-hexahydro-1-benzazonin-2-one and salts thereof.

9. A compound selected from the group consisting of 1-carboxymethyl-3-(1-carboxy-3-phenyl-(1S)-propylamino)-3,4,5,6-tetrahydro-(3R)-1-benzazocin-2-1H-one, 1-carboxymethyl-3-(1-ethoxycarbonyl-3-phenyl-(1S)-propylamino)-3,4,5,6-tetrahydro-(3R)-1-benzazocin-2-1H-one, 3-[(5-benzyloxycarbonylamino-1-ethoxycarbonylpentyl) amino]-1-ethoxycarbonylmethyl-3,4,5,6-tetrahydro-1-benzazocin-2-1H-one and 3-[(5-benzyloxycarbonylamino-1-(S)-carboxypentyl)-amino]-1-carboxymethyl-3,4,5,6-tetrahydro-(3S)-1-benzazocin-2-1H-one and salts thereof.

10. 1-Carboxymethyl-3-(1-carboxy-3-phenyl-(1S)-propylamino)-3,4,5,6-tetrahydro-(3S)-1-benzazocin-2-1H-one and salts thereof.

11. 1-Carboxymethyl-3-(1-ethoxycarbonyl-3-phenyl-(1S)-propylamino)-3,4,5,6-tetrahydro-(3S)-1-benzazocin-2-1H-one and salts thereof.

12. Pharmaceutical preparations comprising a compound according to any one of claims 1 to 8 or a pharmaceutically acceptable salt thereof, in admixture or conjunction with a pharmaceutically suitable carrier.

13. Pharmaceutical preparations comprising a compound according to any one of claims 9 to 11 or a pharmaceutically acceptable salt thereof, in admixture or conjuction with a pharmaceutically suitable carrier.

14. The compounds mentioned in claims 1 to 8, for use in a method for the therapeutic treatment of the human or animal body.

15. The compounds mentioned in claims 9 to 11, for use in a method for the therapeutic treatment of the human or animal body.

16. The compounds mentioned in claims 1 to 8, as inhibitors of the angiotensin-converting enzyme.

17. The compounds mentioned in claims 9 to 11, as inhibitors of the angiotensin-converting enzyme.

18. The use of the compounds mentioned in claims 1 to 8 for the manufacture of pharmaceutical preparations.

19. The use of the compounds mentioned in claims 9 to 11 for the manufacture of pharmaceutical preparations.

20. A process for the manufacture of the compounds of formula I mentioned in claim 1, in which formula all the symbols have the meanings given in claim 1, salts and stereoisomers thereof, characterised in that

a) in a compound of the formula

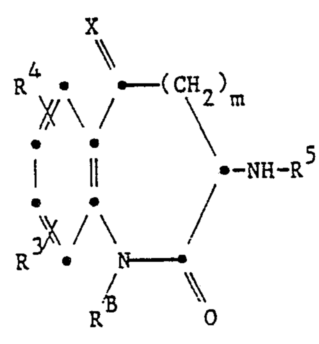

(II)

wherein the carbocyclic ring may also be perhydrogenated and wherein X, $R^B$, $R^3$, $R^4$, $R^5$ and m have the meanings given hereinabove, $R^{A'}$ is introduced by alkylation with a compound of the formula

$$R^A—Z \quad \text{(IIIA)},$$

wherein Z is a reactive esterified hydroxy group and $R^A$ has the meanings given hereinabove, or with a compound of the formula

$$R^1—CO—R^0 \quad \text{(IV)},$$

wherein $R^1$ and $R^0$ have the meanings given hereinabove, in the presence of a reducing agent with temporary protection of any primary and secondary amino groups and/or, optionally, hydroxy and/or oxo groups which may be present in any one of the radicals X, $R^B$, $R^3$ and $R^4$ and/or in the alkylating agent, or

b) a compound of the formula

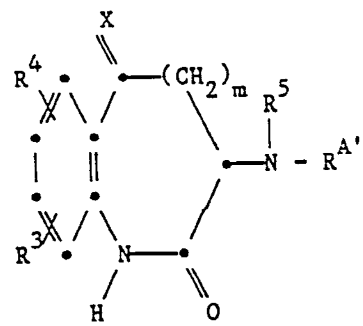

(V)

wherein the carbocyclic ring may also be perhydrogenated and wherein X, $R^3$, $R^4$, $R^5$ and m have the meanings given hereinabove and $R^{A'}$ is hydrogen or $R^A$ as defined hereinabove, is alkylated with a compound of the formula

$$R^B—Z \quad \text{(IIIB)},$$

wherein Z is a reactive esterified hydroxy group and $R^B$ has the meanings given hereinabove, while temporarily protecting any primary and secondary amino groups and/or, optionally, hydroxy and/or oxo groups which may be present in any one of the radicals X, $R^{A'}$, $R^B$, $R^3$ and $R^4$, or

c) a compound of the formula

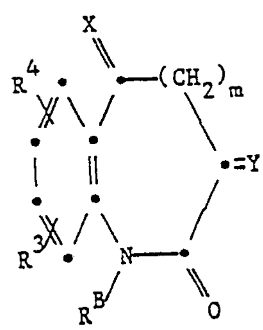

(VI)

wherein the carbocyclic ring may also be perhydrogenated and wherein Y is oxo or dichloro or a reactive esterified hydroxy group Z together with hydrogen, and X, $R^B$, $R^3$, $R^4$ and m have the meanings given hereinabove, is condensed with an amine of the formula

$$R^A—NH—R^5 \quad \text{(VII)},$$

wherein $R^A$ and $R^5$ have the meanings given hereinabove, with the proviso that when Y is oxo or dichloro, the condensation is carried out in the presence of a reducing agent and with temporary protection of the oxo group which may be present as the substituent X, or

d) in a compound of the formula

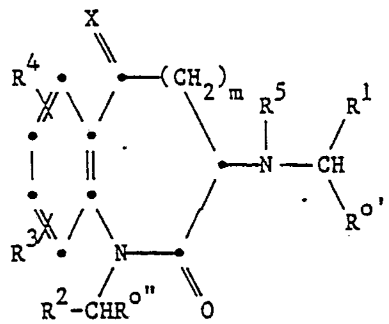

(VIII)

wherein the carbocyclic ring may also be perhydrogenated and wherein X, $R^1$ to $R^5$ and m have the meanings given hereinabove, one of the symbols $R^{0'}$ and $R^{0''}$ is cyano and the other is cyano or $R^0$ as defined hereinabove, the cyano group(s) is (are) subjected to solvolysis, or

e) a compound of the formula

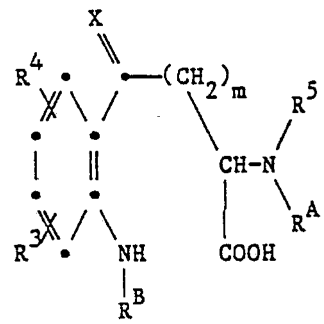

(IX)

wherein the carbocyclic ring may also be perhydrogenated and wherein X, $R^A$, $R^B$, $R^3$, $R^4$, $R^5$ and m have the meanings given hereinabove, or an ester thereof, is cyclised, or

f) a compound that is structurally identical with a compound of the formula I specified above, except for having one or two additional double bond(s) in the heterocyclic ring and/or one additional double bond between the exocyclic nitrogen atom and the C-3-carbon atom or the adjacent carbon atom within the group $R^A$, is treated with a reducing agent in order to saturate this (these) double bond(s), and, if desired, a resulting compound of formula I is converted into another compound of formula I, and/or, if desired, a resulting compound of formula I having salt-forming properties is converted into a salt thereof or a resulting salt into another salt or a free compound is liberated from such a salt, and/or, if desired, an optical isomer that has a specific configuration with respect to at least one centre of chirality is enriched from a mixture of stereoisomeric forms of a resulting compound of formula I.

21. The compounds obtainable according to the process of claim 20.

**Claims** (for the Contracting State AT)

1. A process for the manufacture of benzazocinone and benzazoninone derivatives of the general formula

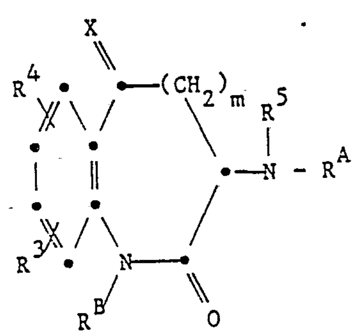

(I)

wherein $R^A$ and $R^B$ are radicals of the formula

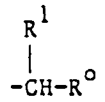 and 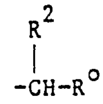

respectively, in which $R^0$ is carboxy or a functionally modified carboxy ; $R^1$ is hydrogen, lower alkyl, amino-lower alkyl, aryl, aryl-lower alkyl, cycloalkyl, cycloalkyl-lower alkyl, acylamino-lower alkyl, mono- or di-lower alkylamino-lower alkyl, lower alkylthio-lower alkyl, carboxy-lower alkyl, esterified carboxy-lower alkyl, carbamoyl-lower alkyl, N-substituted carbamoyl-lower alkyl, hydroxy-lower alkyl, etherified or acylated hydroxy-lower alkyl, aryloxy-lower alkyl, aryl-(thio-, sulfinyl-, or sulfonyl-) lower alkyl, aryl-N-lower alkylamino-lower alkyl or arylamino-lower alkyl ; $R^2$ is hydrogen or lower alkyl ; $R^3$ and $R^4$, each independently, represent hydrogen, lower alkyl, lower alkoxy, lower alkanoyloxy, hydroxy, halogen or trifluoromethyl, or $R^3$ and $R^4$ taken together represent lower alkylenedioxy ; $R^5$ is hydrogen or lower alkyl ; m is 2 or 3 ; and X represents oxo, two hydrogen atoms or hydroxy or acylated hydroxy together with one hydrogen atom ; and wherein the carbocyclic ring may also be perhydrogenated ; salts and stereoisomers of all these compounds, characterised in that

a) in a compound of the formula

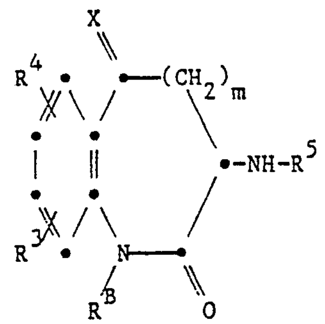

(II)

wherein the carbocyclic ring may also be perhydrogenated and wherein X, $R^B$, $R^3$, $R^4$, $R^5$ and m have the meanings given hereinabove, $R^A$ is introduced by alkylation with a compound of the formula

$$R^A—Z \qquad \text{(IIIA)},$$

wherein Z is a reactive esterified hydroxy group and $R^A$ has the meanings given hereinabove, or with a compound of the formula

$$R^1—CO—R^0 \qquad \text{(IV)},$$

wherein $R^1$ and $R^0$ have the meanings given hereinabove, in the presence of a reducing agent with temporary protection of any primary and secondary amino groups and/or, optionally, hydroxy and/or oxo groups which may be present in any one of the radicals X, $R^B$, $R^3$ and $R^4$ and/or in the alkylating agent, or

b) a compound of the formula

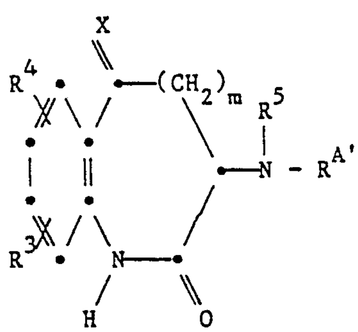

(V)

wherein the carbocyclic ring may also be perhydrogenated and wherein X, $R^3$, $R^4$, $R^5$ and m have the meanings given hereinabove and $R^{A'}$ is hydrogen or $R^A$ as defined hereinabove, is alkylated with a compound of the formula

$$R^B—Z \qquad \text{(IIIB)},$$

wherein Z is a reactive esterified hydroxy group and $R^B$ has the meanings given hereinabove, while temporarily protecting any primary and secondary amino groups and/or, optionally, hydroxy and/or oxo groups which may be present in any one of the radicals X, $R^{A'}$, $R^B$, $R^3$ and $R^4$, or

c) a compound of the formula

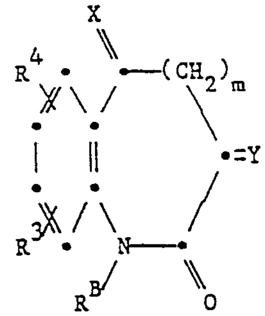

(VI)

wherein the carbocyclic ring may also be perhydrogenated and wherein Y is oxo or dichloro or a reactive esterified hydroxy group Z together with hydrogen, and X, $R^B$, $R^3$, $R^4$ and m have the meanings given hereinabove, is condensed with an amine of the formula

$$R^A—NH—R^5 \quad (VII),$$

wherein $R^A$ and $R^5$ have the meanings given hereinabove, with the proviso that when Y is oxo or dichloro, the condensation is carried out in the presence of a reducing agent and with temporary protection of the oxo group which may be present as the substituent X, or

d) in a compound of the formula

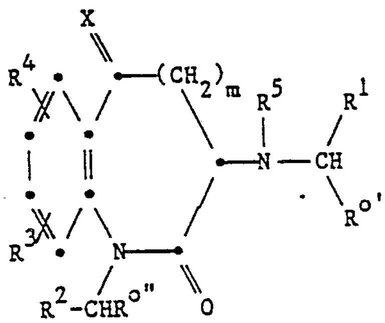

(VIII)

wherein the carbocyclic ring may also be perhydrogenated and wherein X, $R^1$ to $R^5$ and m have the meanings given hereinabove, one of the symbols $R^{0'}$ and $R^{0''}$ is cyano and the other is cyano or $R^0$ as defined hereinabove, the cyano group(s) is (are) subjected to solvolysis, or

e) a compound of the formula

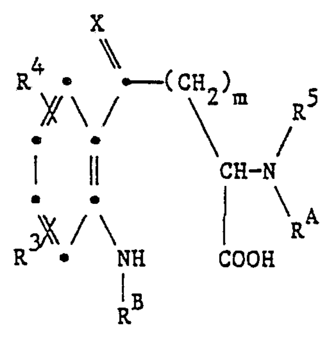

(IX)

wherein the carbocyclic ring may also be perhydrogenated and wherein X, $R^A$, $R^B$, $R^3$, $R^4$, $R^5$ and m have the meanings given hereinabove, or an ester thereof, is cyclised, or

f) a compound that is structurally identical with a compound of the formula I specified above, except for having one or two additional double bond(s) in the heterocyclic ring and/or one additional double bond between the exocyclic nitrogen atom and the C-3-carbon atom or the adjacent carbon atom within the group $R^A$, is treated with a reducing agent in order to saturate this (these) double bond(s), and, if desired, a resulting compound of formula I is converted into another compound of formula I, and/or, if desired, a resulting compound of formula I having salt-forming properties is converted into a salt thereof or a resulting salt into another salt or a free compound is liberated from such a salt, and/or, if desired, an optical isomer that has a specific configuration with respect to at least one centre of chirality is enriched from a mixture of stereoisomeric forms of a resulting compound of formula I.

2. A process according to claim 1, characterised in that compounds of the formula

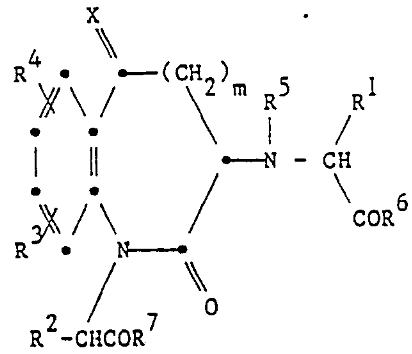

(IA)

wherein the carbocyclic ring may also be perhydrogenated; $R^1$ is hydrogen, lower alkyl, amino-lower alkyl, aryl, aryl-lower alkyl, cycloalkyl, cycloalkyl-lower alkyl, acylamino-lower alkyl, mono- or di-lower alkylamino-lower alkyl, lower alkylthio-lower alkyl, carboxy-lower alkyl, esterified carboxy-lower alkyl, carbamoyl-lower alkyl, N-substituted carbamoyl-lower alkyl, hydroxy-lower alkyl, etherified or acylated hydroxy-lower alkyl, aryloxy-lower alkyl, arylthio-lower alkyl, aryl-N-lower alkylamino-lower alkyl or arylamino-lower alkyl; $R^2$ and $R^5$ represent hydrogen or lower alkyl; $R^3$ and $R^4$ represent hydrogen, lower alkyl, lower alkoxy, lower alkanoyloxy, hydroxy, halogen or trifluoromethyl; or $R^3$ and $R^4$ taken together represent lower alkylenedioxy; X represents oxo, two hydrogen atoms, or one hydroxy or acylated hydroxy group and one hydrogen atom; m is 2 or 3; $R^6$ and $R^7$ independently represent hydroxy, amino, mono- or di-lower alkylamino, lower alkoxy, aryl-lower alkoxy, lower alkanoyloxymethoxy, (amino, mono- or di-lower alkylamino, carboxy or lower alkoxycarbonyl)-lower alkoxy; or salts thereof are manufactured.

3. A process according to claim 1, characterised in that compounds of the formula IA wherein $R^1$ is hydrogen, lower alkyl, amino-lower alkyl, acylamino-lower alkyl, aryl-lower alkyl where aryl is phenyl unsubstituted or mono- or disubstituted by lower alkyl, hydroxy, lower alkoxy, lower alkylenedioxy, lower alkanoyloxy, halogen or by trifluoromethyl; $R^3$ and $R^4$ are hydrogen, lower alkyl, lower alkoxy, halogen or trifluoromethyl; or $R^3$ and $R^4$ taken together represent alkylenedioxy; $R^6$ and $R^7$ independently represent hydroxy, amino, lower alkoxy, phenyl-lower alkoxy, lower alkoxycarbonyl-lower alkoxy, $R^2$, $R^5$, X and m are as defined above; or salts thereof, or said compounds wherein the carbocyclic ring is perhydrogenated are manufactured.

4. A process according to claim 1, characterised in that compounds of the formula IA wherein $R^1$ is hydrogen, lower alkyl, α-amino-lower alkyl, ω-arylmethoxycarbonylamino-lower alkyl, or aryl-lower alkyl where aryl is phenyl unsubstituted or mono-substituted by lower alkyl, hydroxy, lower alkoxy, lower alkanoyloxy, halogen or by trifluoromethyl; $R^2$ and $R^5$ are hydrogen or lower alkyl; $R^3$ is hydrogen; $R^4$ is hydrogen, lower alkyl, lower alkoxy, halogen or trifluoromethyl; X represents oxo, two hydrogen atoms, or one hydroxy or lower alkanoyloxy group and one hydrogen atom; m is 2 or 3; $R^6$ and $R^7$ independently represent hydroxy, amino, lower alkoxy, phenyl-lower alkoxy, lower alkoxycarbonyl-lower alkoxy; or salts thereof; or said compounds wherein the carbocyclic ring is perhydrogenated are manufactured.

5. A process according to claim 1, characterised in that compounds of the formula

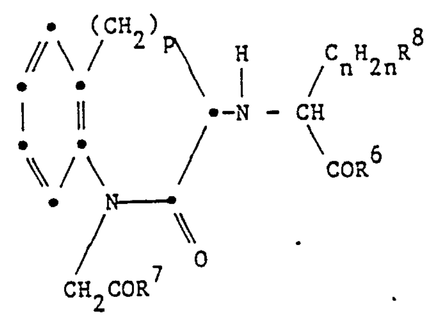

(IB)

wherein n represents an integer from 1 to 4; p is the integer 3 or 4; $R^8$ is hydrogen, amino, benzyloxycarbonylamino, phenyl unsubstituted or monosubstituted by lower alkyl, lower alkoxy, lower alkanoyloxy, halogen, hydroxy or by trifluoromethyl; $R^6$ and $R^7$ independently represent hydroxy, lower alkoxy of up to 4 carbon atoms, benzyloxy or amino; or salts thereof; or said compounds wherein the carbocyclic ring is perhydrogenated are manufactured.

6. A process according to claim 1, characterised in that compounds of the formula

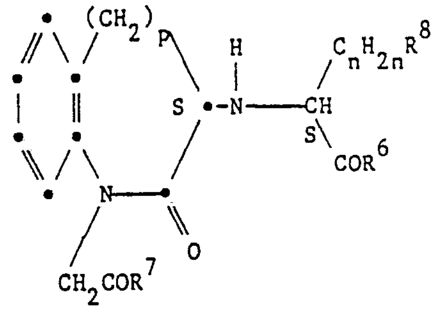

(IC)

wherein S represents the chirality, n represents an integer from 1 to 4; p is the integer 3 or 4; $R^8$ is hydrogen, amino, benzyloxycarbonylamino, phenyl unsubstituted or monosubstituted by lower alkyl, lower alkoxy, lower alkanoyloxy, halogen, hydroxy or by trifluoromethyl; $R^6$ and $R^7$ independently represent hydroxy, lower alkoxy of up to 4 carbon atoms, benzyloxy or amino; or salts thereof are manufactured.

7. A process according to claim 1, characterised in that 1-carboxymethyl-3-(1-carboxy-3-phenyl-(1S)-propylamino)-3,4,5,6-tetrahydro-(3S)-1-benzazocin-2-1H-one or a salt thereof is manufactured.

8. A process according to claim 1, characterised in that 1-carboxymethyl-3-(1-ethoxycarbonyl-3-phenyl-(1S)-propylamino)-3,4,5,6-tetrahydro-(3S)-1-benzazocin-2-1H-one or a salt thereof is manufactured.

9. A process for the manufacture of the compounds of formula I mentioned in claim 1, in which formula all the symbols have the meanings given in claim 1, and stereoisomers and salts thereof, characterised in that process variants a) to e) according to claim 1 are carried out or f) a compound that is identical with a compound of the formula I, except for having one additional double bond at the C-3-carbon atom or between the nitrogen atom and the adjacent carbon atom within the group $R^A$, is treated with a reducing agent in order to saturate this double bond, and, if desired, a resulting compound of formula I is converted into another compound of formula I, and/or, if desired, a resulting compound of formula I having salt-forming properties is converted into a salt thereof or a resulting salt into another salt or a free compound is liberated from such a salt, and/or, if desired, an optical isomer that has a specific configuration with respect to at least one centre of chirality is enriched from a mixture of stereoisomeric forms of a resulting compound of formula I.

10. A process according to claim 9, characterised in that 1-carboxymethyl-3-(1-ethoxycarbonyl-3-phenyl-propylamino)-3,4,5,6-tetrahydro-1-benzazocin-2-1H-one or a salt thereof is manufactured.

11. A process according to claim 9, characterised in that compounds of the formulae IA, IB or IC shown in claims 2 to 6 are manufactured.

12. A process for the manufacture of a pharmaceutical preparation, characterised by processing an active ingredient according to any one of claims 1 to 8, manufactured according to the invention, with a pharmaceutical carrier.

13. A process for the manufacture of a pharmaceutical preparation, characterised by processing an active ingredient according to any one of claims 9 to 11, manufactured according to the invention, with a pharmaceutical carrier.

**Revendications** (pour les Etats Contractants : BE, CH, DE, FR, GB, IT, LI, LU, NL, SE)

1. Composés de formule générale I

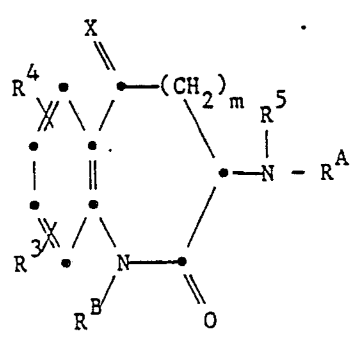

(I)

dans laquelle $R^A$ et $R^B$ représentent des groupes de formules respectives

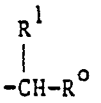 ou 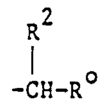

dans lesquelles $R^0$ représente un groupe carboxy ou un groupe carboxy qui a subi une modification fonctionnelle ; $R^1$ représente l'hydrogène, un groupe alkyle inférieur, aminoalkyle inférieur, aryle, aryl-alkyle inférieur, cycloalkyle, cycloalkyl-alkyle inférieur, acylamino-alkyle inférieur, mono- ou di-(alkyle inférieur)-amino-alkyle inférieur, (alkyle inférieur)-thioalkyle inférieur, carboxy-alkyle inférieur, (carboxy estérifié)-alkyle inférieur, carbamoyl-alkyle inférieur, carbamoyl-alkyle inférieur substitué à l'azote, hydroxy-alkyle inférieur, (hydroxy éthérifié ou acylé)-alkyle inférieur, aryloxy-alkyle inférieur, aryl-(thio-, sulfinyl- ou sulfonyl-)-alkyle inférieur, aryl-N-(alkyle inférieur)-amino-alkyle inférieur ou arylamino-alkyle inférieur ; $R^2$ représente l'hydrogène ou un groupe alkyle inférieur ; $R^3$ et $R^4$ représentent chacun indépendamment l'un de l'autre, l'hydrogène, un groupe alkyle inférieur, alcoxy inférieur, alcanoyloxy inférieur, hydroxy, un halogène ou un groupe trifluorométhyle, ou bien $R^3$ et $R^4$ forment ensemble un groupe alkylène-dioxy inférieur ; $R^5$ représente l'hydrogène ou un groupe alkyle inférieur ; m est égal à 2 ou 3 ; et X représente un groupe oxo, deux atomes d'hydrogène ou un groupe hydroxy ou hydroxy acylé et en même temps un atome d'hydrogène ; et dans lesquelles le noyau carbocyclique peut également être perhydrogéné ; les sels et les stéréoisomères de tous ces composés.

2. Composés de formule IA

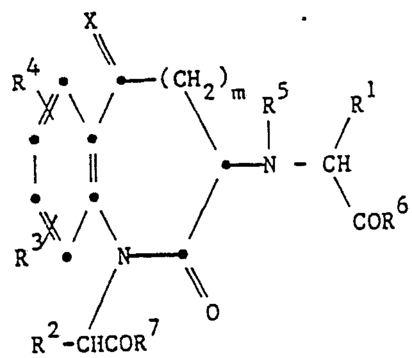

(IA)

dans laquelle le noyau carbocyclique peut également être perhydrogéné ; $R^1$ représente l'hydrogène, un groupe alkyle inférieur, aminoalkyle inférieur, aryle, arylalkyle inférieur, cycloalkyle, cycloalkyl-alkyle inférieur, acylamino-alkyle inférieur, mono- ou di-(alkyle inférieur)-amino-alkyle inférieur, (alkyle inférieur)-thio-alkyle inférieur, carboxy-alkyle inférieur, (carboxy estérifié)-alkyle inférieur, carbamoyl-alkyle inférieur, (carbamoyle substitué à l'azote)-alkyle inférieur, hydroxy-alkyle inférieur, (hydroxy éthérifié ou acylé)-alkyle inférieur, aryloxy-alkyle inférieur, arylthio-alkyle inférieur, aryl-N-(alkyle inférieur)-amino-alkyle inférieur ou arylamino-alkyle inférieur, $R^2$ et $R^5$ représentent l'hydrogène, des groupes alkyle inférieurs ; $R^3$ et $R^4$ représentent l'hydrogène, des groupes alkyle inférieurs, alcoxy inférieurs, alcanoyloxy inférieurs, hydroxy, des halogènes ou des groupes trifluorométhyle ou bien $R^3$ et $R^4$ forment ensemble un groupe alkylène-dioxy inférieur ; X représente un groupe oxo, deux atomes d'hydrogène ou un groupe hydroxy ou hydroxy acylé et un atome d'hydrogène ; m est égal à 2 ou 3 ; $R^6$ et $R^7$ représentent chacun, indépendamment l'un de l'autre, un groupe hydroxy, amino, mono- ou di-(alkyle inférieur)-amino, alcoxy inférieur, aryl-alcoxy inférieur, (alcanoyloxy inférieur)-méthoxy, [amino, mono- ou di-(alkyle inférieur)-amino, carboxy ou (alcoxy inférieur)-carbonyl]-alcoxy inférieur ; ou leurs sels.

3. Composés de formule IA de la revendication 2 dans laquelle $R^1$ représente l'hydrogène, un groupe alkyle inférieur, aminoalkyle inférieur, acylaminoalkyle inférieur, aryl-alkyle inférieur dans lequel la partie aryle consiste en un groupe phényle non substitué ou mono- ou di-substitué par des groupes alkyle inférieurs, hydroxy, alcoxy inférieurs, alkylènedioxy inférieurs, alcanoyloxy inférieurs, des halogènes ou des groupes trifluorométhyle ; $R^3$ et $R^4$ représentent l'hydrogène, des groupes alkyle inférieurs, alcoxy inférieurs, des halogènes ou des groupes trifluorométhyle ou bien $R^3$ et $R^4$ forment ensemble un groupe alkylènedioxy ; $R^6$ et $R^7$ représentent chacun, indépendamment l'un de l'autre, un groupe hydroxy, amino, alcoxy inférieur, phényl-alcoxy inférieur, (alcoxy inférieur)-carbonyl-alcoxy inférieur ; $R^2$, $R^5$, X et m ont les significations indiquées ci-dessus ; ou leurs sels ; ou les composés en question dans lesquels le noyau carbocyclique est perhydrogéné.

4. Composés de formule IA de la revendication 2, dans laquelle $R^1$ représente l'hydrogène, un groupe alkyle inférieur, oméga-amino-alkyle inférieur, oméga-arylméthoxycarbonylamino-alkyle inférieur ou arylalkyle inférieur dans lesquels la partie aryle consiste en un groupe phényle non substitué ou en un groupe phényle monosubstitué par un groupe alkyle inférieur, hydroxy, alcoxy inférieur, alcanoyloxy inférieur, un halogène ou un groupe trifluorométhyle ; $R^2$ et $R^5$ représentent l'hydrogène ou des groupes alkyle inférieurs ; $R^3$ représente l'hydrogène ; $R^4$ représente l'hydrogène, un groupe alkyle inférieur, alcoxy inférieur, un halogène ou un groupe trifluorométhyle ; X représente un groupe oxo, deux atomes

d'hydrogène ou un groupe hydroxy ou alcanoyloxy inférieur et un atome d'hydrogène ; m est égal à 2 ou 3 ; $R^6$ et $R^7$ représentent chacun, indépendamment l'un de l'autre, un groupe hydroxy, amino, alcoxy inférieur, phényl-alcoxy inférieur, (alcoxy inférieur)-carbonyl-alcoxy inférieur ; ou leurs sels ; ou les composés en question dans lesquels le noyau carbocyclique est perhydrogéné.

5. Composés de formule IB

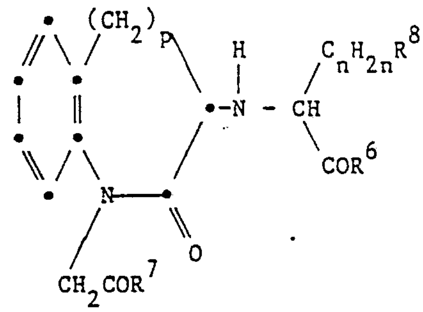

(IB)

dans laquelle n est un nombre entier de 1 à 4, p est un nombre entier égal à 3 ou 4, $R^8$ représente l'hydrogène, un groupe amino, benzyloxycarbonylamino, phényle non substitué ou phényle monosubstitué par un groupe alkyle inférieur, alcoxy inférieur, alcanoyloxy inférieur, un halogène, un groupe hydroxy ou trifluorométhyle ; $R^6$ et $R^7$ représentent chacun, indépendamment l'un de l'autre, un groupe hydroxy, alcoxy inférieur contenant jusqu'à 4 atomes de carbone, benzyloxy ou amino, ou leurs sels ; ou les composés en question dans lesquels le noyau carbocyclique est perhydrogéné.

6. Composés de formule IC

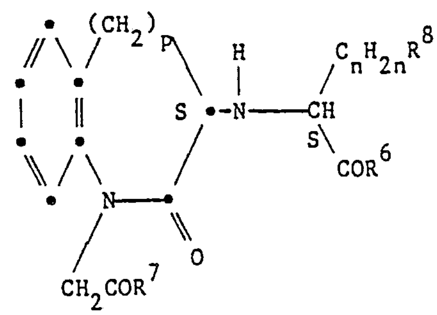

(IC)

dans laquelle S indique la chiralité, n est un nombre entier de 1 à 4 ; p est un nombre entier égal à 3 ou 4, $R^8$ représente l'hydrogène, un groupe amino, benzyloxycarbonylamino, phényle non substitué ou phényle monosubstitué par un groupe alkyle inférieur, alcoxy inférieur, alcanoyloxy inférieur, un halogène, un groupe hydroxy ou trifluorométhyle ; $R^6$ et $R^7$ représentent chacun, indépendamment l'un de l'autre, un groupe hydroxy, alcoxy inférieur contenant jusqu'à 4 atomes de carbone, benzyloxy ou amino ; ou leurs sels.

7. La 1-carboxyméthyl-3-(1-éthoxycarbonyl-3-phénylpropylamino)-3,4,5,6-tétrahydro-1-benzazocine-2-1H-one et ses sels.

8. Un composé du groupe formé par la 1-carboxyméthyl-3-(1-carboxy-3-phénylpropylamino)-3,4,5,6-tétrahydro-1-benzazocine-2-1H-one et la 1-carboxyméthyl-3-(1-éthoxycarbonyl-3-phénylpropylamino)-1,3,4,5,6,7-hexahydro-1-benzazonine-2-one et leurs sels.

9. Un composé du groupe formé par la 1-carboxyméthyl-3-(1-carboxy-3-phényl-(1S)-propylamino)-3,4,5,6-tétrahydro-(3R)-1-benzazocine-2-1H-one, la 1-carboxyméthyl-3-(1-éthoxycarbonyl-3-phényl-(1S)-propylamino)-3,4,5,6-tétrahydro-(3R)-1-benzazocine-2-1H-one, la 3-[(5-benzyloxycarbonylamino-1-éthoxycarbonylpentyl)-amino]-1-éthoxycarbonylméthyl-3,4,5,6-tétrahydro-1-benzazocine-2-1H-one et la 3-[(5-benzyloxycarbonylamino-1-(S)-carboxypentyl)-amino]-1-carboxyméthyl-3,4,5,6-tétrahydro-(3S)-1-benzazocine-2-1H-one et leurs sels.

10. La 1-carboxyméthyl-3-(1-carboxy-3-phényl-(1S)-propylamino)-3,4,5,6-tétrahydro-(3S)-1-benzazocine-2-1H-one et ses sels.

11. La 1-carboxyméthyl-3-(1-éthoxycarbonyl-3-phényl-(1S)-propylamino)-3,4,5,6-tétrahydro-(3S)-1-benzazocine-2-1H-one et ses sels.

12. Compositions pharmaceutiques contenant un composé selon l'une des revendications 1 à 8 ou un sel acceptable pour l'usage pharmaceutique de l'un de ces composés avec un véhicule pharmaceutique approprié.

13. Compositions pharmaceutiques contenant un composé selon l'une des revendications 9 à 11 ou un sel acceptable pour l'usage pharmaceutique d'un tel composé avec un véhicule pharmaceutique approprié.

14. Les composés des revendications 1 à 8, pour l'utilisation dans un procédé pour le traitement thérapeutique de l'organisme humain ou animal.

15. Les composés des revendications 9 à 11, pour l'utilisation dans un procédé pour le traitement thérapeutique de l'organisme humain ou animal.

16. Les composés des revendications 1 à 8, en tant qu'inhibiteurs de l'enzyme convertissant l'angiotensine.

17. Les composés des revendications 9 à 11, en tant qu'inhibiteurs de l'enzyme convertissant l'angiotensine.

18. L'utilisation des composés des revendications 1 à 8 pour la préparation de compositions pharmaceutiques.

19. L'utilisation des composés des revendications 9 à 11 pour la préparation de compositions pharmaceutiques.

20. Procédé de préparation des composés de formule I de la revendication 1, dans laquelle tous les symboles ont les significations indiquées dans la revendication 1, et de leurs sels et stéréoisomères, caractérisé en ce que

a) dans un composé de formule

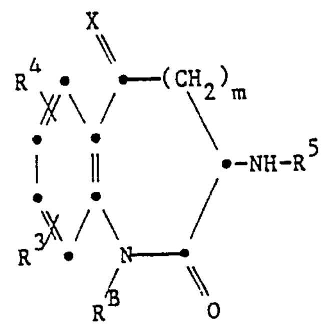

(II)

dans laquelle le noyau carbocyclique peut également être perhydrogéné et X, $R^B$, $R^3$, $R^4$, $R^5$ et m ont les significations indiquées ci-dessus, on introduit $R^A$ par alkylation à l'aide d'un composé de formule

$$R^A—Z \qquad (IIIA)$$

dans laquelle Z représente un groupe hydroxy estérifié réactif et $R^A$ a les significations indiquées ci-dessus, ou à l'aide d'un composé de formule

$$R^1—CO—R^0 \qquad (IV)$$

dans laquelle $R^1$ et $R^0$ ont les significations indiquées ci-dessus, en présence d'un agent réducteur, avec protection transitoire de groupes amino primaires et secondaires éventuels et/ou le cas échéant de groupes hydroxy et/ou qui peuvent exister dans l'un quelconque des groupes X, $R^B$, $R^3$ et $R^4$ et/ou dans l'agent alkylant, ou bien

b) on alkyle un composé de formule

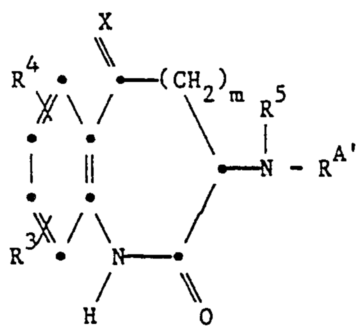

(V)

dans laquelle le noyau carbocyclique peut également être perhydrogéné et X, $R^3$, $R^4$, $R^5$ et m ont les significations indiquées ci-dessus, $R^{A'}$ représentant l'hydrogène ou ayant les significations de $R^A$ définies ci-dessus, à l'aide d'un composé de formule

$$R^B—Z \qquad (IIIB)$$

dans laquelle Z représente un groupe hydroxy estérifié réactif et $R^B$ a les significations indiquées ci-dessus, en protégeant transitoirement des groupes amino primaires ou secondaires éventuels et/ou le cas échéant des groupes hydroxy et/ou oxo qui peuvent exister dans l'un quelconque des groupes X, $R^{A'}$, $R^B$, $R^3$ et $R^4$, ou bien

c) on condense un composé de formule

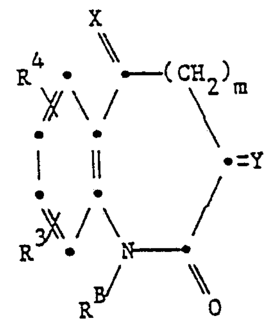

(VI)

dans laquelle le noyau carbocyclique peut également être perhydrogéné et Y représente un groupe oxo, un substituant dichloro ou un groupe hydroxy estérifié réactif Z et en même temps l'hydrogène, et X, $R^B$, $R^3$, $R^4$ et m ont les significations indiquées ci-dessus, avec une amine de formule

$$R^A—NH—R^5 \quad (VII)$$

dans laquelle $R^A$ et $R^5$ ont les significations indiquées ci-dessus, en effectuant la condensation, lorsque Y représente un groupe oxo ou un substituant dichloro, en présence d'un agent réducteur et avec protection transitoire du groupe oxo qui peut être présent en tant que substituant X, ou bien

d) dans un composé de formule

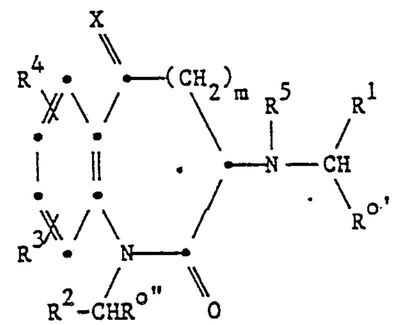

(VIII)

dans laquelle le noyau carbocyclique peut également être perhydrogéné et X, $R^1$ à $R^5$ et m ont les significations indiquées ci-dessus, l'un des symboles $R^{0'}$ et $R^{0''}$ représente un groupe cyano et l'autre un groupe cyano ou $R^0$ tel que défini ci-dessus, on soumet le ou les groupes cyano à solvolyse, ou bien

e) on cyclise un composé de formule

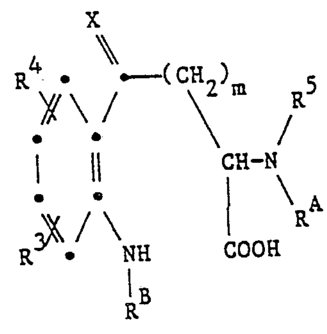

(IX)

dans laquelle le noyau carbocyclique peut également être perhydrogéné et X, $R^A$, $R^B$, $R^3$, $R^4$, $R^5$ et m ont les significations indiquées ci-dessus, ou un ester d'un tel composé, ou bien

f) on traite un composé à structure identique à celle d'un composé de formule I ci-dessus sauf qu'il contient une ou deux doubles liaisons en plus dans le noyau hétérocycle et/ou une double liaison en plus entre l'atome d'azote exocyclique et l'atome de carbone C 3 ou l'atome de carbone voisin à l'intérieur du groupe $R^A$, par un agent réducteur afin de saturer cette ou ces doubles liaisons, et si on le désire, on convertit un composé obtenu, répondant à la formule I, en un autre composé de formule I, et/ou, si on le désire, on convertit un composé obtenu, répondant la formule I et salifiable, en un sel, ou un sel obtenu

en un autre sel, ou bien on libère un composé à partir d'un tel sel et/ou si on le désire, on enrichit un isomère optique ayant une configuration spécifique par rapport à au moins un centre de chiralité dans un mélange de formes stéréoisomères d'un composé obtenu répondant à la formule I.

21. Les composés obtenus par le procédé de la revendication 20.

**Revendications** (pour l'Etat contractant AT)

1. Procédé de préparation de dérivés de la benzazocinone et de la benzazoninone répondant à la formule générale

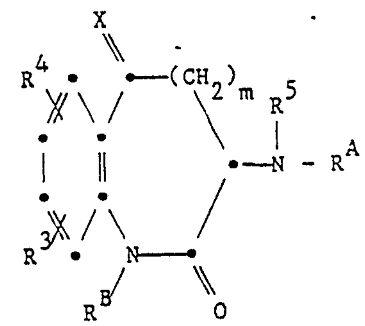

(I)

dans laquelle $R^A$ et $R^B$ représentent des groupes de formules respectives

$$\underset{-CH-R^{o}}{\overset{R^1}{|}} \quad \text{ou} \quad \underset{-CH-R^{o}}{\overset{R^2}{|}}$$

$R^0$ représente un groupe carboxy ou un groupe carboxy qui a subi une modification fonctionnelle ; $R^1$ représente l'hydrogène, un groupe alkyle inférieur, amino-alkyle inférieur, aryle, aryl-alkyle inférieur, cyclo-alkyle, cycloalkyl-alkyle inférieur, acylamino-alkyle inférieur, mono- ou di-(alkyle inférieur)-amino-alkyle inférieur, (alkyle inférieur)-thio-alkyle inférieur, carboxy-alkyle inférieur, (carboxy estérifié)-alkyle inférieur, carbamoyl-alkyle inférieur, (carbamoyle substitué à l'azote)-alkyle inférieur, hydroxyalkyle inférieur (hydroxy éthérifié ou acylé)-alkyle inférieur, aryloxy-alkyle inférieur, aryl-(thio-, sulfinyl- ou sulfonyl-)-alkyle inférieur, aryl-N-(alkyle inférieur)-amino-alkyle inférieur ou arylamino-alkyle inférieur, $R^2$ représente l'hydrogène ou un groupe alkyle inférieur ; $R^3$ et $R^4$ représentent chacun, indépendamment l'un de l'autre, l'hydrogène, un groupe alkyle inférieur, alcoxy inférieur, alcanoyloxy inférieur, hydroxy, un halogène ou un groupe trifluorométhyle ou bien $R^3$ et $R^4$ forment ensemble un groupe alkylène-dioxy inférieur ; $R^5$ représente l'hydrogène ou un groupe alkyle inférieur ; m est égal à 2 ou 3 et X représente un groupe oxo, deux atomes d'hydrogène ou un groupe hydroxy ou hydroxy acylé et un atome d'hydrogène ; et dans laquelle le noyau carbocyclique peut également être perhydrogéné ; des sels et stéréoisomères de tous ces composés, caractérisé en ce que :

a) dans un composé de formule

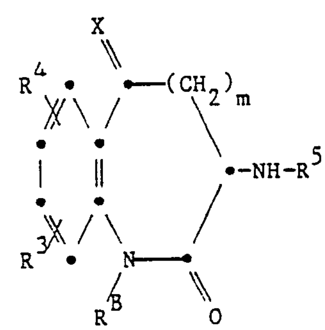

(II)

dans laquelle le noyau carbocyclique peut également être perhydrogéné et X, $R^B$, $R^3$, $R^4$, $R^5$ et m ont les significations indiquées ci-dessus, on introduit $R^A$ par alkylation à l'aide d'un composé de formule

$$R^A—Z \qquad \text{(IIIA)}$$

dans laquelle Z représente un groupe hydroxy estérifié réactif et $R^A$ a les significations indiquées ci-dessus, ou à l'aide d'un composé de formule

$$R^1—CO—R^0 \qquad (IV)$$

dans laquelle $R^1$ et $R^0$ ont les significations indiquées ci-dessus, en présence d'un agent réducteur, en protégeant transitoirement des groupes amino primaires et secondaires éventuels et/ou le cas échéant des groupes hydroxy et/ou oxo qui peuvent être présents dasn l'un quelconque des groupes X, $R^B$, $R^3$ et/ou $R^4$ et/ou dans l'agent alkylant, ou bien

b) on alkyle un composé de formule

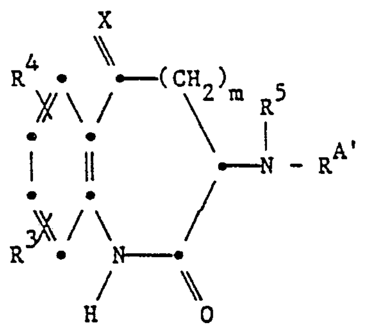

(V)

dans laquelle le noyau carbocyclique peut également être perhydrogéné et X, $R^3$, $R^4$, $R^5$ et m ont les significations indiquées ci-dessus, et $R^{A'}$ représente l'hydrogène ou a les significations de $R^A$ indiquées ci-dessus, à l'aide d'un composé de formule

$$R^B—Z \qquad (IIIB)$$

dans laquelle Z représente un groupe hydroxy estérifié réactif et $R^B$ a les significations indiquées ci-dessus, en protégeant transitoirement des groupes amino primaires et secondaires éventuels et/ou le cas échéant des groupes hydroxy et/ou oxo qui peuvent exister dans l'un quelconque des groupes X, $R^{A'}$, $R^B$, $R^3$ et $R^4$, ou bien

c) on condense un composé de formule

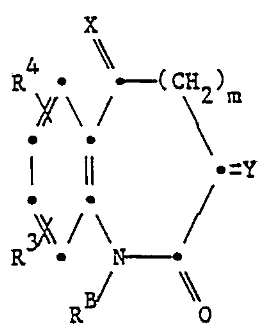

(VI)

dans laquelle le noyau carbocyclique peut également être perhydrogéné et Y représente un groupe oxo, un substituant dichloro ou un groupe hydroxy estérifié réactif Z et en même temps l'hydrogène, et X, $R^B$, $R^3$, $R^4$ et m ont les significations indiquées ci-dessus, avec une amine de formule

$$R^A—NH—R^5 \qquad (VII)$$

dans laquelle $R^A$ et $R^5$ ont les significations indiquées ci-dessus, en effectuant la condensation, lorsque Y représente un groupe oxo ou un substituant dichloro, en présence d'un agent réducteur et avec protection transitoire du groupe oxo qui peut être présent en tant que substituant X, ou bien

d) dans un composé de formule

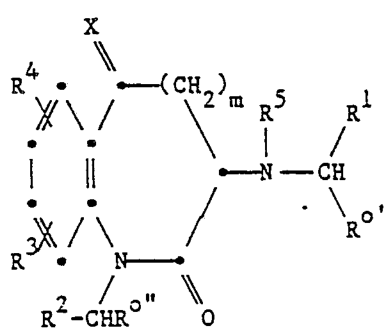

(VIII)

dans laquelle le noyau carbocyclique peut également être perhydrogéné et dans laquelle X, $R^1$ à $R^5$ et m ont les significations indiquées ci-dessus, l'un des symboles $R^{0'}$ et $R^{0''}$ représente un groupe cyano et l'autre un groupe cyano ou $R^0$ tel que défini ci-dessus, on soumet le ou les groupes cyano à une solvolyse, ou bien

e) on cyclise un composé de formule

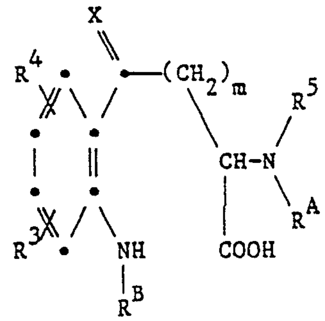

(IX)

dans laquelle le noyau carbocyclique peut également être perhydrogéné et X, $R^A$, $R^B$, $R^3$, $R^4$, $R^5$ et m ont les significations indiquées ci-dessus, ou un ester d'un tel composé, ou bien

f) on traite un composé à structure identique à celle d'un composé de formule I ci-dessus sauf qu'il contient une ou deux doubles liaisons en plus dans le noyau hétérocyclique et/ou une double liaison en plus entre l'atome d'azote exocyclique et l'atome de carbone C 3 ou l'atome de carbone voisin à l'intérieur du groupe $R^A$, par un agent réducteur afin de saturer cette ou ces doubles liaisons, et si on le désire, on convertit un composé obtenu, répondant à la formule I, en un autre composé de formule I et/ou si on le désire, on convertit un composé obtenu, répondant à la formule I et salifiable, en un sel ou un sel obtenu en un autre sel, ou bien on libère un composé à partir d'un tel sel, et/ou si on le désire, on enrichit un isomère optique, ayant une configuration spécifique par rapport à au moins un centre de chiralité dans un mélange de formes stéréoisomères d'un composé obtenu répondant à la formule I.

2. Procédé selon la revendication 1, caractérisé en ce que l'on prépare des composés de formule

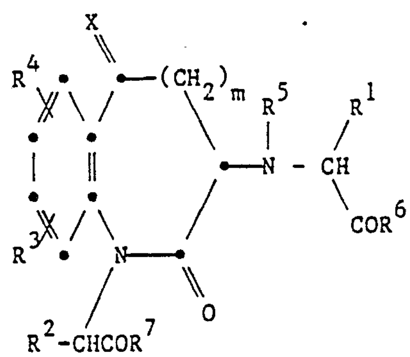

(IA)

dans laquelle le noyau carbocyclique peut également être perhydrogéné ; $R^1$ représente l'hydrogène, un groupe alkyle inférieur, aminoalkyle inférieur, aryle, arylalkyle inférieur, cycloalkyle, cycloalkyl-alkyle inférieur, acylamino-alkyle inférieur, mono- ou di-(alkyle inférieur)-amino-alkyle inférieur, (alkyle inférieur)-thio-alkyle inférieur, carboxy-alkyle inférieur, (carboxy estérifié)-alkyle inférieur, carbamoyl-alkyle inférieur, (carbamoyle substitué à l'azote)-alkyle inférieur, hydroxyalkyle inférieur, (hydroxy éthérifié ou acylé)-alkyle inférieur, aryloxy-alkyle inférieur, arylthio-alkyle inférieur, aryl-N-(alkyle inférieur)-amino-alkyle inférieur ou arylamino-alkyle inférieur ; $R^2$ et $R^5$ représentent l'hydrogène ou des groupes alkyle inférieurs ; $R^3$ et $R^4$ représentent l'hydrogène, des groupes alkyle inférieurs, alcoxy inférieurs, alcanoyloxy inférieurs, hydroxy, des halogènes ou des groupes trifluorométhyle ou bien $R^3$ et $R^4$ forment ensemble un groupe alkylène-dioxy inférieur ; X représente un groupe oxo, deux atomes d'hydrogène ou bien un groupe hydroxy ou hydroxy acylé et un atome d'hydrogène ; m est égal à 2 ou 3 ; $R^6$ et $R^7$ représentent chacun, indépendamment l'un de l'autre, un groupe hydroxy, amino, mono- ou di-(alkyle inférieur)-amino, alcoxy inférieur, aryl-alcoxy inférieur, (alcanoyloxy inférieur)-méthoxy, [amino, mono- ou di-(alkyle inférieur)-amino, carboxy ou (alcoxy inférieur)-carbonyl]-alcoxy inférieur ; ou leurs sels.

3. Procédé selon la revendication 1, caractérisé en ce que l'on prépare des composés de formule IA dans laquelle $R^1$ représente l'hydrogène, un groupe alkyle inférieur, aminoalkyle inférieur, acylamino-alkyle inférieur, aryl-alkyle inférieur dans lequel la partie aryle consiste en un groupe phényle non substitué ou en un groupe phényle mono- ou di-substitué par des groupes alkyle inférieurs, hydroxy, alcoxy inférieurs, alkylènedioxy inférieurs, alcanoyloxy inférieurs, des halogènes ou des groupes

trifluorométhyle ; $R^3$ et $R^4$ représentent l'hydrogène, des groupes alkyle inférieurs, alcoxy inférieurs, des halogènes ou des groupes trifluorométhyle ou bien $R^3$ et $R^4$ forment ensemble un groupe alkylènedioxy ; $R^6$ et $R^7$ représentent chacun, indépendamment l'un de l'autre, un groupe hydroxy, amino, alcoxy inférieur, phényl-alcoxy inférieur, (alcoxy inférieur)-carbonylalcoxy inférieur ; $R^2$, $R^5$, X et m ont les significations indiquées ci-dessus, ou leurs sels ; ou les composés en question dans lesquels le noyau carbocyclique est perhydrogéné.

4. Procédé selon la revendication 1, caractérisé en ce que l'on prépare des composés de formule IA dans laquelle $R^1$ représente l'hydrogène, un groupe alkyle inférieur, oméga-aminoalkyle inférieur, oméga-arylméthoxycarbonylamino-alkyle inférieur ou aryl-alkyle inférieur dans lesquels la partie aryle consiste en un groupe phényle non substitué ou en un groupe phényle monosubstitué par un groupe alkyle inférieur, hydroxy, alcoxy inférieur, alcanoyloxy inférieur, un halogène ou un groupe trifluorométhyle ; $R^2$ et $R^5$ représentent l'hydrogène ou des groupes alkyle inférieurs, $R^3$ représente l'hydrogène ; $R^4$ représente l'hydrogène, un groupe alkyle inférieur, alcoxy inférieur, un halogène ou un groupe trifluorométhyle ; X représente un groupe oxo, deux atomes d'hydrogène ou un groupe hydroxy ou alcanoyloxy inférieur et un atome d'hydrogène ; m est égal à 2 ou 3 ; $R^6$ et $R^7$ représentent chacun, indépendamment l'un de l'autre, un groupe hydroxy, amino, alcoxy inférieur, phényl-alcoxy inférieur, (alcoxy inférieur)-carbonyl-alcoxy inférieur ; ou leurs sels ; ou les composés en question dans lesquels le noyau carbocyclique est perhydrogéné.

5. Procédé selon la revendication 1, caractérisé en ce que l'on prépare des composés de formule

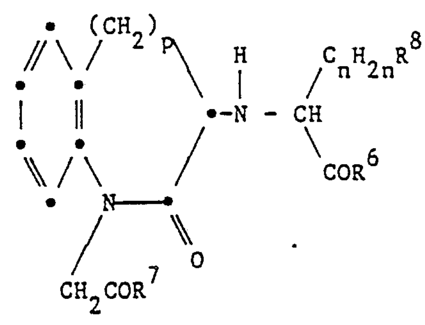

(IB)

dans laquelle n est un nombre entier de 1 à 4, p est un nombre entier égal à 3 ou 4, $R^8$ représente l'hydrogène, un groupe amino, benzyloxycarbonylamino, phényle non substitué ou phényle monosubstitué par un groupe alkyle inférieur, alcoxy inférieur, alcanoyloxy inférieur, un halogène, un groupe hydroxy ou trifluorométhyle ; $R^6$ et $R^7$ représentent chacun, indépendamment l'un de l'autre, un groupe hydroxy, alcoxy inférieur contenant jusqu'à 4 atomes de carbone, benzyloxy ou amino, ou leurs sels ; ou les composés en question dans lesquels le noyau carbocyclique est perhydrogéné.

6. Procédé selon la revendication 1, caractérisé en ce que l'on prépare des composés de formule

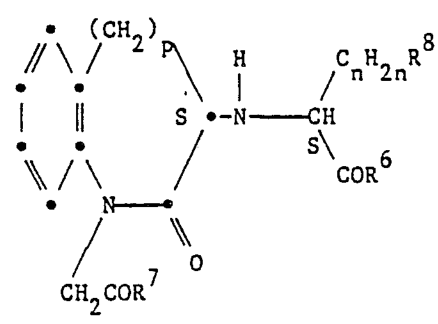

(IC)

dans laquelle S indique la chiralité, n est un nombre entier de 1 à 4 ; p est égal à 3 ou 4, $R^8$ représente l'hydrogène, un groupe amino, benzyloxycarbonylamino, phényle non substitué ou phényle monosubstitué par un groupe alkyle inférieur, alcoxy inférieur, alcanoyloxy inférieur, un halogène, un groupe hydroxy ou trifluorométhyle, $R^6$ et $R^7$ représentent chacun, indépendamment l'un de l'autre, un groupe hydroxy, alcoxy inférieur contenant jusqu'à 4 atomes de carbone, benzyloxy ou amino ; ou leurs sels.

7. Procédé selon la revendication 1, caractérisé en ce que l'on prépare la 1-carboxyméthyl-3-(1-carboxy-3-phényl-(1S)-propylamino)-3,4,5,6-tétrahydro-(3S)-1-benzazocine-2-1H-one ou un sel de ce composé.

8. Procédé selon la revendication 1, caractérisé en ce que l'on prépare la 1-carboxyméthyl-3-(1-éthoxycarbonyl-3-phényl-(1S)-propylamino)-3,4,5,6-tétrahydro-(3S)-1-benzazocine-2-1H-one ou un sel de ce composé.

9. Procédé de préparation des composés de formule I de la revendication 1, dans laquelle tous les symboles ont les significations indiquées dans la revendication 1, et de leurs stéréoisomères et sels,

caractérisé en ce que l'on exécute les variantes opératoires a) à e) de la revendication 1, ou bien f) on traite un composé à structure identique à celle d'un composé de formule I, sauf qu'il contient une double liaison en plus sur l'atome de carbone C 3 ou entre l'atome d'azote et l'atome de carbone voisin dans le groupe $R^A$, par un agent réducteur afin de saturer cette double liaison, et si on le désire, on convertit un composé obtenu, répondant à la formule I, en un autre composé de formule I et/ou, si on le désire, on convertit un composé obtenu, répondant à la formule I et salifiable, en un sel, ou un sel obtenu en un autre sel, ou bien on libère un composé à partir d'un tel sel et/ou on enrichit si on le désire un isomère optique ayant une configuration spécifique eu égard à au moins un centre de chiralité, dans un mélange de formes stéréoisomères d'un composé obtenu répondant à la formule I.

10. Procédé selon la revendication 9, caractérisé en ce que l'on prépare la 1-carboxyméthyl-3-(1-éthoxycarbonyl-3-phényl-propylamino)-3,4,5,6-tétrahydro-1-benzazocine-2-1H-one ou un sel de ce composé.

11. Procédé selon la revendication 9, caractérisé en ce que l'on prépare des composés de formule IA, IB ou IC des revendications 2 à 6.

12. Procédé de préparation de compositions pharmaceutiques, caractérisé en ce que l'on combine une substance active préparée selon l'invention selon l'une des revendications 1 à 8, avec un véhicule pharmaceutique.

13. Procédé de préparation d'une composition pharmaceutique, caractérisé en ce que l'on combine une substance active préparée selon l'invention selon l'une des revendications 9 à 11, avec un véhicule pharmaceutique.